(19) Europäisches Patentamt
European Patent Office
Office européen des brevets

(11) EP 0 973 793 B1

(12) # FASCICULE DE BREVET EUROPEEN

(45) Date de publication et mention de la délivrance du brevet:
**10.07.2002 Bulletin 2002/28**

(21) Numéro de dépôt: **98920587.7**

(22) Date de dépôt: **08.04.1998**

(51) Int Cl.[7]: **C07J 41/00**, A61K 31/56

(86) Numéro de dépôt international:
**PCT/FR98/00709**

(87) Numéro de publication internationale:
**WO 98/45316 (15.10.1998 Gazette 1998/41)**

(54) **NOUVEAUX STEROIDES 4-HALOGENES, LEUR PROCEDE ET INTERMEDIAIRES DE PREPARATION, LEUR APPLICATION COMME MEDICAMENTS ET LES COMPOSITIONS PHARMACEUTIQUES LES RENFERMANT**

NEUE 4-HALOGENIERTE STEROIDE, EIN VERFAHREN UND ZWISCHENPRODUKTEN ZU IHRER HERSTELLUNG, IHRE VERWENDUNG ALS MEDIKAMENTEN UND PHARMAZEUTISCHE ZUSAMMENSETZUNGEN DAVON

NOVEL 4- HALOGENATED STEROIDS, PREPARATION METHOD AND INTERMEDIATES, APPLICATION AS MEDICINES AND PHARMACEUTICAL COMPOSITIONS CONTAINING SAME

(84) Etats contractants désignés:
**AT BE CH CY DE DK ES FI FR GB GR IE IT LI LU NL PT SE**
Etats d'extension désignés:
**AL LT LV MK RO SI**

(30) Priorité: **09.04.1997 FR 9704321**

(43) Date de publication de la demande:
**26.01.2000 Bulletin 2000/04**

(73) Titulaire: **Aventis Pharma S.A.**
**92160 Antony (FR)**

(72) Inventeurs:
- **BOUALI, Yamina**
**F-94800 Villejuif (FR)**
- **NIQUE, François**
**F-94170 Le Perreux sur Marne (FR)**
- **TEUTSCH, Jean-Georges**
**F-93500 Pantin (FR)**
- **VAN DE VELDE, Patrick**
**F-75019 Paris (FR)**

(56) Documents cités:
**FR-A- 2 640 977** **US-A- 4 676 932**

- **JIN L ET AL: "ANTISTROGENIC ACTIVITY OF TWO 11BETA-ESTRADIOL DERIVATIVES ON MCF-7 BREAST CANCER CELLS" STEROIDS: STRUCTURE, FUNCTION, AND REGULATION, vol. 60, no. 8, août 1995, pages 512-518, XP002040770**
- **ANSTEAD G M ET AL: "The estradiol pharmacophore: Ligand structure-estrogen receptor binding affinity relationships and a model for the receptor binding site" STEROIDS: STRUCTURE, FUNCTION, AND REGULATION, vol. 62, no. 3, mars 1997, page 268-303 XP004057108**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 973 793 B1

Printed by Jouve, 75001 PARIS (FR)

## Description

La présente invention concerne des composés stéroïdes 4-halogènes, leur procédé de préparation, leur application comme médicament et les compositions pharmaceutiques les renfermant.

L'ostéoporose est une pathologie qui se caractérise par une réduction quantitative et qualitative du tissu osseux, suffisante pour entraîner des fractures vertébrales ou périphériques, ce de façon spontanée ou à l'occasion de traumatisme minime. Bien que cette affection soit d'origine multifactorielle, c'est la ménopause qui, chez la femme, constitue le facteur prépondérant de la perte osseuse ou ostéopénie.

Cette ostéopénie se manifeste par une raréfaction et une modification de l'architecture de l'os spongieux qui a pour conséquence d'accentuer la fragilité squelettique et le risque fracturaire. La perte osseuse s'accentue fortement après la ménopause en raison de la suppression de la fonction ovarienne et atteint 3 à 5 % par an pour se ralentir après 65 ans.

Dans un but thérapeutique, la carence hormonale post ménopausique peut être compensée par une hormonothérapie substitutive où l'oestrogène joue un rôle majeur en préservant le capital osseux. Mais l'estrogénothérapie au long cours s'accompagne parfois d'effet indésirable sur l'appareil génital (hyperplasie endométriale, tumeur mammaire...), ce qui constitue un inconvénient majeur et limite son application.

Il convient donc de trouver d'autres composés que l'oestradiol ayant une activité oestrogène dissociée, à savoir une activité oestrogène au niveau osseux, tout en n'ayant pas ou peu d'activité d'hyperplasie endométriale, ni d'activité de prolifération de tumeur mammaire.

. Jin 1. Et al. (Steroids, 60 (1995), P.512-518 et Anstead, G.M. et al Steroids, 62 (1996), P. 268-303 décrivent des dérivés de l'estradiol présentant une affinité au récepteur estradiol.

L'invention a donc pour objet les composés de formule générale (I) :

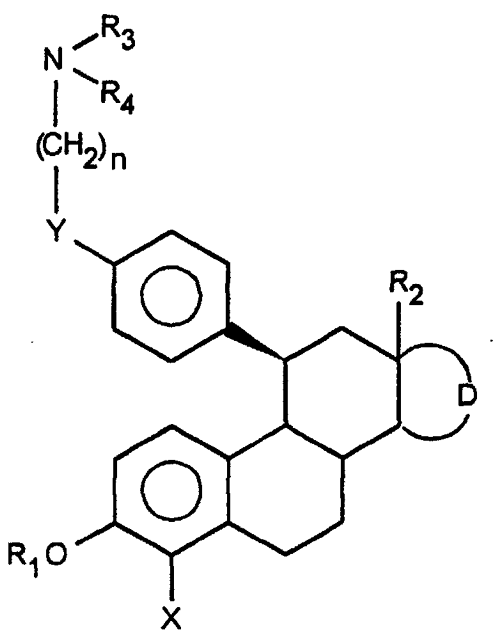

(I)

dans laquelle :

- $R_1$ représente un atome d'hydrogène, un radical $(CH_2)_m$-Ar, (CO)-Ar, $(CH_2)_m$-Alk ou (CO)-Alk,
- $R_2$ représente un radical dérivé d'un hydrocarbure, linéaire ou ramifié, saturé ou insaturé renfermant de 1 à 6 atomes de carbone
- D représente le reste d'un cycle pentagonal ou hexagonal éventuellement substitué et éventuellement porteur d'insaturation,
- X représente un atome d'halogène,
- Y est choisi parmi O, S, SO, $SO_2$ et NH,
- n est un entier variant de 2 à 8,
- soit $R_3$ et $R_4$ identiques ou différents représentent un atome d'hydrogène, un groupement $(CH_2)_m$-Ar, $(CH_2)_m$-Het ou $(CH_2)_m$Alk,
- soit $R_3$ et $R_4$ forment ensemble avec l'atome d'azote auquel ils sont liés un hétérocycle mono ou polycyclique, saturé

ou insaturé, aromatique ou non aromatique, de 3 à 15 chaînons renfermant éventuellement de 1 à 3 hétéroatomes additionnels choisis parmi l'oxygène, le soufre et l'azote, non substitué ou substitué,
Ar représentant un groupement aryle carbocyclique renfermant de 6 à 18 atomes de carbone, Het représentant un radical dérivé d'un hétérocycle aromatique ou non aromatique, saturé ou non saturé, comportant de 1 à 9 atomes de carbone et de 1 à 5 hétéroatomes choisis parmi les atomes d'oxygène, d'azote ou de soufre, Alk représentant un radical dérivé d'un hydrocarbure, non aromatique, linéaire, ramifié ou cyclique, saturé ou insaturé et comportant de 1 à 12 atomes de carbone, les radicaux Ar, Het ou Alk pouvant être substitués ou non substitués, m représente 0, 1, 2 ou 3, ainsi que leurs sels d'addition avec les bases ou les acides.

On entend par halogène : iode, brome, chlore ou fluor.
On entend par $(CH_2)_m$ les valeurs suivantes : simple liaison dans le cas où m est égal à 0, $CH_2$, $(CH_2)_2$ et $(CH_2)_3$.
Par le terme Ar représentant le groupe aryle carbocyclique renfermant de 6 à 18 atomes de carbone, on entend un dérivé d'un hydrocarbure cyclique aromatique tel que le radical phényle, naphtyle, phénanthrényle ou bien un dérivé d'un hydrocarbure bicyclique ou tricyclique condensé comportant un cycle benzénique tel que indanyle, indényle, dihydronaphtyle, tétrahydronaphtyle ou fluorényle. La jonction s'effectue au niveau du cycle benzénique. Il s'agit de préférence du phényle.
Par le terme (Het) représentant un radical dérivé d'un hétérocycle aromatique ou non aromatique, saturé ou non saturé, comportant de 1 à 9 atomes de carbone et de 1 à 5 hétéroatomes choisis parmi les atomes d'oxygène, d'azote et de soufre, on désigne notamment :

- les radicaux monocyclique hétérocycliques, par exemple les radicaux thiényle, furyle, pyrannyle, pyrrolyle, imidazolyle, pyrazolyle, pyridyle, pyrazinyle, pyrimidinyle, pyridazinyle, thiazolyle, oxazolyle, furazannyle, pyrrolinyle, imidazolinyle, pyrazolinyle, thiazolinyle, triazolyle, tétrazolyle,
- les cycles condensés hétérocycliques, par exemple le benzofurannyle, le benzothiényle, le benzimidazolyle, le benzothiazolyle, le naphto[2,3-b]thiényle, le thianthrényle, l'isobenzofurannyle, le chroményle, le xanthényle, le phénoxathiinyle, l'indolizinyle, l'isoindolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, le carbazolyle, le béta-carbolinyle, l'acridinyle, le phénazinyle, le phénothiazinyle, le phénoxazinyle, l'indolinyle, l'isoindolinyle, l'imidazopyridyle, l'imidazopyrimidinyle ou encore les systèmes polycycliques condensés constitués de monocycliques hétérocycliques tels que définis ci-dessus comme par exemple le furo[2,3-b]pyrrole ou le thiéno[2,3-b]furanne,
- ou les hétérocycles saturés tels que pyrrolidine, pipéridine, morpholine.

Par le terme (Alk) représentant un radical dérivé d'un hydrocarbure, non aromatique, linéaire, ramifié ou cyclique, saturé ou insaturé, on désigne dans le cas des hydrocarbures acycliques les radicaux alkyles tels que méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthylheptyle ou n-décyle, les radicaux alkényles tels que vinyle, propényle, isopropényle, allyle, 2-méthylallyle, butényle ou isobutényle, ou les radicaux alkynyles tels que éthynyle, propynyle, propargyle, butynyle ou isobutynyle, et dans le cas des radicaux cycliques, les radicaux cycloalkyles, tels que cyclopropyle, cyclobutyle, cyclopentyle ou cyclohexyle.
Il s'agira de préférence des radicaux méthyle et éthyle. Par CO-Alk on entend de préférence $COCH_3$ et COEt, par CO-Ar on entend de préférence le radical benzoyle, lorsque m est différend de zéro, $(CH_2)_m$-Ar sera de préférence le groupement benzyle.
Lorsque $R_3$ et $R_4$ forment ensemble avec l'atome d'azote auquel ils sont liés un hétérocycle, il s'agit notamment des hétérocycles mono ou bicycliques renfermant éventuellement un autre hétéroatome choisi parmi l'oxygène et l'azote tels que les hétérocycles insaturés suivants : pyrrolyle, imidazolyle, indolyle, pyridyle, pyrazinyle, pyrimidinyle, pyridazinyle, thiazolyle, oxazolyle, furazolinyle, pyrazolinyle, thiazolinyle, ou, plus particulièrement, les hétérocycles saturés suivants :

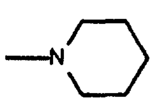 , 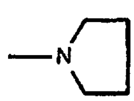 , 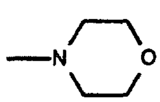 , 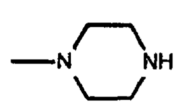 et 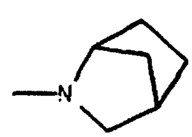

Lorsque les différents groupements Alk, Ar, Het, ainsi que le reste d'un cycle pentagonal ou hexagonal cité plus haut, sont substitués, ils peuvent l'être notamment par les radicaux suivants :

- halogène, à savoir fluor, chlore, brome ou iode, alkoxy tel que méthoxy, éthoxy, propyloxy, isopropyloxy, butyloxy, alkylthio tel que méthylthio, éthylthio, propylthio, isopropylthio, butylthio, amino, alkylamino tel que méthylamino ou éthylamino, dialkylamino tel que diméthylamino, diéthylamino, méthyléthylamino, chacun de ces radicaux dialkylamino étant éventuellement sous forme oxydée, aminoalkyle tel que aminométhyle ou aminoéthyle, dialkylaminoalkyle tel que diméthylamino méthyle ou éthyle, dialkylaminoalkyloxy tel que diméthylamino éthyloxy, hydroxyle éventuellement acylé, acyle tel que acétyle, propionyle, butyryle, benzoyle, carboxy libre, estérifié tel que alkoxy carbonyle par exemple méthoxy carbonyle ou éthoxy carbonyle, cyano, trifluorométhyle, aryle tel que phényle, aralkyle tel que benzyle, alkyle, alkényle ou alkynyle ces radicaux étant eux-mêmes éventuellement substitués par les radicaux halogène, alkyle, alkoxy, alkylthio, aminoalkyle ou dialkylamino indiqués ci-dessus.

Bien entendu, l'expression "substitué" indique qu'un ou plusieurs substituants, identiques ou différents, peuvent être présents. Dans le cas de (Het), les substituants peuvent être au niveau de NH ou d'un atome de carbone.
Bien entendu les valeurs de $R_1$, $R_2$, $R_3$ et $R_4$, sont indépendantes les unes des autres.
L'invention s'étend naturellement aux sels des composés de formule (I), comme par exemple les sels formés avec des acides minéraux ou organique sur l'amine. Il peut alors s'agir des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques. Lorsque les composés de formule (I) comportent une fonction acide, l'invention s'étend aux sels des métaux alcalins, alcalino terreux ou d'ammonium éventuellement substitués.
L'invention a plus particulièrement pour objet les composés de formule générale (I) telle que définie plus haut dans laquelle (D) représente le reste d'un cycle pentagonal de formule :

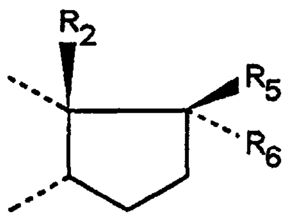

dans laquelle $R_2$ conserve la même signification que précédemment,
soit $R_5$ représente un radical OH, O-$(CH_2)_m$-Alk, O-(CO)-Alk, O-$(CH_2)_m$-Ar, O-(CO)-Ar, O-$(CH_2)_m$-Het, O-(CO)-Het et $R_6$ représente un atome d'hydrogène, un radical alkyle, alkényle ou alkynyle renfermant de 1 à 6 atomes de carbone substitué ou non substitué, m, Alk, Ar et Het étant tels que définis précédemment,
soit $R_5$ et $R_6$ forment ensemble avec l'atome de carbone qui les porte l'un des cycles suivants :

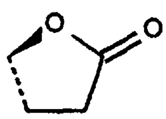

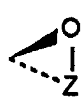

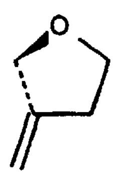

dans lequel Z représente un groupement $-(CH_2)_1-$ ou $-CH=CH-(CH_2)_{1'}$ ; 1 étant un entier compris entre 1 et 4 et 1' étant un entier égal à 1 ou 2,
soit $R_5$ et $R_6$ forment ensemble un groupement oxo ou =N-OH, ainsi que leurs sels d'addition avec les acides ou les bases.
L'invention a tout particulièrement pour objet les composés de formule (I) telle que définie précédemment répondant à la formule générale (I') :

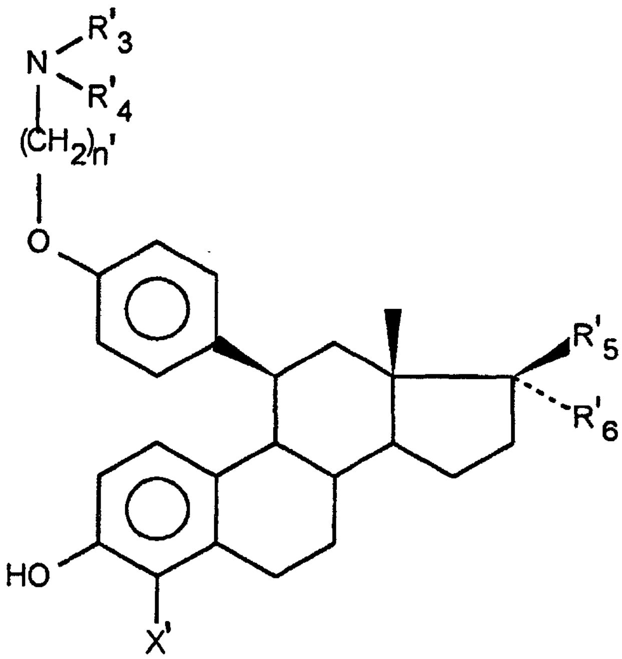

(I')

dans laquelle :

X' représente un atome de chlore ou de brome
n' est compris entre 2 et 5,
soit $R'_3$ et $R'_4$ identiques ou différents représentent un radical alkyle renfermant de 1 à 6 atomes de carbone
soit $R'_3$ et $R'_4$ forment ensemble, avec l'atome d'azote auquel ils sont liés, un reste mono ou polyclique saturé de 3 à 15 chainons renfermant éventuellement un hétéroatome additionnel choisi parmi l'oxygène, le soufre et l'azote,
$R'_5$ et $R'_6$ ont la même signification que $R_5$ et $R_6$,

ainsi que leurs sels d'addition avec les acides et les bases.

L'invention a tout particulièrement pour objet les composés de formule (I) telle que définie précédemment répondant à la formule générale (I') dans laquelle :

soit $R'_5$ représente un radical OH et $R'_6$ représente un atome d'hydrogène, un radical alkyle, alkényle ou alkynyle renfermant de 1 à 6 atomes de carbone, substitué ou non substitué, soit $R'_5$ et $R'_6$ forment ensemble avec l'atome de carbone qui les porte l'un des cycles suivants :

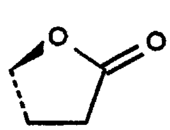 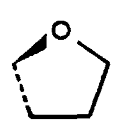 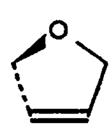 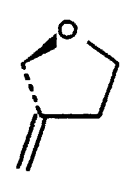

soit $R'_5$ et $R'_6$ forment ensemble un groupement oxo,
ainsi que leurs sels d'addition avec les acides ou les bases.

L'invention a tout particulièrement pour objet les composés de formule (I) répondant à la formule générale (I') telle que définie précédemment dans laquelle :

X' représente un atome de chlore
n' est égal à 2,
soit $R'_3$ et $R'_4$ identiques ou différents représentent un radical alkyle renfermant de 1 à 6 atomes de carbone
soit $R'_3$ et $R'_4$ forment ensemble avec l'atome d'azote les hétérocycles saturés suivants :

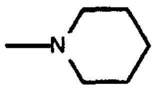 , 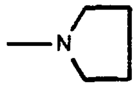 , 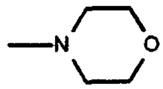 , 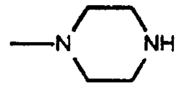 et 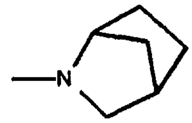

et soit $R'_5$ représente un radical OH et $R'_6$ représente un atome d'hydrogène, un radical alkyle, alkényle ou alkynyle renfermant de 1 à 6 atomes de carbone, substitué ou non substitué,
soit $R'_5$ et $R'_6$ forment ensemble avec l'atome de carbone qui les porte l'un des cycles suivants :

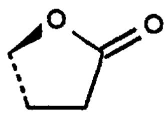 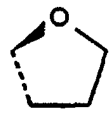 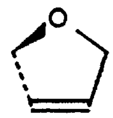 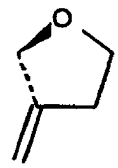

soit $R'_5$ et $R'_6$ forment ensemble un groupement oxo,

ainsi que leurs sels d'addition avec les acides ou les bases.
L'invention a tout particulièrement pour objet les composés de formule (I) ainsi que leurs sels d'addition avec les acides dont les noms suivent :

- 4-chloro-3-hydroxy-11β-[4-[2-(diéthylamino)éthoxy]phényl]-estra-1,3,5(10)-trièn-17-one,
- 4-chloro-3-hydroxy-11β-[4-[2-(diméthylamino)éthoxy]phényl]-estra-1,3,5(10)-trièn-17-one,
- 4-chloro-3-hydroxy-11β-[4-[2-(1-pipéridinyl)éthoxy]phényl]-estra-1,3,5(10)-trièn-17-one,
- 4-chloro-3-hydroxy-11β-[4-[2-(1-pyrrolidinyl)éthoxy] phényl]-estra-1,3,5(10)-trièn-17-one,
- 4-bromo-3-hydroxy-11β-[4-[2-(1-pipéridinyl)éthoxy]phényl]-estra-1,3,5(10)-trièn-17-one,
- 4-chloro-11β-[4-(2-(diméthylamino)éthoxy]phényl]-estra-1,3,5(10)-triène-3,17béta-diol,
- 4-chloro-11β-[4-[2-(diethylamino)éthoxy]phényl]-estra-1,3,5(10)-triène-3,17béta-diol,
- 4-chloro-11β-[4-[2-(1-pipéridinyl)éthoxy]phényl]-estra-1,3,5(10)-triène-3,17béta-diol,
- 4-bromo-11β-[4-[2-(1-pipéridinyl)éthoxy]phényl]-estra-1,3,5(10)-triène-3,17béta-diol,
- 4-chloro-11β-[4-[2-(1-pyrrolidinyl)éthoxy]phényl]-estra-1,3,5(10)-triène-3,17béta-diol,
- 4-chloro-11β-[4-[2-(diéthylamino)éthoxy]phényl]-19-nor-l7alpha-pregna-1,3,5(10)-trièn-20-yne-3,17béta-diol,
- 4-chloro-11β-[4-[3-(1-pipéridinyl)propoxy]phényl]-estra-1,3,5(10)-triène-3,17béta-diol,
- 4-chloro-11β-[4-[4-(1-pipéridinyl)butoxy]phényl]-estra-1,3,5(10)-triène-3,17béta-diol,
- 4-chloro-11β-[4-[5-(1-pipéridinyl)pentyloxy]phényl]-estra-1,3,5(10)-triène-3,17béta-diol,
- gamma lactone de l'acide (17alpha)-4-chloro-3,17béta-dihydroxy-11β-[4-[2-(diéthylamino)éthoxy]phényl]-19-nor-pregna-1,3,5(10)-triène-21-carboxylique,
- gamma lactone de l'acide (17alpha)-4-chloro-3,17béta-dihydroxy-11β-[4-[2-(1-pyrrolidinyl)éthoxy]phényl]-19-nor pregna-1,3,5(10)-triène-21-carboxylique,
- gamma lactone de l'acide (17alpha)-4-chloro-3,17béta-dihydroxy-11β-[4-[2-(1-pipéridinyl)éthoxy]phényl]-19-nor-pregna-1,3,5(10)-triène-21-carboxylique,
- gamma lactone de l'acide (17alpha)-4-chloro-3,17béta-dihydroxy-11β-[4-[3-(1-pipéridinyl)propoxy]phényl]-19-nor-pregna-1,3,5(10)-triène-21-carboxylique,
- gamma lactone de l'acide (17alpha)-4-chloro-3,17béta-dihydroxy-11β-[4-[4-(1-pipéridinyl)butoxy]phényl]-19-nor-pregna-1,3,5(10)-triène-21-carboxylique,
- gamma lactone de l'acide (17alpha)-4-chloro-3,17béta-dihydroxy-11β-[4-[5-(1-pipéridinyl)pentyloxy]phényl]-19-nor-pregna-1,3,5(10)-triène-21-carboxylique,
- (17béta)-4-chloro-11β-[4-[2-(diéthylamino)éthoxy]phényl]-spiro[estra-1,3,5(10)-triène-17,2'(5'H)furan]-3-ol,
- (17béta)-4-chloro-11β-[4-[2-(1-pipéridinyl)éthoxy]phényl]-spiro[estra-1,3,5(10)-triène-17,2'(5'H)furan]-3-ol,
- (17béta)-4-chloro-11β-[4-[2-(1-pyrrolidinyl)éthoxy]phényl]-spiro[estra-1,3,5(10)-triène-17,2' (5'H)furan]-3-ol,
- (17béta)-4-chloro-4',5'-dihydro-11β-[4-[2-(1-pipéridinyl) éthoxy]phényl]-spiro[estra-1,3,5(10)-triène-17,2'(3'H)furan]-3-ol,
- (17béta)-4-chloro-11β-[4-[3-(1-pipéridinyl)propoxy]phényl]-spiro[estra-1,3,5(10)-triène-17,2'(5'H)furan]-3-ol,
- (17béta)-4-chloro-4',5'-dihydro-11β-[4-[3-(1-pipéridinyl) propoxy]phényl]-spiro[estra-1,3,5(10)-triène-17,2'(3'H) furan]-3-ol,

- (17béta)-4-chloro-11β-[4-[4-(1-pipéridinyl)butoxy]phényl]-spiro[estra-1,3,5(10)-triène-17,2'(5'H)furan]-3-ol,
- (17béta) -4-chloro-4',5'-dihydro-11β-[4-[4-(1-pipéridinyl)-butoxy]phényl]-spiro[estra-1,3,5(10)-triène-17,2'(3'H)furan]-3-ol,
- 4-chloro-11β-[4-[2-(diéthylamino)éthoxy]phényl]-17alpha-méthyl-estra-1,3,5(10)-triène-3,17béta-diol,
- 4-chloro-17alpha-méthyl-11β-[4-[2-(1-pipéridinyl)éthoxy] phényl]-estra-1,3,5(10)-triéne-3,17béta-diol,
- 4-chloro-17alpha-méthyl-11β-[4-[2-(1-pyrrolidinyl)éthoxy] phényl]-estra-1,3,5(10)-triène-3,17béta-diol,
- 11β-[4-[2-[2-aza-bicyclo(2.2.1)hept-2-yl]éthoxy] phényl]-4-chloro-17alpha-méthyl-estra-1,3,5(10)-triène-3,17béta-diol,
- 11β-[4-[2-[2-aza-bicyclo(2.2.1)hept-2-yl]éthoxy] phényl]-4-chloro-estra-1,3,5(10)-triène-3,17béta-diol,
- 4-chloro-11β-[4-[2-[méthyl(1-méthyléthyl)amino]éthoxy] phényl]-estra-1,3,5(10)-triène-3,17béta-diol,
- 4-chloro-11β-[4-[2-(tétrahydro-(1H)-1,4-thiazin-4-yl) éthoxy]phényl]-estra-1,3,5(10)-triène-3,17béta-diol,
- 4-chloro-11β-[4-[2-[éthyl(propyl)amino]éthoxy]phényl]-estra-1,3,5(10)-triène-3,17béta-diol,
- 4-chloro-11β-[4-[2-[éthyl(méthyl)amino]éthoxy]phényl]-estra-1,3,5(10)-triène-3,17béta-diol,
- 4-chloro-11β-[4-[2-[(1,1-diméthyl-2-propynyl)amino]éthoxy] phényl]-estra-1,3,5(10)-triène-3,17béta-diol,
- 4-chloro-11β-[4-[2-[hexahydro-1H-azépin-1-yl)éthoxy] phényl]-estra-1,3,5(10)-triène-3,17béta-diol,
- 4-chloro-11β-[4-[2-[cyclohexyl(méthyl)amino]éthoxy]phényl]-estra-1,3,5(10)-triène-3,17béta-diol,
- 4-chloro-11β-[4-[2-[butyl(éthyl)amino]éthoxy]phényl]-estra-1,3,5(10)-triène-3,17béta-diol,
- 4-chloro-11β-[4-[2-(1-azétidinyl)éthoxy]phényl]-estra-1,3,5(10)-triène-3,17béta-diol,
- 4-chloro-11β-[4-[2-[éthyl(2-propényl)amino]éthoxy]phényl]-estra-1,3,5(10)-triène-3,17béta-diol,
- 4-chloro-11β-[4-[2-[cyclopentyl(méthyl)amino]éthoxy] phényl]-estra-1,3,5(10)-triène-3,17béta-diol,
- 4-chloro-11β-[4-[2-(dipropylamino)éthoxy]phényl]-estra-1,3,5(10)-triène-3,17béta-diol,
- 4-chloro-11β-[4-[2-(3-thiazolidinyl)éthoxy]phényl]-estra-1,3,5(10)-triène-3,17béta-diol,
- 4-chloro-11β-[4-[2-[éthyl(1-méthyléthyl)amino]éthoxy] phényl]-estra-1,3,5(10)-triène-3,17béta-diol,
- 4-chloro-11β-[4-[2-[méthyl(propyl)amino]éthoxy]phényl]-estra-1,3,5(10)-triène-3,17béta-diol,
- 4-chlore-11β-[4-[2-[butyl(méthyl)amino]éthoxy]phényl]-estra-1,3,5(10)-triène-3,17béta-diol,
- 4-chloro-11β-[4-[2-[méthyl(2-propynyl)amino]éthoxy]phényl]-estra-1,3,5(10)-triène-3,17béta-diol,
- 4-chloro-11β-[4-[2-(4-méthyl-1-pipéridinyl)éthoxy]phényl]-estra-1,3,5(10)-triène-3,17béta-diol,
- 4-chloro-11β-[4-[2-(1-pipéridinyl)éthylthio]phényl]-estra-1,3,5(10)-triène-3,17béta-diol,
- 4-chloro-11β-[4-[2-(1-pipéridinyl)éthylsulfinyl]phényl]-estra-1,3,5(10)-triène-3,17béta-diol,
- acide 4-[4-chloro-llbéta-[4-[2-(diéthylamino)éthoxy]phényl] 17béta-hydroxy-estra-1,3,5(10)-trièn-3-yl-oxy]-butanoïque,
- oxime de 4-chloro-3-hydroxy-llbéta-[4-[2-(1-pipéridinyl) éthoxy]phényl]-estra-1,3,5(10)-trièn-17-one,
- acide 4-[[2-[4-(4-chloro-3,17béta-dihydroxy-estra-1,3,5(10)-trièn-llbéta-yl)phénoxy]éthyl]éthylamino]-butanoïque.

L'invention a également tout particulièrement pour objet le composé de formule (I) telle que définie ci-dessus, dont le nom suit :

- 4-chloro-11β-[4-[2-(diéthylamino)éthoxy]phényl]-estra-1,3,5(10)-triène-3,17béta-diol, ainsi que ses sels d'addition avec les acides.

L'invention a également pour objet un procédé de préparation des composés de formule générale (I) telle que définie précédemment, caractérisé en ce que l'on soumet un composé de formule générale (II) :

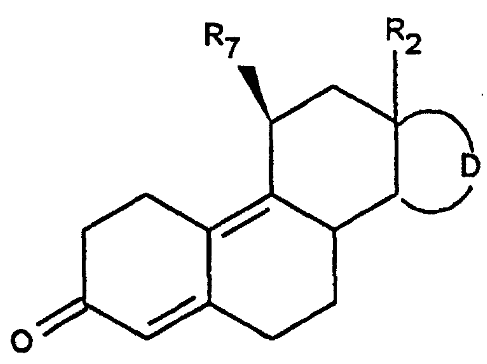

(II)

dans laquelle D et $R_2$ sont tels que définis précédemment, $R_7$ représente un des groupements suivants :

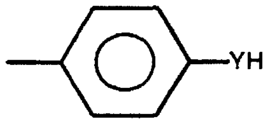
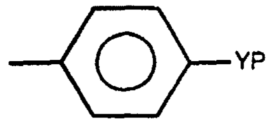
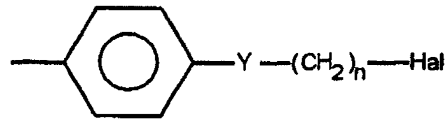

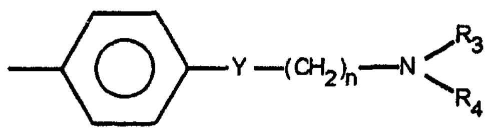

dans lesquels n, Y, $R_3$ et $R_4$ sont tels que définis précédemment, P est un groupement protecteur, Hal représente un halogène,
à l'action d'un réactif d'halogénation afin d'obtenir le composé de formule (III) :

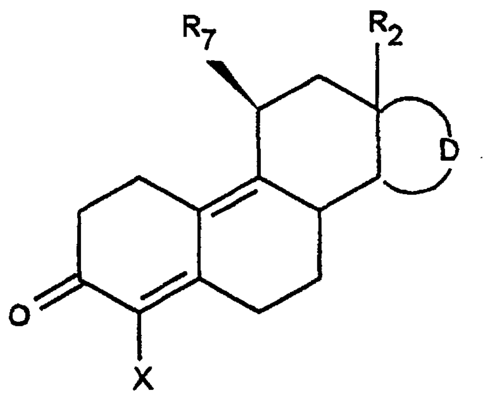

(III)

que l'on soumet à l'action d'un réactif d'aromatisation du cycle A, puis à l'action d'une base afin d'obtenir le composé de formule (IV) correspondant à certains composés de formule générale (I) :

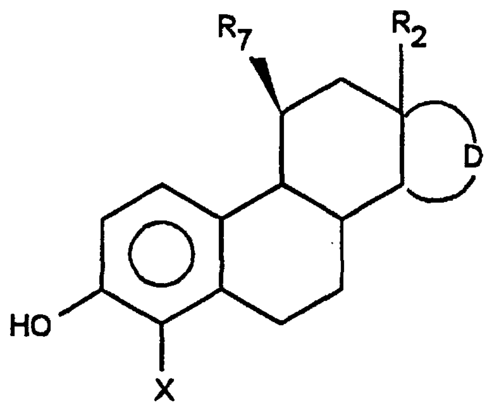

(IV)

composés de formule (II), (III) ou (IV)
que l'on soumet, si désiré et si nécessaire, dans un ordre approprié, à l'une ou plusieurs des réactions suivantes :

- protection des composés dans lesquels $R_7$ est un groupement -Ph-YH,
- déprotection des composés dans lesquels $R_7$ est un groupement Ph-YP,
- action d'un composé de formule $Hal_1$-$(CH_2)_n$-$Hal_2$ sur les composés dans lesquels $R_7$ est un groupement -Ph-YH, $Hal_1$ ou $Hal_2$ identiques ou différents représentant un halogène afin d'obtenir des composés dans lesquels $R_7$ est

un groupement $-Ph-Y-(CH_2)_n-Hal_2$,

- action d'un composé de formule $R_3-NH-R_4$ sur les composés dans lesquels $R_7$ est un groupement $Ph-Y-(CH_2)_n-Hal_2$, afin d'obtenir des composés dans lesquels $R_7$ est un groupement $Ph-Y-(CH_2)_n-NR_3R_4$,
- action d'un sel d'halogénure ($M-Hal_3$) sur les composés dans lesquels $R_7$ est un groupement $Ph-Y-(CH_2)_n-Hal_2$ afin d'obtenir des composés dans lesquels $R_7$ est un groupement $-Ph-Y-(CH_2)_n-Hal_3$,
- protection du groupement OH en position 3 ou 17,
- déprotection du groupement OH en position 3 ou 17,
- alkylation du groupement OH en position 3 ou 17,
- acylation du groupement OH en position 3 ou 17,
- action d'un agent de réduction lorsque D représente le reste d'un cycle pentagonal tel que défini précédemment et $R_5$ et $R_6$ forment ensemble un groupement oxo,
- action d'un organométallique sur les composés de formule (IV) avec D représentant le reste d'un cycle pentagonal tel que défini précédemment et $R_5$ et $R_6$ formant ensemble un groupement oxo,
- action d'un agent de lactonisation sur les composés de formule (IV) avec D représentant le reste d'un cycle pentagonal tel que défini précédemment et $R_5$ et $R_6$ formant ensemble un groupement oxo,
- action d'un agent de réduction de la double liaison, lorsque D représente le reste d'un cycle pentagonal tel que défini précédemment et $R_5$ et $R_6$ forment ensemble avec le carbone qui les porte, un groupement $O-(CH_2)_{n'}-CH=CH-$,
- action d'un agent de réduction de la double liaison, lorsque D représente le reste d'un cycle pentagonal tel que défini précédemment, et $R_6$ est un radical alkényle ou alkynyle renfermant de 2 à 6 atomes de carbone,
- halogénation en position 4, puis aromatisation du cycle A, du composé de formule générale (II),
- aromatisation du cycle A du composé de formule (III),
- salification.

L'action d'un réactif d'halogénation tel que le N-bromosuccinimide ou le N-chlorosuccinimide sur les composés de formule (II) s'effectue notamment en présence d'un solvant aprotique dipolaire tel que le diméthylformamide.

La réaction d'aromatisation suivie de la réaction de saponification (action de la base) s'effectue selon les méthodes classiques telles que décrites dans le brevet européen 0097572. On utilise de préférence un mélange d'anhydride acétique et de bromure d'acétyle comme agent d'aromatisation puis une base telle que la soude dans le méthanol comme agent de saponification.

Les réactions de protection et de déprotection sont les méthodes classiques connues de l'homme du métier. Une revue assez complète se trouve dans l'ouvrage suivant : Protective groups in organic synthesis T.W greene, John Wiley & sons (1981).

Le groupement protecteur P représente de préférence un radical alkyle renfermant de 1 à 4 atomes de carbone, un groupement benzyle, un groupement $R_CR_DR_ESi$, dans lequel $R_C$, $R_D$ et $R_E$ identiques ou différents, indépendamment l'un de l'autre représentent chacun un radical alkyl renfermant de 1 à 4 atomes de carbone ou un groupement phényle. Il s'agit tout particulièrement des groupements $Si(Me)_2CMe_3$ ou $-Si(Ph)_2CMe_3$.

A titre d'exemple, les réactions de déprotection de composés de formule (II), (III) ou (IV) avec $R_7$=Ph-OP ou des composés de formule (IV) dont le 3-OH est protégé (3-OP), lorsque P est un radical méthyle, peuvent s'effectuer par action de tribromoborane dans le dichlorométhane ou d'acide chlorhydrique dans la pyridine, les réactions de déprotection lorsque P est un groupement benzyle peuvent s'effectuer par action d'hydrogène en présence de palladium sur charbon dans l'acétate d'éthyle ou par action d'acide trifluoroacétique, les réactions de déprotection lorsque P est un groupement tertbutyldiphénylsilyle peuvent s'effectuer par action de fluorure de tétrabutyl ammonium en solution dans le tétrahydrofuranne.

Lorsque P est un groupement tétrahydropyrannyle, la déprotection s'effectue en présence d'un acide aqueux dans un solvant alcoolique et de préférence par action de l'acide chlorhydrique dans le méthanol.

L'action d'un composé de formule $Hal_1-(CH_2)_n-Hal_2$ sur un composé de formule (II), (III) ou (IV) dans lequel $R_7$=Ph-YH peut s'effectuer, notamment lorsque Y=O, en présence d'une base dans un solvant tel que l'acétone.

L'action d'un composé de formule $R_3-NH-R_4$ sur les composés dans lesquels $R_7$ est un groupement $Ph-Y-(CH_2)_n-Hal_2$ dans les conditions classiques des substitutions nucléophiles, notamment en présence d'un solvant aprotique tel que le tétrahydrofuranne.

La réaction de substitution d'un halogène par un autre lorsque notamment $R_7$ est un groupement $Ph-Y-(CH_2)_n-Cl$ s'effectue de préférence par action de NaI dans la méthyléthylcétone.

Les réactions d'alkylation ou d'acylation du groupement OH en position 3 ou 17 sont opérées par les méthodes classiques connues de l'homme du métier.

La réduction du 17-céto en alcool correspondant ($R_5$=OH et $R_6$=H) s'effectue selon les méthodes classiques, notamment par action d'un borohydrure alcalin tel que le borohydrure de sodium dans le méthanol ou l'éthanol ou par action de tétrahydrure d'aluminium et de lithium.

L'action d'un organométallique sur le 17-céto permet d'avoir accès aux produits de formule (IV) dans laquelle D représente le reste d'un cycle pentagonal tel que défini précédemment, $R_5$ est un hydroxyle et $R_6$ représente un radical alkyle, alkényle, alkynyle éventuellement substitué.

L'organométallique dérivé d'un alkyle, alkényle ou alkynyle est choisi parmi les magnésiens de formule Alk-MgHal et les lithiens de formule AlkLi dans lesquelles Alk représente un groupement alkyle, alkényle ou alkynyle renfermant au plus 8 atomes de carbone Hal représente un atome d'halogène. Dans un mode préféré d'exécution du procédé, Hal représente un atome de chlore, de brome ou d'iode, de préférence de brome.

De préférence la réaction a lieu en présence du chlorure de cérium. Dans un mode préféré d'exécution du procédé, Hal représente un atome de chlore, de brome ou d'iode, de préférence de brome.

La réaction de lactonisation à partir du 17 céto s'effectue selon la méthode de STURTZ (réf : G. STURTZ et J-J. YAOUANC, Synthesis, (1980), 289) notamment en présence de bisdiméthylamidophosphate d'alkyle en présence d'un alkyllithien tel que le nbutyllithium dans le tétrahydrofuranne.

La réaction de réduction totale ou partielle lorsque $R_6$ est un radical alkényle ou alkynyle ou lorsque $R_5$ et $R_6$ forment ensemble avec le carbone qui les porte, un groupement $O-(CH_2)_{m'}-CH=CH-$, peut s'effectuer soit de manière totale par action d'hydrogène en présence d'un catalyseur tel que le palladium sur charbon ou un catalyseur au rhodium tel que le réactif de Wilkinson soit de manière partielle (alkynyle devient alkényle) par action d'un catalyseur empoisonné tel que le palladium sur sulfate de baryum empoisonné par la pyridine ou la triéthylamine.

Les réactions d'estérification et de salification sont effectuées par les méthodes courantes connues de l'homme du métier.

L'invention a plus particulièrement pour objet un procédé de préparation des composés de formule générale (I') tel que décrit précédemment caractérisé en ce qu'on soumet un composé de formule générale (II') :

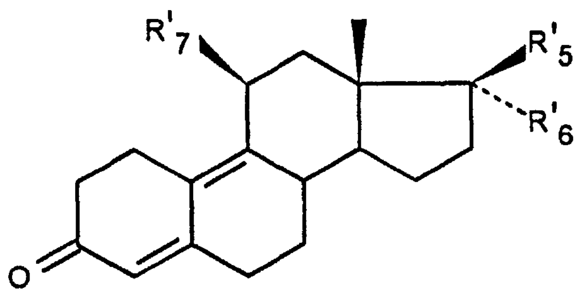

(II')

dans laquelle $R'_5$ et $R'_6$ sont tels que définis précédemment, $R'_7$ représente :

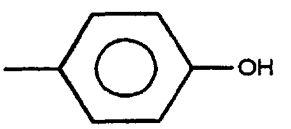

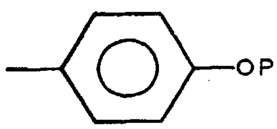

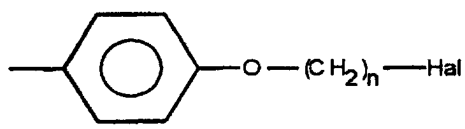

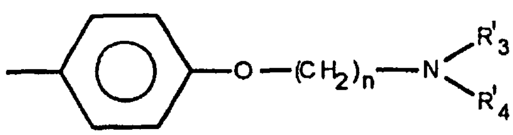

à l'action d'un réactif d'halogénation afin d'obtenir le composé de formule (III') :

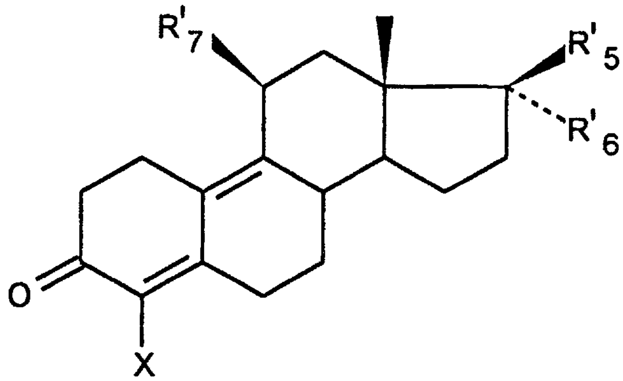

(III')

que l'on soumet à l'action d'un réactif d'aromatisation du cycle A, puis à l'action d'une base afin d'obtenir le composé de formule (IV') correspondant à certains composés de formule générale (I') :

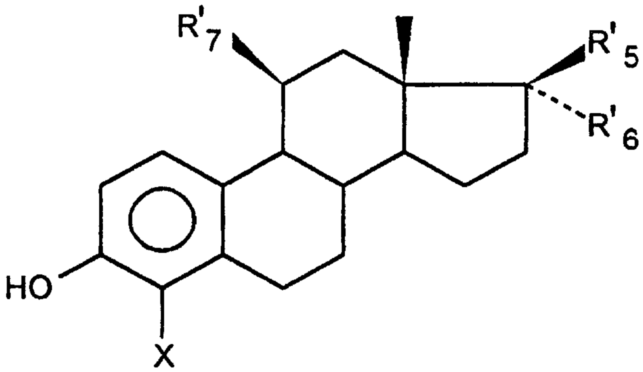

(IV')

composés de formule (II'), (III') ou (IV'), que l'on soumet, si désiré et si nécessaire, dans un ordre approprié, à l'une ou plusieurs des réactions suivantes :

- protection des composés dans lesquels $R'_7$ est un groupement -Ph-OH,
- déprotection des composés dans lesquels $R'_7$ est un groupement Ph-OP,
- action d'un composé de formule $Hal_1$-$(CH_2)_n$-$Hal_2$ sur les composés dans lesquels $R'_7$ est un groupement -Ph-OH, $Hal_1$ ou $Hal_2$ identiques ou différents représentant un halogène afin d'obtenir des composés dans lesquels $R_7$ est un groupement -Ph-O-$(CH_2)_n$-$Hal_2$,
- action d'un composé de formule $R'_3$-NH-$R'_4$ sur les composés dans lesquels $R'_7$ est un groupement Ph-O-$(CH_2)_n$-$Hal_2$, afin d'obtenir des composés dans lesquels $R'_7$ est un groupement Ph-O-$(CH_2)_n$-$NR'_3R'_4$,
- action d'un sel d'halogénure (M-$Hal_3$) sur les composés dans lesquels $R'_7$ est un groupement Ph-O-$(CH_2)_n$-$Hal_2$ afin d'obtenir des composés dans lesquels $R_7$ est un groupement -Ph-O-$(CH_2)_n$-$Hal_3$,
- protection du groupement OH en position 3 ou 17,
- déprotection du groupement OH en position 3 ou 17,
- alkylation du groupement OH en position 17,
- acylation du groupement OH en position 17,
- action d'un agent de réduction lorsque $R'_5$ et $R'_6$ forment ensemble un groupement oxo,
- action d'un organométallique sur les composés de formule (IV') avec $R'_5$ et $R'_6$ formant ensemble un groupement oxo,
- action d'un agent de lactonisation sur les composés de formule (IV') avec $R'_5$ et $R'_6$ formant ensemble un groupement oxo,
- action d'un agent de réduction de la double liaison, lorsque $R'_5$ et $R'_6$ forment ensemble avec le carbone qui les porte, un groupement O-$(CH_2)_{1'}$-CH=CH-,
- action d'un agent de réduction de la double liaison, lorsque $R'_6$ est un radical alkényle ou alkynyle renfermant de 2 à 6 atomes de carbone,
- halogénation en position 4, puis aromatisation du cycle A, du composé de formule (II'),

- aromatisation du composé de formule (III'),
- salification.

Les composés de formule générale (I) ainsi que leurs sels d'addition avec les acides pharmaceutiquement acceptables possèdent des activités oestrogène, antioestrogène et antiprolifératives.
A ce titre, les composés de formule (I) peuvent être utilisés dans le traitement des troubles liés à une hypofolliculinie, par exemple les aménorrhées, les dysménorrhées, les avortements répétés, les troubles prémenstruels, dans le traitement de certaines pathologies estrogèno-dépendantes telles que les adénomes ou les carcinomes prostatiques, les carcinomes mammaires et ses métastases ou le traitement des tumeurs bénignes du sein, en tant qu'anti-utérotrophique ainsi que dans le traitement substitutif de la ménopause ou de la périménopause.
Parmi les symptômes et les conséquences liées à la ménopause, on entend plus précisément les bouffées de chaleur, les sueurs, l'atrophie et la sécheresse vaginale, les symptômes urinaires et à long terme la diminution de la masse osseuse et l'augmentation du risque de fracture, ainsi que la perte de la protection cardio-vasculaire offerte par les oestrogènes.
En particulier, les composés de formule (I) ainsi que leurs sels d'addition avec les acides ou les bases pharmaceutiquement acceptables, peuvent ainsi être utilisés dans la prévention ou le traitement de l'ostéoporose.
Les composés de formule (I) ainsi que leurs sels d'addition avec les acides ou les bases pharmaceutiquement acceptables, peuvent également être utilisés dans la prévention ou le traitement de l'ostéoporose chez l'homme.
Ils peuvent également être utilisés dans la prévention ou le traitement des ostéoporoses secondaires (par exemple cortisoniques, liées à une immobilisation).
Les composés de formule (I) ainsi que leurs sels d'addition avec les acides ou les bases pharmaceutiquement acceptables possèdent notamment une activité estrogénique dissociée.
Par activité estrogénique dissociée, on entend une activité estrogénique au niveau osseux tout en ne manifestant qu'une activité minimale au niveau utérin entraînant ainsi l'absence de prolifération endométriale (activité bien inférieure à celle de l'oestradiol).
Par ailleurs, les composés selon l'invention présentent les avantages suivants :

- Ils présentent une activité anti-oestrogène au niveau du sein. A l'opposé de l'oestradiol ils ne stimulent pas la croissance de cellules tumorales mammaires humaines et même peuvent inhiber leur croissance. Les composés selon l'invention sont donc particulièrement avantageux pour le traitement de la ménopause en ce qui concerne les femmes à risque de cancer mammaire (antécédents familiaux) qui sont donc exclues d'un traitement substitutif par l'oestradiol.

Ils peuvent être également utilisables dans le traitement des cancers mammaires.

- Ils entraînent un abaissement du taux de cholestérol sérique à un niveau équivalent à celui induit par l'estradiol. Ils renforcent ainsi la protection cardiovasculaire.
- Enfin les composés selon l'invention ne présentant aucune activité oestrogène au niveau utérin, ne nécessitent pas d'être administrés en association avec un composé progestomimétique.

L'invention a donc pour objet les composés de formule (I) ainsi que leurs sels d'addition avec les acides ou les bases pharmaceutiquement acceptables, à titre de médicaments.
L'invention a plus particulièrement pour objet les composés de formule (I) ainsi que leurs sels d'addition avec les acides ou les bases pharmaceutiquement acceptables, à titre de médicaments destinés à la prévention ou au traitement de l'ostéoporose.
L'invention s'étend aux compositions pharmaceutiques renfermant comme principe actif au moins l'un des médicaments tels que définis ci-dessus.
Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, d'implants, d'anneaux intravaginal, de patchs, lesquels sont préparés selon les méthodes usuelles.
Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.
La posologie utile varie en fonction de l'affection à traiter et de la voie d'administration ; elle peut varier par exemple de 1 à 1000 mg par jour chez l'adulte par voie orale.
Les composés de formule générale (II) ou (II') sont des composés connus et décrits dans le brevet suivant :

Brevet Européen 0057115.

Les composés de formule générale (II) ou (II') avec

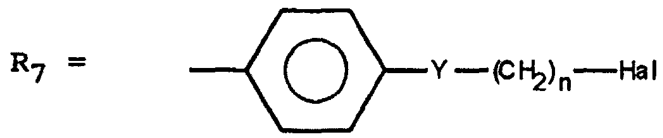

ou

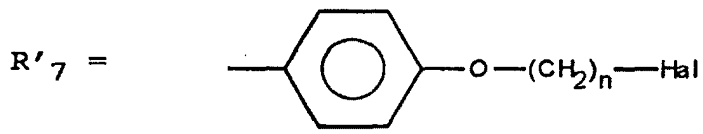

peuvent également être formés à partir des composés de formule (IIa) :

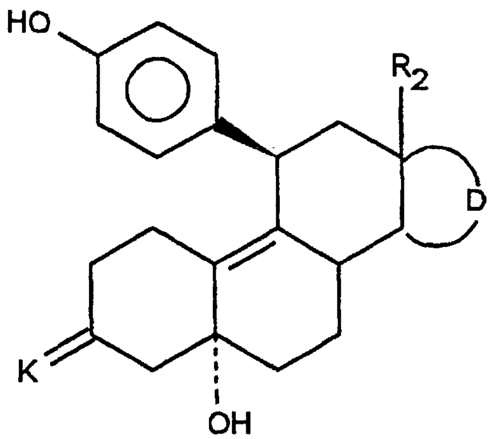

(IIa)

ou (II'a) :

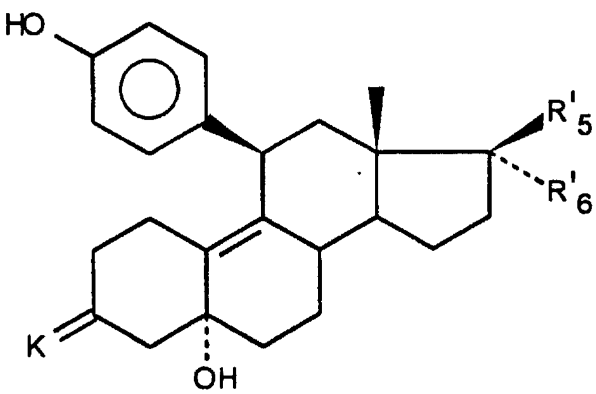

(II'a)

dans lesquelles D, $R_2$, $R'_5$ et $R'_6$ sont tels que définis précédemment et K représente un groupement protecteur de la fonction cétone,

que l'on soumet à l'action d'un réactif de O-alkylation de formule Hal-$(CH_2)_n$-Hal puis à l'action d'un réactif de déshydratation également susceptible de libérer la cétone.

Les composés de formule (IIa) ou (II'a) sont également des composés connus et décrits dans le brevet suivant :

Brevet US n° 5 043 332.

L'invention a également pour objet, à titre de produits intermédiaires, les composés de formule (III), (III'), (IV) et (IV').

Les exemples ci-dessous illustrent l'invention sans toutefois la limiter.

Solvants décrits dans les exemples : AcOEt (acétate d'éthyle), TEA (triéthylamine), $CH_2Cl_2$ (dichlorométhane), $CHCl_3$ (chloroforme), MeOH (méthanol), $NH_4OH$ (hydroxyde d'ammonium), iPrOH (alcool isopropylique).

**EXEMPLE 1 : 4-chloro-3-hydroxy-11béta-[4-[2-(1-pipéridinyl) éthoxy] phényl]-estra-1,3,5(10)-trièn-17-one**

Stade A : llbéta-[4-(2-bromoéthoxy) phényl]-estra-4,9-diène-3,17-dione

a) O-alkylation

On dissout sous atmosphère inerte 8,0 g de 3-(1,2-éthanediyl acétal) cyclique de 5α-hydroxy-11β-(4-hydroxyphényl)-estr-9-èn-3,17-dione, dans 80 ml de 1,2-dibromométhane à 99 %, 21 ml de soude à 50 %, 0,800 g de bromure de tétrabutyl ammonium, et agite au reflux pendant 1 heure.

b) hydrolyse acide

On ajoute à température ambiante 93 ml d'acide chlorhydrique 6M, agite 45 minutes, extrait, lave, sèche et évapore sous pression réduite jusqu'à obtention du produit brut (m = 13,17 g) que l'on recristallise dans un mélange 50 ml de dichlorométhane/50 ml d'éther isopropylique. On obtient 5,96 g + 7,0 g de produit attendu pur (Rf $CH_2Cl_2$/ ACOEt 70/30 = 0,45). F = 208°C.
IR ($CHCl_3$)
1735 $cm^{-1}$ : 17 céto ; 1658 et 1609 $cm^{-1}$ : cétone conjuguée ; 1583 et 1509 : aromatique.

Stade B : 11béta-[4-(2-bromoéthoxy) phényl]-4-chloro-estra-4,9-diène-3,17-dione (introduction du cl en position 4)

A une solution, sous atmosphère inerte, à 60°C, de 5,025 g du produit obtenu au stade A, dans 67 ml de diméthylformamide, on ajoute 1,86 g de N-chlorosuccinimide et agite 10 minutes. On ajoute de l'eau salée, extrait, sèche et évapore sous pression réduite jusqu'à obtention du produit brut (m = 8,149 g) que l'on purifie par chromatographie sur silice en éluant avec un mélange cyclohexane/acétate d'éthyle (60/40), on obtient 5,43 g du produit attendu pur (Rf essence G/ACOEt 60/40 = 0, 35). IR ($CHCl_3$)
1736 $cm^{-1}$ : 17-céto ; 1677 $cm^{-1}$ : cétone conjuguée ; 1609, 1582, 1550 et 1509 $cm^{-1}$ : C=C et aromatique.

Stade C : 11béta-[4-(2-bromoéthoxy) phényl]-4-chloro-3-hydroxy-estra-1,3,5(10)trièn-17-one (aromatisation du cycle A)

a) Aromatisation

A une solution, sous atmosphère inerte, à température ambiante de 4,7 g du produit obtenu au stade B, dans 50 ml de dichlorométhane/siliporite, on ajoute tout un refroidissant, 4,7 ml d'anhydride acétique et 4,7 ml de bromure d'acétyle et agite pendant 5 heures 30.

b) Saponification de l'acétate phénolique

Après évaporation sous pression réduite, du dichlorométhane à température ambiante, on ajoute, tout en refroidissant, sous atmosphère inerte, 47 ml de tétrahydrofuranne, 47 ml de méthanol puis 47 ml de soude, et agite 1 h à température ambiante. Après acidification avec de l'acide chlorhydrique, on extrait, lave, sèche et évapore sous pression réduite jusqu'à obtention du produit brut (m = 4,84 g) que l'on purifie par chromatographie sur silice en éluant avec un mélange cyclohexane/acétate d'éthyle (70/30). On obtient 4,37 g de produit attendu (Rf = essence G/ACOEt 70/30 = 0,18).

IR ($CHCl_3$)
3537 $cm^{-1}$ : OH-phénolique ; 1733 $cm^{-1}$ : 17-céto ; 1609, 1580, 1511 et 1481 $cm^{-1}$ : aromatique.

Stade D : 4-chloro-3-hydroxy-llbéta-[4-(2-iodoéthoxy) phényl]-estra-1,3,5(10)trièn-17-one (ioduration)

A une solution sous atmosphère inerte, à température ambiante, de 4,11 g du dérivé bromé préparé au stade C dans 80 ml de méthyléthyl cétone, on ajoute 2,44 g d'iodure de sodium et agite 1 nuit au reflux. On ajoute de l'eau, extrait, sèche et évapore sous pression réduite jusqu'à obtention de 4,184 g de produit brut attendu (Rf = méthanol/eau (90/10) = 0,30).

Stade E : 4-chloro-3-hydroxy-11béta-[4-[2-(1-pipéridinyl) éthoxy] phényl]-estra-1,3,5(10)trièn-17-one (substitution de l'iode par la pipéridine)

on dissout sous atmosphère inerte, à température ambiante, 1,248 g du dérivé iodé obtenu au stade D, dans 25 ml de tétrahydrofuranne/siliporite, ajoute 1,35 ml de pipéridine et chauffe à reflux 1 heure 30. Après évaporation du tétrahydrofuranne sous pression réduite à température ambiante, on ajoute de l'eau et de l'acétate d'éthyle, lave, sèche et évapore sous pression réduite jusqu'à obtention de 1,185 g dérivé aminé brut que l'on purifie par chromatographie sur silice en éluant avec le mélange acétate d'éthyle/triéthylamine 95/5. On obtient 1,039 g de produit pur attendu (Rf = acétate d'éthyle/TEA (95/5) = 0,20).
RMN $CDCl_3$
0,45 ppm (s) : $CH_3$ en 18 ; 2,48 ppm : $CH_2$-N cycliques, 2,71 ppm (t) : $CH_2$-N de la chaîne ; 3,99 ppm (t) : $CH_2$-OAr ; 3,99 ppm : $H_{11}$ ; 6,63 ppm : $H_2$ ; 6,81 ppm : $H_1$ ; 6,60 ppm : Ar-O ;
6,91 ppm : Ar-C.

**EXEMPLE 2 : 4-chloro-3-hydroxy-11béta-[4-[2-(1-pirrolidinyl) éthoxy] phényl]-estra-1,3,5(10)-trièn-17-one**

On opère comme à l'exemple 1 stade E, mais à partir de 6 g du dérivé iodé obtenu au stade D de l'exemple 1, 60 ml de tétrahydrofuranne/siliporite et 4,6 ml de pyrrolidine.
On obtient 4,2 g de produit pur attendu (Rf : ACOEt/TEA (80/20) = 0,24).
RMN ($CDCl_3$) :
0,45 ppm (s) : $CH_3$ en 18 ; 1,78 ppm (m) : $CH_2$ en béta de N ; 2,59 ppm (m) : $CH_2$ en alpha de N ; 2,84 ppm (t) : $CH_2$-N de la chaîne ; 3,98 ppm (t) : $CH_2$-OAr ; 3,98 ppm (t) : $H_{11}$, $CH_2$-O de la chaîne ; 6,62 ppm : $H_2$ ; 6,81 ppm : $H_1$ ; 6,62 ppm : Ar-O ; 6,91 ppm : Ar-C.

**EXEMPLE 3 : 4-chloro-3-hydroxy-11béta-[4-[2-diéthylamino) éthoxy] phényl]-estra-1,3,5(10)-trièn-17-one**

On opère comme à l'exemple 1 stade E, mais à partir de 1,2 g du dérivé iodé obtenu au stade D de l'exemple 1, 25 ml de tétrahydrofuranne/siliporite et 1 ml de diéthylamine.
On obtient 0,688 g de produit pur attendu (Rf :
ACOEt/TEA (95/5) = 0,22).
RMN ($CDCl_3$) :
0,45 ppm (s) : $CH_3$ en 18 ; 1,03 ppm (t) : N-$CH_2$-$\underline{CH_3}$ ; 2,60 ppm (q) : N-$\underline{CH_2}$-$CH_3$ ; 2,81 ppm (t) : $CH_2$-N de la chaîne ; 3,93 ppm (t) : $CH_2$-OAr ; 4,00 ppm (t) : $H_{11}$ ; 6,63 ppm : $H_2$ ; 6,82 ppm : $H_1$ ; 6,63 ppm : Ar-O ; 6,91 ppm : Ar-C.

**EXEMPLE 4 : 4-bromo-3-hydroxy-11béta-[4-[2-(1-pipéridinyl) éthoxy] phényl]-estra-1,3,5 (10)-trièn-17-one**

Stade A : 11béta-[4-(2-chloroéthoxy) phényl]-estra-4,9-diène-3,17-dione (O-alkylation)

A une suspension de 5 g de 11béta-(4-hydroxyphényl)-estra-4,9-diène-3,17-dione, dans 50 ml d'acétone, sont additionnés (7,9 ml x 2) de 1-bromo-2-chloroéthane suivis de 6 ml de soude 50 % et de 500 mg de bromure de tétrabutylamonium. Le mélange est porté 4 h au reflux. On ajoute de l'eau, extrait, sèche et évapore sous pression réduite jusqu'à obtention d'un produit brut que l'on purifie dans l'éther.
On obtient 5,45 g de produit attendu (Rf $CH_2Cl_2$/acétone 90/10 = 0,82).
RMN ($CDCl_3$) :
0,58 3H (s) : $CH_3$ en 18 ; 3,80 2H (t) : $C\underline{H_2}Cl$ ; 4,28 2H (t) : $C\underline{H_2}O$ ; 4,49 1H (d) : $H_{11}$ ; 5,80 1H (s) : $H_4$ ; 6,82 2H (d) : 2H arom. ; 7,08 2H (d) : H arom.

Stade B : 4-bromo-11béta-[4-(2-chloroéthoxy) phényl]-estra-4,9-diène-3,17-dione (introduction du Br en position 4)

A une suspension de 5,35 g du produit du stade A dans 70 ml de diméthylformamide, sous atmosphère d'azote, on ajoute une solution de 2,43 g de NBromo succinimide. Après agitation 2 heures à température ambiante, on ajoute 200 ml d'acétate d'éthyle et 400 ml d'eau saturée de NaCl, extrait, lave, sèche et évapore sous pression réduite jusqu'à obtention de 8 g du produit brut que l'on purifie par chromatographie en éluant avec un mélange dichlorométhane/acétone 98/2. On obtient 4,66 g de produit pur attendu. (Rf $CH_2Cl_2$/acétone 95/5 = 0,92).
RMN ($CDCl_3$) :
0,57 3H (s) : C$\underline{H}_3$ en 18 ; 3,25 1H (dt) : $H_6$ ; 3,80 2H (t) : C$\underline{H}_2$Cl ; 4,20 2H (t) : C$\underline{H}_2$O ; 4,38 1H (d,large) : $H_{11}$ ; 7,07 et 6,83 4H : AA'BB' H arom.

Stade C : 4-bromo-11béta-[4-(2-chloro éthoxy) phényl]-3-hydroxy-estra-1,3,5(10)-trièn-17-one (aromatisation du cycle A)

On opère comme au stade C de l'exemple 1, à partir de 4,5 g de produit obtenu au stade précédent. On obtient 3,05 g de produit attendu. (Rf $CH_2Cl_2$/ACOEt 90/10 = 0,64).
RMN ($CDCl_3$) :
0,45 3H (s) : C$\underline{H}_3$ en 18 ; 3,75 2H (t) : C$\underline{H}_2$Cl ; 4,12 2H (t) : C$\underline{H}_2$O ; 4,00 1H (t) : $H_{11}$ ; 5,48 1H (s) : OH ; 6,93 et 6,64 2H : AA'BB' 4H arom ; 6,85 et 6,64 2H (d) : $H_1H_2$.

Stade D : 4-bromo-3-hydroxy-llbéta-[4-(2-iodo éthoxy) phényl]-estra-1,3,5(10)-trièn-17-one (ioduration)

On opère comme stade D de l'exemple 1, à partir de 1 g du produit obtenu au stade précédent. On obtient 1,14 g de produit attendu.

Stade E : 4-bromo-3-hydroxy-llbéta-[4-[2-(l-pipéridinyl) éthoxy] phényl]-estra-1,3,5(10)-trièn-17-one (substitution de l'iode par la pipéridine)

On opère comme au stade E de l'exemple 1 à partir de 1,1 g de produit obtenu au stade précédent. On obtient 270 mg de produit pur attendu (Rf ACOEt/TEA 90/10 = 0,43).
RMN ($CDCl_3$) :
0,45 3H (s) : C$\underline{H}_3$ en 18 ; 2,47 4H (m) : C$\underline{H}_2$N cyclique ; 2,70 2H (t) : C$\underline{H}_2$-N ; 3,98 2H (t) : C$\underline{H}_2$-O ; 3,98 1H (t large) : $H_{11}$ ; 6,88 et 6,62 2H (d) : $H_1$ et $H_2$ ; 6,90 et 6,62 4H : AA'BB' H aromatique.

**EXEMPLE 5 : 4-chloro-llbéta-[4-[3-(1-pipéridinyl) propoxy] phényl]-estra-1,3,5(10)-triène-3,17-béta-diol**

Stade A : 4-chloro-11béta-[4-(phénylméthoxy) phényl]-estra-4,9-diène-3,17-dione (chloration en 4)

A une solution, sous atmosphère inerte, à 60°C, de 13,6 g de 11β-[4-(phénylméthoxy)phényl]-estra-4,9-diène-3,17-dione dans 120 ml de diméthylformamide, on ajoute 5,295 g de N-chlorosuccinimide, agite 10 minutes puis coule dans une solution aqueuse saturée de NaCl, extrait, lave, sèche et évapore sous pression réduite jusqu'à obtention du produit brut (m = 19,128 g). On opère un deuxième essai, réunit les produits bruts et purifie par chromatographie en éluant avec le mélange acétate d'éthyle-cyclohexane 30/70. On obtient 23,28 g de produit pur attendu. (Rf ACOEt/cyclohexane 30/70 = 0,19).
RMN ($CDCl_3$) :
0,58 (s) : C$\underline{H}_3$ en 18 ; 3,25 (dt) : 1H équatorial $H_6$ ; 4,39 (dl) : $H_{11}$ ; 5,02 (s) : C$\underline{H}_2$Ph ; 6,89 : H en ortho de Ph-O ; 7,06 : H en méta de Ph-O ; 7,29 à 7,45 : H aromatique du $CH_2$-Ph.

Stade B : 4-chloro-3-[[(2,2-diméthyléthyl) (diphényl)silyl] oxy] 11-béta-[4-(phénylméthoxy) phényl]-estra-1,3,5(10)-trièn-17-one (aromatisation/saponification/blocage du phénol)

a) aromatisation et saponification

On opère comme au stade C de l'exemple 1 à partir du produit obtenu au stade précédent. On obtient 13,5 g de produit pur attendu (3-OH).

b) blocage du phénol

On dissout sous atmosphère inerte 13,5 g + 5,6 g de produit obtenu au stade précédent, 191 ml de dichloro méthane/siliporite, 14,5 ml de terbutyl diphényl chlorosilane et 258 mg de 4-DMAP, et on agite 23 heures au reflux. On coule ensuite dans l'eau, extrait, lave, sèche et évapore sous pression réduite jusqu'à obtention de 41,322 g de produit brut sous forme d'huile que l'on purifie par chromatographie en éluant avec le mélange Acétate d'éthyle/éther de pétrole 20/80 puis 40/60. On obtient 1,275 g de produit pur attendu. (Rf ACOEt/éther de pétrole 20/80 = 0,27).
RMN ($CDCl_3$) :
0,42 (s) : C$\underline{H}_3$ en 18 ; 1,11 (s) : C(C$\underline{H}_3$)$_3$ ; 7,25 à 7,40 - 7,60 à 7,75 Si-Ph ; 3,85 (t) : $H_{11}$ ; 4,94 (s) : C$\underline{H}_2$-Ph ; 6,65 : H en ortho (Ph-O) ; 6,84 : H en para (Ph-O) ; 6,17 (d) : $H_1$ ; 6,47 (d) : $H_2$.

Stade C : 4-chloro-3-[[(2,2-diméthyléthyl) (diphényl)silyl] oxy] 11-béta-[4-(hydroxyphényl)-estra-1,3,5(10)-trièn-17-one (déprotection (débenzylation))

On ajoute à une suspension, sous atmosphère inerte, à température ambiante, de 20,82 g du produit obtenu au stade précédent dans 420 ml de méthanol, 15,6 g d'hydroxyde de palladium sur magnésie et 40 ml de 1,4-cyclohexadiène, puis porte au reflux pendant 8 heures. Après filtration et évaporation sous pression réduite, on obtient 22 g de produit brut que l'on purifie par chromatographie en éluant avec un mélange cyclohexane/acétate d'éthyle 7/3. On obtient 19,4 g de produit pur attendu. (Rf cyclohexane/ACOEt 7/3 = 0,27). RMN ($CDCl_3$) :
0,42 (s) : C$\underline{H}_3$ en 18 ; 1,11 (s) : C(C$\underline{H}_3$)$_3$ ; 3,83 (tl) : $H_{11}$ ; 4,56 (s) : O$\underline{H}$ ; 6,17 (d) - 6,46 (d) : $H_1$, $H_2$ ; 7,25 à 7,43 (m) 6H et 7,64 (m) 4H : $SiPh_2$.

Stade D : 4-chloro-3-[[(2,2-diméthyléthyl) (diphényl)silyl] oxy] 11-béta-[4-(3-iodopropoxy) phényl]-estra-1,3,5(10)-trièn-17-one (O-alkylation)

On mélange pendant 4 heures, à température ambiante 3,18 g du produit obtenu au stade précédent, 15 ml de 1,3-diodopropane, 800 mg de soude broyée et 300 mg de bromure de tétrabutylammonium, acidifie par de l'acide chlorhydrique 2N, extrait, lave, sèche et évapore sous pression réduite jusqu'à obtention du produit brut (39,8 g) que l'on purifie par chromatographie en éluant avec le mélange éther de pétrole/ACOEt 75/25. On obtient 2,43 g de produit pur attendu. (Rf : éther de pétrole/AcOEt 80/20 = 0,3).
RMN ($CDCl_3$) :
0,41 (s) : C$\underline{H}_3$ en 18 ; 1,10 (s) : C(C$\underline{H}_3$)$_3$ ; 3,34 (t) : O-$CH_2$-$CH_2$-C$\underline{H}_2$-I ; 3,85 (tl) : $H_{11}$ ; 3,91 (t) : O-C$\underline{H}_2$-$CH_2$-$CH_2$-I ; 6,16 (d) - 6,46 (d) : $H_1$, $H_2$ ; 7,25 à 7,45 6H et 7,66 4H : $SiPh_2$.

Stade E : 4-chloro-3-[[(2,2-diméthyléthyl) (diphényl)silyl] oxy] 11-béta-[4-[3-(1-pipéridinyl) propoxy] phényl]-estra-1,3,5 (10)-trièn-17-one (substitution de l'iode par la pipéridine)

On opère comme au stade E de l'exemple 1, à partir du produit obtenu au stade précédent. On obtient 2,07 g du produit pur attendu (Rf AcOEt/TEA 98/2 = 0,23).
RMN ($CDCl_3$) :
0,42 (s) : C$\underline{H}_3$ en 18 ; 1,10 (s) : C(C$\underline{H}_3$)$_3$ ; 2,52 : $CH_2$-N cyclique ; 2,83 : $CH_2$-N de la chaîne ; 3,84 (tl) : $H_{11}$ ; 3,88 (t) : $CH_2$-O ; 6,16 (d) - 6,47 (d) : $H_1$, $H_2$ ; 6,56 - 6,80 : Ph-O ; 7,25 - 7,40 6H et 7,65 4H : $SiPh_2$.

Stade F : 4-chloro-11béta-[4-[3-(1-pipéridinyl) propoxy] phényl]-estra-1,3,5(10)-triène-3,17-béta-diol (réduction 17-céto et déprotection)

a) Réduction du 17-céto

A une solution sous gaz inerte, à température ambiante de 600 mg du produit obtenu au stade précédent dans 4 ml de méthanol et 2 ml de tétrahydrofuranne, on ajoute, tout en refroidissant au bain de glace, 63 mg de borohydrure de sodium à 97 % et agite 50 minutes. On rajoute de l'acétate d'éthyle, lave à l'eau salée, sèche et évapore sous pression réduite jusqu'à obtention de 614 mg du produit brut attendu.

b) Déprotection du phénol en 3

A une solution sous gaz inerte, à température ambiante de 614 mg du produit obtenu précédemment dans 6 ml de tétrahydrofuranne, on ajoute 1,6 ml d'une solution de fluorure de tétrabutyle ammonium, dans le tétrahydrofuranne et agite 50 minutes à température ambiante. On verse dans de l'eau, extrait, lave, sèche et évapore sous

pression réduite jusqu'à obtention de 840 mg de produit brut que l'on purifie par chromatographie en éluant avec le mélange dichlorométhane/méthanol/hydroxyle d'ammonium 90/10/1. On obtient 215 g de produit pur attendu.
(Rf $CH_2Cl_2$/MeOH/$NH_4$OH 90/10/1 = 0,3).
RMN ($CDCl_3$) :
0,38 (s) : C<u>H</u>$_3$ en 18 ; 2,45 (m) : $CH_2$-N ; 3,72 (t) : $H_{17}$ ; 3,86 : $H_{11}$, $CH_2$-O ; 6,61 - 6,93 : Ph-O ; 6,61 - 6,75 : $H_1$, $H_2$.

**<u>EXEMPLE 6 :</u> 4-chloro-llbéta-[4-[4-(1-pipéridinyl) butoxy] phényl]-estra-1,3,5(10)-triène-3,17-béta-diol**

On opère de la même façon qu'à l'exemple 5, l'O-alkylation pouvant s'effectuer :

<u>soit</u> avec le 4-chloro-1-butanol, par la réaction de "Mitsunobu" en présence de triphénylphosphine, diéthylazodicarboxylate dans le tétrahydrofuranne et le produit chloré obtenu étant transformé en produit iodé selon le procédé décrit dans l'exemple 1 stade D,
<u>soit</u> par action directe avec le 1,4-diiodobutane (cf ex. 6). On obtient 189 mg de produit pur attendu (Rf $CH_2Cl_2$/MeOH/$NH_4$OH 90/10/1 = 0,24).

**<u>EXEMPLE 7 :</u> 4-chloro-11béta-[4-[5-(1-pipéridinyl) pentyloxy] phényl]-estra-1,3,5(10)-triène-3,17-béta-diol**

On opère comme à l'exemple 5, l'O-alkylation s'effectuant avec le 1,5-diiodopentane. On obtient 125 mg de produit pur attendu. (Rf $CH_2Cl_2$/MaOH/$NH_4$OH 90/10/1 = 0,30).

**<u>EXEMPLE 8</u> : (17béta) 4-chloro-llbéta-[4-[2-(1-pipéridinyl) éthoxy] phényl]-spiro-[estra-1,3,5(10)-triène-17,2' (5'H) furan]-3-ol**

<u>Stade A :</u> (17béta) 4-chloro-llbéta-(4-hydroxyphényl) spiro-[estra-4,9-diène-17,2'(5'H)-furan]-3-one (chloration)

A une suspension de 10,46 g de (17béta)-11béta-(4-hydroxy phényl)-spiro-[estra-4,9-diène-17,2'(5'H)-furan]-3-one (WO 87/05908) dans 100 ml de diméthylformamide, sous atmosphère inerte, à 60°C, on ajoute 4,51 g de N-chloro succinimide et laisse réagir 10 minutes sous agitation. On verse ensuite sur une solution glacée de chlorure de sodium, extrait, sèche et évapore sous pression réduite jusqu'à obtention de 20,85 g de produit brut. On rajoute 8,93 g de produit obtenu dans un essai annexe mené à l'identique et on purifie l'ensemble par chromatographie en éluant avec un mélange $CH_2Cl_2$/acétone 95/5, puis recristallisation dans l'éther éthylique. On obtient 0,98 g de produit pur attendu. (Rf $CH_2Cl_2$/acétone 95/5 = 0,2). F = 258°C.

<u>Stade B</u> : (17béta)-4-chloro-11béta-[4-(2-bromoéthoxy) phényl]-spiro-[estra-4,9-diène-17,2'(5'H) furan]-3-one

On opère comme à l'exemple 1 stade A, mais à partir de 1,2-dibromoéthane. On obtient 5,34 g de produit pur attendu. (Rf essence G/AcOEt 75/25 = 0,21).

<u>Stade C :</u> Aromatisation et saponification

<u>Stade D :</u> Ioduration

<u>Stade E :</u> Condensation de la pipéridine

Les stades C, D et E s'effectuent de manière analogue aux stades C, D et E de l'exemple 1.
On obtient 0,657 g de produit attendu pur (Rf AcOEt/TEA 92/8 = 0,21).
RMN ($CDCl_3$) :
0,48 (s) : C<u>H</u>$_3$ en 18 ; 2,47 (m) : $CH_2$-N cyclique ; 2,70 (t) : $CH_2$-N chaîne ; 3,89 (tl) : $H_{11}$ ; 3,99 (t) : $CH_2$-O chaîne ; 4,56 : $CH_2$-O cycle ; 5,78 : OH ; 5,87 : H'$_3$, H'$_4$ ; 6,60 - 6,80 : $H_1$ et $H_2$ ; 6,60 - 6,86 : Ph-O.

**<u>EXEMPLE 9</u> : (17béta) 4-chloro-11béta-[4-[2-(diéthylamino) éthoxy] phényl]-spiro-[estra-1,3,5(10)-triène-17,2' (5'H) furan]-3-ol**

On opère comme à l'exemple 8 mais avec condensation finale de diéthylamine sur le dérivé iodé. On obtient 0,512 g de produit attendu pur (Rf $CH_2Cl_2$/MeOH/NH4OH 93/7/0,2 = 0,29).
RMN ($CDCl_3$) :
0,48 (s) : $CH_3$ en 18 ; 1,03 (t) : $CH_2$-C<u>H</u>$_3$ ; 2,60 (q) : C<u>H</u>$_2$-$CH_3$ ; 2,81 (t) : $CH_2$-N ; 3,89 (tl) : $H_{11}$ ; 3,93 (t) : C<u>H</u>$_2$-O chaîne ;

4,56 : $CH_2$-O cycle (H'$_3$) ; 5,87 : H'$_3$, H'$_4$ ; 6,61 - 6,79 : $H_1$ et $H_2$ ; 6,61 - 6,86 : Ph-O.

**EXEMPLE 10 : (17béta) 4-chloro-11béta-[4-[2-(1-pyrrolidinyl) éthoxy] phényl]-spiro-[estra-1,3,5(10)-triène-17,2' (5'H) furan]-3-ol**

On opère comme à l'exemple 8 mais avec condensation finale de la pyrrolidine sur le dérivé iodé. On obtient 0,628 g de produit attendu. F = 226-227°C. (Rf $CH_2Cl_2$/MeOH/$NH_4$OH 93/7/0,2 = 0,25).
RMN ($CDCl_3$) :
0,48 (s) : $CH_3$ en 18 ; 2,59 (m) : $CH_2$-N cyclique ; 2,80 (m) : $CH_2$-N chaîne ; 3,89 (tl) : $H_{11}$ ; 3,98 (t) : $CH_2$-O de la chaîne ; 4,56 (m) : $CH_2$-O cyclique (H'$_5$) ; 5,87 (m) : H'$_3$, H'$_4$ ; 6,60 - 6,78 (d) : $H_1$ et $H_2$ ; 6,60 - 6,86 AA'BB' : Ph-O.

**EXEMPLE 11 : (17béta) 4-chloro-11béta-[4-[3-(1-pipéridinyl) propoxy] phényl]-spiro-[estra-1,3,5(10)-triène-17,2' (5'H) furan]-3-ol**

On opère comme à l'exemple 8, l'O-alkylation s'effectuant directement avec le 1,3-diiodopropane (évite le stade D de l'exemple 8). On obtient 0,591 g de produit attendu pur (Rf AcOEt/TEA 92/8 = 0,19).
RMN ($CDCl_3$) :
0,48 (s) : $CH_3$ en 18 ; 2,30 à 2,50 : $CH_2$-N ; 3,87 (m) : $CH_2$-O chaîne, $H_{11}$ ; 4,56 : $CH_2$-O cycle (H'$_5$) ; 5,88 : H'$_3$, H'$_4$ ; 6,61 - 6,79 : $H_1$ et $H_2$ ; 6,61 - 6,86 : Ph-O.

**EXEMPLE 12 : (17béta) 4-chloro-llbéta-[4-[4-(1-pipéridinyl) butoxy] phényl]-spiro-[estra-1,3,5(10)-triène-17,2' (5'H) furan]-3-ol**

On opère comme à l'exemple 8 stades A, B, C, D, E, l'O-alkylation s'effectuant avec le 1-bromo-4-chlorobutane. On obtient 0,494 g de produit pur.
Rf AcOEt/TEA 95/5 = 0,22
F = 154°C
RMN ($CDCl_3$) :
0,50 (s) : $CH_3$ en 18 ; 2,36 (6H) : C$\underline{H_2}$-N cycle, chaîne ; 3,85 (t) : C$\underline{H_2}$-O ; 3,89 (t) : $H_{11}$ ; 4,57 (s) : $CH_2$-O cyclique (H'$_5$) ; 6,60 (m) (3H) - 6,86 (m) (2H) : Ph-O et $H_2$ ; 6,79 (d) : $H_1$.

**EXEMPLE 13 : 4-chloro-3-hydroxy-11béta-[4-[2-(1-diméthylamino) éthoxy] phényl]-estra-1,3,5(10)-trièn-17-one**

Stade A : 4-chloro-llbéta-[4-[2-(diméthylamino) éthoxy] phényl]-estra-4,9-diène-3,17-dione (introduction du 4-chloro)

A une solution sous atmosphère inerte, à température ambiante, de 1,08 g de 11β-[4-(2-(diméthylamino) éthoxy) phényl]-estra-4,9-diène-3,17-dione dans 11 ml de pyridine, on ajoute 6 ml de chlorure de sulfuryle à 10 % dans le dichlorométhane et agite 30 minutes à environ -36°C. On verse sur du bicarbonate de sodium, extrait, lave, sèche et évapore sous pression réduite jusqu'à obtention de 1,84 g de produit brut, que l'on purifie par chromatographie, en éluant avec le mélange acétate d'éthyle/triéthylamine 80/20. On obtient 616 mg de produit pur attendu. (Rf AcOEt/TEA 8/2 = 0,35). Stade B : 4-chloro-llbéta-[4-[2-(diméthylamino) éthoxy] phényl]-3-hydroxy-estra-1,3,5(10)-trièn-17-one (aromatisation et saponification)
La réaction d'aromatisation puis de saponification s'effectuant comme à l'exemple 1, stade C, à partir de 700 mg du produit obtenu au stade précédent. On obtient 360 mg de produit pur attendu. F = 254°C (Rf $CH_2Cl_2$/iPrOH/$NH_4$OH 93/7/0,7 = 0,18).
RMN ($CDCl_3$) :
0,44 (s) : $CH_3$ en 18 ; 2,30 (s) : $NMe_2$ ; 2,67 (m) : $CH_2$-N ; 3,94 (m) : $CH_2$-O ; 4,00 : $H_{11}$ ; 6,62 - 6,91 : Ph-O ; 6,62 - 6,81 (d) : $H_1$, $H_2$.
Le composé de l'exemple 3 a été préparé de la même manière.

**EXEMPLE 14 : gamma lactone de l'acide 4-chloro-3,17béta-dihydroxy-11béta-[4-[2-(1-pyrrolidinyl) éthoxy] phényl]-19-nor-17alpha-pregna-1,3,5(10)-triène-21-carboxylique**

A 5,93 ml de n-butyllithium à 15 % dans l'hexane, sous atmosphère inerte, à température ambiante, on ajoute 6 ml de tétrahydrofuranne/siliporite, puis à -50°C, 0,921 g de bis-diméthylamido phosphate d'allyle en solution dans le tétrahydrofuranne, enfin à -30°C, 0,586 g du produit obtenu à l'exemple 2, et on agite 1 heure 45 à température ambiante.
On ajoute 8 ml d'acide chlorhydrique 2N, 50 ml d'une solution saturée de bicarbonate de sodium, extrait, lave,

sèche et évapore sous pression réduite jusqu'à obtention de 0,590 g de produit brut que l'on purifie par chromatographie en éluant avec le mélange acétate d'éthyle/triéthylamine 60/40, puis recristallisation dans l'isobutanol. On obtient 0,162 g de produit attendu pur. F = 231°C.
Rf AcOEt/TEA 60/40 = 0,20.
RMN ($CDCl_3$) :
0,51 (s) : $CH_3$ en 18 ; 1,79 (m) : $CH_2$ en bêta de N cycle ; 2,58 (m) : $CH_2$ en alpha de N cycle ; 2,83 (t) : $CH_2$-N de la chaîne ; 3,99 (t) : $CH_2$-OAr ; 3,99 (t) : $H_{11}$ ; 6,62 : $H_2$ ; 6,82 : $H_1$ ; 6,62 : ArO ; 6,82 : ArC.

**EXEMPLE 15 : gamma lactone de l'acide 4-chloro-3,17béta-dihydroxy-11béta-[4-[2-(1-pipéridinyl) éthoxy] phényl]-19-nor-17alpha-pregna-1,3,5(10)-triène-21-carboxylique**

On opère comme à l'exemple 14, mais à partir de 1,179 g du produit de l'exemple 1. On obtient 0,388 g du produit attendu pur. (Rf $CH_2Cl_2$/iPrOH/$NH_4OH$ (95/5/0,5 = 0,20)).
RMN ($CDCl_3$) :
0,52 (s) : $CH_3$ en 18 ; 2,50 (m) : $CH_2$-N cyclique ; 2,71 (t) : $CH_2$-N de la chaîne ; 4,00 (t) : $CH_2$-OAr ; 4,00 (t masqué) : $H_{11}$ ; 6,62 : $H_2$ ; 6,81 : $H_1$ ; 6,62 : ArO ; 6,85 : ArC.

**EXEMPLE 16 : gamma lactone de l'acide 4-chloro-3,17béta-dihydroxy-11béta-[4-[2-(diéthylamino) éthoxy] phényl]-19-nor-17alpha-pregna-1,3,5(10)-triène-21-carboxylique**

On opère comme à l'exemple 14, mais à partir de 0,428 g du produit de l'exemple 3. On obtient 0,195 g du produit attendu pur. (Rf AcOEt/TEA/Essence G (50/30/20 = 0,25))
RMN ($CDCl_3$) :
0,51 (s) : $CH_3$ en 18 ; 1,03 (t) : N-$CH_2$-$C\underline{H}_3$ ; 2,60 (q) : N-$C\underline{H}_2$-$CH_3$ ; 2,81 (t) : $CH_2$-N de la chaîne ; 3,94 (t) : $C\underline{H}_2$-O-Ar ; 3,98 (t) : $H_{11}$ ; 6,59 : $H_2$ ; 6,79 : $H_1$ ; 6,59 : ArO ; 6,85 : ArC.

**EXEMPLE 17 : gamma lactone de l'acide(17-alpha)-4-chloro-3,17béta-dihydroxy-11béta-[4-[3-(1-pipéridinyl) propoxy] phényl]-19-nor-pregna-1,3,5(10)-triène-21-carboxylique**

Stade A : Lactonisation

On opère comme à l'exemple 14, mais à partir de 1,44 g du composé de l'exemple 5 stade E. On obtient 2,36 g de produit brut que l'on engage directement dans la réaction de déprotection du phénol en 3.

Stade B : Déprotection du phénol en 3.

A une solution de 2,36 g du produit obtenu au stade précédent dans 24 ml de tétrahydrofuranne, on ajoute 3,8 ml de solution fluorure de tétrabutyl ammonium dans le tétrahydrofuranne et agite 50 minutes à température ambiante. On verse dans l'eau, extrait, sèche et évapore sous pression réduite jusqu'à obtention de 695 mg de produit brut que l'on purifie par chromatographie en éluant avec le mélange dichlorométhane/méthanol 93/7, puis par recristallisation. On obtient 238 mg de produit pur attendu. F = 242°C. Rf $CH_2Cl_2$/MeOH/$NH_4OH$ 93/7/0,7 = 0,28.
RMN ($CDCl_3$) :
0,51 (s) : $CH_3$ en 18 ; 2,30 à 2,60 : $CH_2$-N ; 3,88 : $CH_2$-O ; 3,98 (tl) : $H_{11}$ ; 6,61, 6,87 : Ar-O, Ar-C ; 6,61 - 6,76 : $H_1$, $H_2$.

**EXEMPLE 18 : gamma lactone de l'acide 4-chloro-3,17béta-dihydroxy-llbéta-[4-[4-(1-pipéridinyl) butoxy] phényl]-19-nor-17alpha-pregna-1,3,5(10)-triène-21-carboxylique**

On opère comme à l'exemple 17, mais à partir de 1,2 g du produit de l'exemple 6 stade E. On obtient 310 mg de produit pur attendu (Rf $CH_2Cl_2$/MeOH/$NH_4OH$ 95/5/0,5 = 0,17).
RMN ($CDCl_3$) :
0,51 (s) : $CH_3$ en 18 ; 3,83 (t) : $CH_2$-O chaîne ; 3,99 (tl) : $H_{11}$ ; 6,61 - 6,85 : Ar-O, Ar-C ; 6,61 - 6,79 : $H_1$, $H_2$.

**EXEMPLE 19 : gamma lactone de l'acide 4-chloro-3,17béta-dihydroxy-11béta-[4-[5-(1-pipéridinyl) pentyloxy] phényl]-19-nor-17alpha-pregna-1,3,5(10)-triène-21-carboxylique**

On opère comme à l'exemple 17, mais à partir de 1,44 g du produit de l'exemple 7 stade E. On obtient 330 mg de produit pur attendu (Rf AcOEt/TEA 90/10 = 0,22).
RMN ($CDCl_3$) :

0,52 (s) : $CH_3$ ; 3,83 (t) : $CH_2$-O ; 3,99 (t) : $H_{11}$ ; 6,60 (m) 3H, 6,67 (d) $H_1$, 6,85 (d) 2H : Aromatiques $H_1$, $H_2$.

**EXEMPLE 20 : 4-chloro-llbéta-[4-[2-(diéthylamino) éthoxy] phényl]-estra-1,3,5(10)-triene-3,17béta-diol**

A une solution sous atmosphère inerte, de 241 mg du composé de l'exemple 3, dans 4 ml de méthanol, on ajoute 37 mg de borohydrure de sodium, agite 1 heure au bain de glace, puis ajoute 2 ml d'acide chlorhydrique puis coule dans une solution aqueuse de bicarbonate de sodium, extrait à l'acétate d'éthyle, lave, sèche et évapore sous pression réduite jusqu'à obtention de 245 mg de produit brut que l'on purifie par chromatographie en éluant avec un mélange dichlorométhane/ méthanol/ hydroxyde d'ammonium 90/10/1. On obtient 195 mg de produit pur attendu. Rf $CH_2Cl_2$/MeOH/$NH_4OH$ 90/10/1 = 0,27.
RMN ($CDCl_3$) :
0,32 (S) : $CH_3$ ; 1,03 (t) : $CH_2C\underline{H}_3$ ; 2,60 (q) : $C\underline{H}_2CH_3$ ; 2,81 (t) : $CH_2$-N ; 3,68 : $H_{17}$ ; 3,93 (t) : $CH_2$-O ; 6,62 : ArO ; 6,89 : ArC ; 6,80 (d) : $H_1$ : 6,62 (d) : $H_2$.

De manière analogue à l'exemple 20, on obtient les produits de formule (I') avec $R'_5$ = OH et $R'_6$ = H suivants :

| | Produit de départ | n' | X' | $NR'_3R'_4$ | Rf s. |
|---|---|---|---|---|---|
| Ex. 21 | Ex. 4 | 2 | Br | Pipéridino | 0,45 AcOEt/TEA 90/10 |
| Ex. 22 | Ex. 1 | 2 | Cl | Pipéridino | 0,13 AcOEt/TEA 95/5 |
| Ex. 23 | Ex. 2 | 2 | Cl | Pyrrolidino | 0,13 $CH_2Cl_2$/MeOH $NH_4OH$ 93/7/0,5 |
| Ex. 24 | Ex. 13 | 2 | Cl | $NMe_2$ | 0,25 $CH_2Cl_2$/ iPrOH/$NH_4OH$ 90/10/1 |

**EXEMPLE 25 : 4-chloro-11béta-[4-[2-(diéthylamino) éthoxy] phényl]-19-nor-17-alpha-pregna-1,3,5(10)-trièn-20-yne-3,17béta-diol**

A une solution sous atmosphère inerte, de 250 mg de produit de l'exemple 3, dans 3 ml de tétrahydrofuranne/ siliporite, on ajoute 4,7 ml d'une solution 0,43 M/l d'adétylure de potassium, agite 1 heure à température ambiante, puis on ajoute 4 ml d'acide chlorhydrique. On coule dans une solution aqueuse saturée de bicarbonate de sodium, extrait, lave, sèche et évapore sous pression réduite jusqu'à obtention de 260 mg de produit brut que l'on purifie par chromatographie en éluant avec le mélange $CH_2Cl_2$/MeOH/$NH_4OH$ 90/10/0,5. On obtient 215 mg de produit pur attendu.
Rf $CH_2Cl_2$/MeOH/$NH_4OH$ 90/10/0,5 = 0,31.
RMN ($CDCl_3$) :
0,43 (s) : $CH_3$ en 18 ; 1,04 (t) : $CH_2C\underline{H}_3$ ; 2,62 (q) : $C\underline{H}_2CH_3$ ; 2,64 (s) : C≡C-$\underline{H}$ ; 2,83 (t) : $CH_2$-N ; 3,95 (t) : $CH_2$-O ; 4,00 (t1) : $H_{11}$ ; 6,62 : Ph-O ; 6,90 : Ph-C ; 6,82 (d) : $H_1$ ; 6,62 (d) : $H_2$.

**EXEMPLE 26 : (17béta)-4-chloro-4',5'-dihydro-11béta-[4-[2-(1-pipéridinyl) éthoxy] phényl]-spiro[estra-1,3,5(10)-triène-17,2'-(3'H)-furan]-3-ol**

A une solution de 0,411 g du produit de l'exemple 8 dans 15 ml d'éthanol et 5 ml de tétrahydrofuranne, on ajoute 0,041 g de catalyseur Pd/C 9,5 % et on hydrogène pendant 2 H 30 (vol. de $H_2$ absorbé = 17,5 $cm^3$). Après filtration du catalyseur, on évapore sous pression réduite jusqu'à obtention de 0,42 g de produit brut que l'on purifie par chromatographie en éluant avec le mélange acétate d'éthyle/triéthylamine 92/8. On obtient 0,33 g de produit pur attendu. F = 170°C. Rf AcOEt/TEA 92/8 = 0,21.

De manière analogue à l'exemple 26, on obtient les produits de formule (I'), dans laquelle $R'_3$ et $R'_6$ forment ensemble avec le carbone qui les porte le cycle saturé

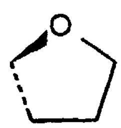 ,

suivants

| | Produit de départ | n' | X' | $NR'_3R'_4$ | Rf s. |
|---|---|---|---|---|---|
| Ex. 27 | Ex. 11 | 3 | Cl | Pipéridino | 0,19 AcOEt/TEA 92/8 |

(suite)

| | Produit de départ | n' | X' | $NR'_3R'_4$ | Rf s. |
|---|---|---|---|---|---|
| Ex. 28 | Ex. 12 | 4 | Cl | Pipéridino | 0,22 AcOEt/TEA 95/5 |

**EXEMPLE 29 : 4-chloro-11β-[4-[2-(diéthylamino)éthoxy]phényl]-17alpha-méthyl-estra-1,3,5(10)-triène-3,17béta-diol**

a) Préparation du "cerien"

A 2,42 g de $CeCl_3$-$7H_2O$, préalablement séché sous pression réduite à 140°C pendant 2 heures, puis mis sous atmosphère inerte, on ajoute 24 ml de THF anhydre, agite 2 heures la suspension, refroidit à -78°C et ajoute 4,35 ml de $CH_3Li$ dans l'éther et agite 30 minutes à -78°C.

b) Condensation

On ajoute à -78°C, 0,644 g du composé préparé à l'exemple 3 en solution dans 8,5 ml de THF anhydre et maintient 1 heure à cette température. On coule dans 25 ml d'une solution saturée de $NH_4Cl$, extrait, lave, sèche et évapore sous pression réduite jusqu'à obtention de 0,599 g de produit brut que l'on purifie par chromatographie (après avoir rajouté 0,192 g de produit brut obtenu au cours d'un essai annexe mené à l'identique) en éluant avec le mélange $CH_2Cl_2/CH_3OH/NH_4OH$ (92/8/0,2), puis avec le mélange AcOEt/TEA (87/13). On obtient 0,402 g de produit pur attendu.
Rf $CH_2Cl_2$/MeOH/$NH_4OH$ (92/8/0,2) = 0,18
F = 161-163°C.
RMN ($CDCl_3$) :
0,44 (s) : $CH_3$ en 18 ; 1,28 (s) : $CH_3$ en 17 ; 1,03 (t) : $CH_2C\underline{H}_3$ ; 2,61 (q) : $C\underline{H}_2$-$CH_3$ ; 2,81 (t) : $CH_2$-N ; 3,94 (t) : $C\underline{H}_2$-O ; 3,96 : $H_{11}$ ; 6,61-6,80 : $H_1$ et $H_2$ ; 6,61-6,83 : Ph-O.

**EXEMPLE 30 : 4-chloro-17alpha-méthyl-11β-[4-[2-(1-pipéridinyl) êthoxy] phényl]-estra-1,3,5(10)-triène-3,17béta-diol.**

On opère comme à l'exemple 28, mais à partir de 2,79 g du produit obtenu à l'exemple 1.
On obtient 2,12 g de produit pur attendu.
Rf $CH_2Cl_2/CH_3OH/NH_4OH$ (93/7/0,2) = 0,19
F = 163°C.
RMN ($CDCl_3$) :
0,44 (s) : $CH_3$ en 18 ; 1,28 (s) : $CH_3$ en 17 ; 2,48 (t1) 4H : $CH_2N$ cycle ; 2,71 (t) : $CH_2$-N chaîne ; 3,99 (t) : $C\underline{H}_2$-O ; =4,00 masqué : $H_{11}$ ; 6,80 (d) : $H_1$ ; 6,98 (d) : $H_2$ ; 6,60 - 6,88 : Ph-O.
On a également préparé les composés suivants :

| EX | X | n | $R_1$ | Y | $NR_3R_4$ | $R_5$ | $R_6$ | Rf |
|---|---|---|---|---|---|---|---|---|
| 31 | Cl | 2 | H | O | 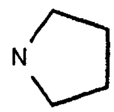 | OH | Me | (a) 90/10/0,4 0,25 |
| 32 | Cl | 2 | H | O | 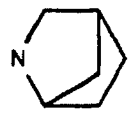 | OH | Me | (a) 90/10/0,9 0,25 |
| 33 | Cl | 2 | H | O | 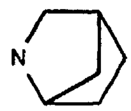 | OH | H | (a) 90/10/1 0,26 |
| 34 | Cl | 2 | H | O | NMeiPr | OH | H | (a) 90/10/0,5 0,22 |
| 35 | Cl | 2 | H | O | 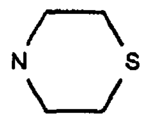 | OH | H | (b) 30/70 0,22 |
| 36 | Cl | 2 | H | O | NEtPr | OH | H | (a) 90/10/0,5 0,27 |
| 37 | Cl | 2 | H | O | NMeEt | OH | H | (b) 30/70 0,22 |
| 38 | Cl | 2 | H | O | 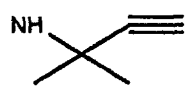 | OH | H | (a) 95/5/0,5 0,28 |
| 39 | Cl | 2 | H | O | 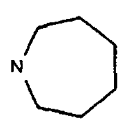 | OH | H | (b) 30/70 0,33 |

| EX | X | n | $R_1$ | Y | $NR_3R_4$ | $R_5$ | $R_6$ | Rf |
|---|---|---|---|---|---|---|---|---|
| 40 | Cl | 2 | H | O | 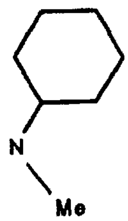 | OH | H | (b) 30/70 0,32 |
| 41 | Cl | 2 | H | O | NEtBu | OH | H | (a) 90/10/0,5 0,37 |
| 42 | Cl | 2 | H | O | 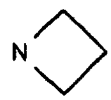 | OH | H | (a) 90/10/0,5 0,20 |
| 43 | Cl | 2 | H | O | 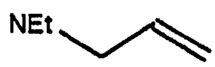 | OH | H | (a) 95/5/0,5 0,25 |
| 44 | Cl | 2 | H | O | 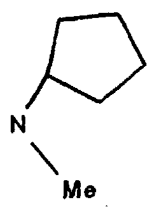 | OH | H | (a) 90/10/0,5 0,30 |
| 45 | Cl | 2 | H | O | $NPr_2$ | OH | H | (a) 95/5/0,5 0,20 |
| 46 | Cl | 2 | H | O | 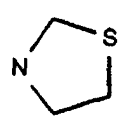 | OH | H | (b) 30/70 0,28 |
| 47 | Cl | 2 | H | O | NEtiPr | OH | H | (a) 90/10/0,5 0,22 |
| 48 | Cl | 2 | H | O | NMePr | OH | H | (a) 90/10/0,5 0,22 |
| 49 | Cl | 2 | H | O | NMeBu | OH | H | (a) 90/10/0,5 0,22 |

| EX | X | n | $R_1$ | Y | $NR_3R_4$ | $R_5$ | $R_6$ | Rf |
|---|---|---|---|---|---|---|---|---|
| 50 | Cl | 2 | H | O | 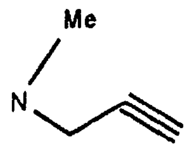 | OH | H | (a) 95/5/0,5<br>0,22 |
| 51 | Cl | 2 | H | O | 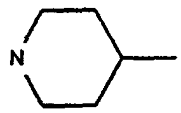 | OH | H | (a) 90/10/0,5<br>0,32 |
| 52 | Cl | 2 | H | S | 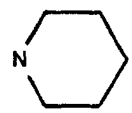 | OH | H | (a) 93/7/0,5<br>0,30 |
| 53 | Cl | 2 | H | SO | 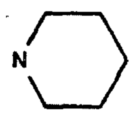 | OH | H | (a) 93/7/0,5<br>0,23 |
| 54 | Cl | 2 | $(CH_2)_3$<br>-COOH | O | $NEt_2$ | OH | H | (a) 80/20/1<br>0,25 |
| 55 | Cl | 2 | H | O | 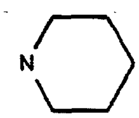 | =N-OH | | (a) 90/10/0,5<br>0,18 |
| 56 | Cl | 2 | H | O | 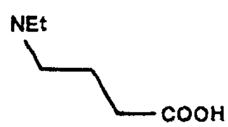 | OH | H | |

a) $CH_2Cl_2/MeOH/NH_4OH$

b) TEA/AcOEt

## Tests pharmacologiques

### Effet sur la prolifération de cellules mammaires

L'activité proliférative des molécules est étudiée comparativement à celle de l'oestradiol sur les cellules mammaires humaines MCF-7 en culture.

Pour mettre en évidence un effet agoniste de l'oestradiol et/ou des molécules testées, le milieu de culture d'entretien des cellules (riche en facteurs de croissance et en stéroïdes) est remplacé par un milieu appauvri, entre autres dépourvu de stéroides (DMEM supplémenté par 5 % de sérum déstéroïdé et sans rouge de phénol). Les cellules subissent ce sevrage deux jours avant le début de l'essai.

Après 7 jours de culture en présence des produits à étudier, la prolifération cellulaire est évaluée par dosage du DNA. Dans chaque essai, l'effet de l'oestradiol à $10^{-10}$M (croissance cellulaire en présence d'oestradiol moins croissance cellulaire en présence du solvant) détermine le 100 % de l'activité agoniste. L'activité des molécules est évaluée en comparaison à ce témoin interne. Les molécules induisant une croissance cellulaire identique à celle observée avec le solvant seul sont classées "inactives", celles induisant une croissance cellulaire inférieure à celle observée avec le solvant sont classées "inhibiteur".

| | ACTIVITE |
|---|---|
| Estradiol | Agoniste |
| Exemple 20 | Mixte * |
| Exemple 22 | Inactif |
| Exemple 23 | Mixte |
| Exemple 1 | Mixte |
| Exemple 30 | Mixte |

* Mixte : légère activité agoniste aux très faibles concentrations et activité inhibitrice aux concentrations plus fortes.

### Etude de l'impact osseux d'un produit chez la rate femelle ovariectomisée à l'âge de 3 mois.

Les composés A, B, C, D, E sont testés afin de déterminer leur effet sur la masse osseuse et sur l'activité de formation et de résorption dans le modèle de la rate ovariectomisée à l'âge de 3 mois. Les animaux sont traités en préventif.

**Animaux :**

| | |
|---|---|
| Espèce | rat |
| Souche | Sprague-Dawley |
| Sexe | femelle |
| Poids | 250 g à 280 g |
| Nbre d'animaux/groupe | 8 |

**Produits :**

1 - Produit à tester : Produits des exemples 20, 22, 23, 30 et 1.

- véhicule(s) : huile de maïs, méthylcellulose 0,5 %
- dose(s) : une dose par produit testé (0,3 mg/kg/j)
- nombre d'administrations : une fois/jour ; 5 jours/semaine pendant 4 semaines
- voie d'administration : voie orale pour les produits
- volumes : 5 ml/kg (p.o.)
- délai entre la dernière injection et le sacrifice : 24 heures

* nombre d'administrations : 20.

2 - Produit de référence : le 17β oestradiol est administré par voie sous cutanée à la dose 0,1 mg/kg/j en solution dans un mélange d'huile de germe de mais-alcool benzylique (99:1, v/v) sous un volume de 0,2 ml/kg.

**Protocole expérimental**

Animaux

L'étude est réalisée chez des rats femelles ovariectomisées à l'âge de 3 mois. Les animaux sont maintenus dans une pièce climatisée (température 20°C ± 2°C) et groupés par 4 dans des boîtes. Les animaux reçoivent, ad libitum, de l'eau déminéralisée et des aliments comprimés (bouchons : AO4CR-10 UAR).

Chirurgie

Des rats femelles âgées de 3 mois pesant environ 250 g sont ovariectomisées sous anesthésie à l'Imalgène 1000, à la dose de 100 mg/kg par voie intrapéritonéale (i.p.) et sous un volume de 1 ml/kg. Ils reçoivent également du Nembutal (3 mg/kg i.p. sous un volume de 0,3 ml/kg).
Après incision latérale, les plans cutanés et musculaires sont sectionnés. L'exérèse de chaque ovaire se fait après ligature de l'oviducte.
Les rats témoins "SHAM" sont anesthésiés dans les mêmes conditions. Après incision des plans cutanés et musculaires, chaque ovaire est exposé puis replacé in situ.

**Traitement**

Les effets des produits sont déterminés en traitement préventif. Ils sont administrés immédiatement après l'ovariectomie. Les animaux répartis en groupes de 8. Groupe 1 : rats témoins "SHAM" recevant le ou les véhicules Groupe 2 : rats témoins "OVX" recevant le ou les véhicules Groupes X : rats "OVX" recevant respectivement les doses définies du ou des produits à tester.

Prélèvements sanguins

Au terme des 4 semaines (durée de l'étude) les animaux sont décapités par guillotine. Les sérums recueillis après centrifugation sont conservés à -20°C.
Un bilan lipidique sera établi à partir des dosages sériques du cholestérol total, des triglycérides et des phospholipides sur une aliquote de sérum de 500 µl. La baisse du taux de cholestérol sérique est exprimée en % par rapport au taux présenté par les animaux ovariectomisés ne recevant que le solvant.

Prélèvements d'organes

Après sacrifice des animaux, les organes suivants sont prélevés :

- tractus génital
  Les utérus sont prélevés. Ces derniers sont pesés. L'augmentation du poids est exprimée, en % du poids de l'utérus des animaux ovariectomisés ne recevant que le solvant.
- au niveau osseux :
  La masse osseuse (BMD ou Bone mineral density = densité minérale osseuse) est mesurée par absorptiométrie biphotonique à rayons X en double énergie (DEXA). Les mesures sont réalisées sur les os excisés et débarrassés de tous les tissus mous. La BMD (Bone mineral density) est mesurée sur l'os entier ainsi que sur la partie métaphysaire au niveau de l'extrémité proximale pour le tibia gauche. Cette zone est définie comme étant la région la plus riche en os trabéculaire ; et par conséquent, est la plus sensible aux variations de volume osseux et de densité minérale osseuse.

Les résultats sont exprimés en % selon la formule :

$$\frac{\text{BMD produit testé - BMD OVX}}{\text{BMD SHAM - BMD OVX}} \times 100$$

| | Dose voie mg/kg | OS TIBIA BMD % | UTERUS Poids % | Cholestérol % |
|---|---|---|---|---|
| OVX | | 0 | | |
| SHAM | | 100 | | |
| Estradiol | 0,1 sc | 105 | 359 | -35 |
| Ex. 20<br>Ex. 20 | 0,3 po<br>1,0 po | 69<br>74 | 60<br>50 | -43<br>-40 |
| Ex. 22 | 0,3 po | 63 | 57 | -52 |
| Ex. 23<br>Ex. 23 | 0,3 po<br>1,0 po | 67<br>71 | 64<br>74 | -53<br>-53 |
| Ex. 1<br>Ex. 1 | 0,3 po<br>1,0 po | 57<br>64 | 55<br>55 | -50<br>-48 |
| Ex. 30<br>Ex. 30 | 0,3 po<br>1,0 po | 52<br>71 | 68<br>55 | -51<br>-48 |

**Revendications**

**1.** Composés de formule générale (I) :

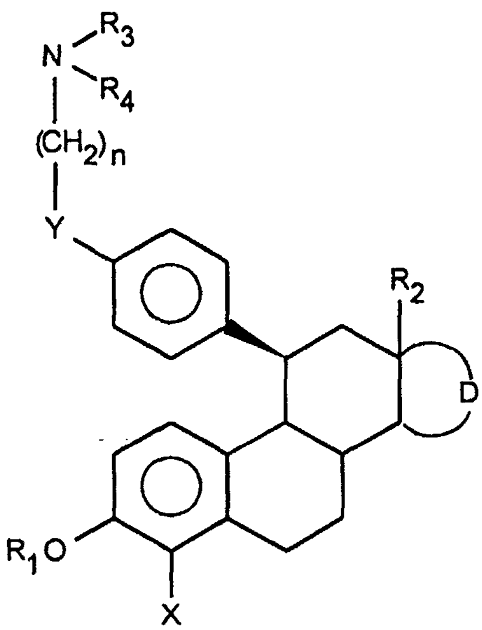

(I)

dans laquelle :

$R_1$ représente un atome d'hydrogène, un radical $(CH_2)_m$-Ar, (CO)-Ar, $(CH_2)_m$-Alk ou (CO)-Alk,
$R_2$ représente un radical dérivé d'un hydrocarbure, linéaire ou ramifié, saturé ou insaturé renfermant de 1 à 6 atomes de carbone
D représente le reste d'un cycle pentagonal ou hexagonal éventuellement substitué et éventuellement porteur d'insaturation,
X représente un atome d'halogène,
Y est choisi parmi O, S, SO, $SO_2$ et NH,
n est un entier variant de 2 à 8,

soit $R_3$ et $R_4$ identiques ou différents représentent un atome d'hydrogène, un groupement $(CH_2)_m$-Ar, $(CH_2)_m$-Het ou $(CH_2)_m$Alk,
soit $R_3$ et $R_4$ forment ensemble avec l'atome d'azote auquel ils sont liés un hétérocycle mono ou polycyclique, saturé ou insaturé, aromatique ou non aromatique, de 3 à 15 chaînons renfermant éventuellement de 1 à 3 hétéroatomes additionnels choisis parmi l'oxygène, le soufre et l'azote, non substitué ou substitué,
Ar représentant un groupement aryle carbocyclique renfermant de 6 à 18 atomes de carbone, Het représentant un radical dérivé d'un hétérocycle aromatique ou non aromatique, saturé ou non saturé, comportant de 1 à 9 atomes de carbone et de 1 à 5 hétéroatomes choisis parmi les atomes d'oxygène, d'azote ou de soufre, Alk représentant un radical dérivé d'un hydrocarbure, non aromatique, linéaire, ramifié ou cyclique, saturé ou insaturé et comportant de 1 à 12 atomes de carbone, les radicaux Ar, Het ou Alk pouvant être substitués ou non substitués, m représente 0, 1, 2 ou 3, ainsi que leurs sels d'addition avec les bases ou les acides.

**2.** Composes de formule générale (I) telle que définie à la revendication 1, dans laquelle D représente le reste d'un cycle pentagonal de formule :

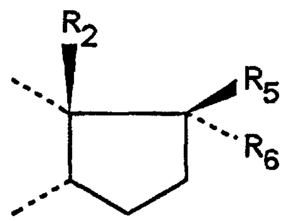

dans laquelle $R_2$ conserve la même signification qu'à la revendication 1,
soit $R_5$ représente un radical OH, O-$(CH_2)_m$-Alk, O-(CO)-Alk, O-$(CH_2)_m$-Ar, O-(CO)-Ar, O-$(CH_2)_m$-Het, O-(CO)-Het et $R_6$ représente un atome d'hydrogène, un radical alkyle, alkényle ou alkynyle renfermant de 1 à 6 atomes de carbone substitué ou non substitué, m, Alk, Ar et Het étant tels que définis à la revendication 1,
soit $R_5$ et $R_6$ forment ensemble avec l'atome de carbone qui les porte l'un des cycles suivants :

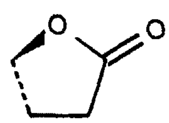
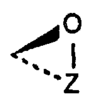
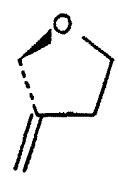

dans lequel Z représente un groupement $-(CH_2)_1-$ ou $-CH=CH-(CH_2)_{1'}$, 1 étant un entier compris entre 1 et 4 et 1' étant un entier égal à 1 ou 2,
soit $R_5$ et $R_6$ forment ensemble un groupement oxo ou =N-OH, ainsi que leurs sels d'addition avec les acides ou les bases.

**3.** Composés de formule générale (I) telle que définie à la revendication 1, répondant à la formule générale (I')

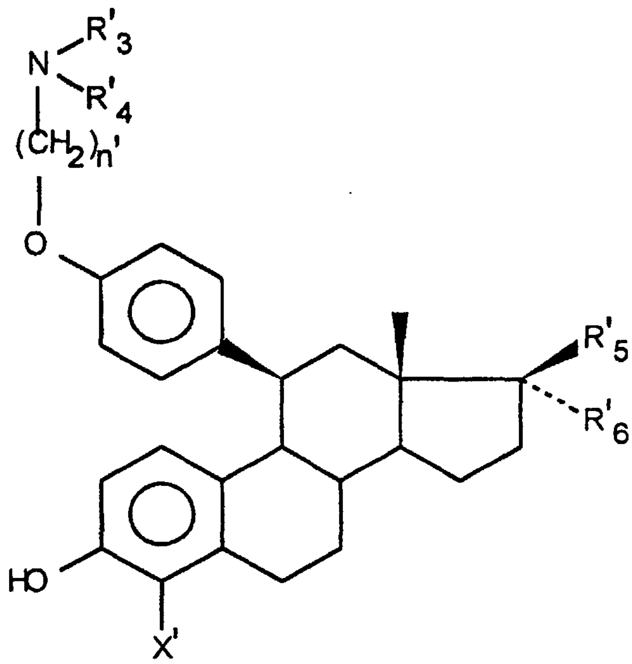

(I')

dans laquelle :

X' représente un atome de chlore ou de brome
n' est compris entre 2 et 5,

soit $R'_3$ et $R'_4$ identiques ou différents représentent un radical alkyle renfermant de 1 à 6 atomes de carbone
soit $R'_3$ et $R'_4$ forment ensemble, avec l'atome d'azote auquel ils sont liés, un reste mono ou polyclique saturé de 3 à 15 chaînons renfermant éventuellement un hétéroatome additionnel choisi parmi l'oxygène, le soufre et l'azote, $R'_5$ et $R'_6$ ont la même signification que $R_5$ et $R_6$, selon la revendication 2 ainsi que leurs sels d'addition avec les acides et les bases.

4. Composés de formule générale (I') telle que définie à la revendication 3, dans laquelle :
soit $R'_5$ représente un radical OH et $R'_6$ représente un atome d'hydrogène, un radical alkyle, alkényle ou alkynyle renfermant de 1 à 6 atomes de carbone, substitué ou non substitué,
soit $R'_5$ et $R'_6$ forment ensemble avec l'atome de carbone qui les porte l'un des cycles suivants :

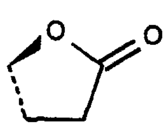

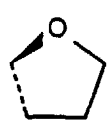

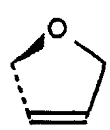

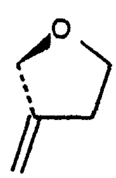

soit $R'_5$ et $R'_6$ forment ensemble un groupement oxo,
ainsi que leurs sels d'addition avec les acides ou les bases.

5. Composés de formule générale (I') telle que définie à la revendication 3 ou 4, dans laquelle :

X' représente un atome de chlore,
n' est égal à 2,

soit $R'_3$ et $R'_4$ identiques ou différents représentent un radical alkyle renfermant de 1 à 6 atomes de carbone,
soit $R'_3$ et $R'_4$ forment ensemble avec l'atome d'azote les hétérocycles saturés suivants :

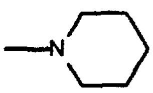 , 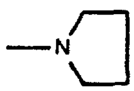 , 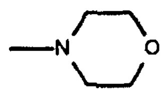 , 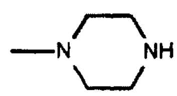 et 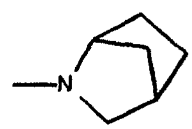

et soit $R'_5$ représente un radical OH et $R'_6$ représente un atome d'hydrogène, un radical alkyle, aikényle ou alkynyle renfermant de 1 à 6 atomes de carbone, substitué ou non substitué,
soit $R'_5$ et $R'_6$ forment ensemble avec l'atome de carbone qui les porte l'un des cycles suivants :

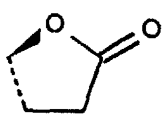 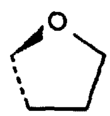 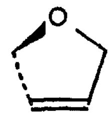 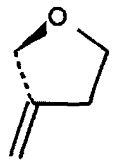

soit $R'_5$ et $R'_6$ forment ensemble un groupement oxo,
ainsi que leurs sels d'addition avec les acides ou les bases.

**6.** Composés de formule (I) ou (I') telle que définie à l'une quelconque des revendications 1 à 5, dont les noms suivent :

- 4-chloro-3-hydroxy-11β-[4-[2-(diéthylamino)éthoxy]phényl]-estra-1,3,5(10)-trièn-17-one,
- 4-chloro-3-hydroxy-11β-[4-[2-(diméthylamino)éthoxy]phényl]-estra-1,3,5(10)-trièn-17-one,
- 4-chloro-3-hydroxy-11β-[4-[2-(1-pipéridinyl)éthoxy]phényl]-estra-1,3,5(10)-trièn-17-one,
- 4-chloro-3-hydroxy-11β-[4-[2-(1-pyrrolidinyl)éthoxy] phényl]-estra-1,3,5(10)-trièn-17-one,
- 4-bromo-3-hydroxy-11β-[4-[2-(1-pipéridinyl)éthoxy)phényl]-estra-1,3,5(10)-trièn-17-one,
- 4-chloro-11β-[4-[2-(diméthylamino)éthoxy]phényl]-estra-1,3,5(10)-triène-3,17béta-diol,
- 4-chloro-11β-[4-[2-(diéthylamino)éthoxy]phényl]-estra-1,3,5(10)-triène-3,17béta-diol,
- 4-chloro-11β-[4-[2-(1-pipéridinyl)éthoxy]phényl]-estra-1,3,5(10)-triène-3,17béta-diol,
- 4-bromo-11β-[4-[2-(1-pipéridinyl)éthoxy]phényl]-estra-1,3,5(10)-triène-3,17béta-diol,
- 4-chloro-11β-[4-[2-(1-pyrrolidinyl)éthoxy)phényl]-estra-1,3,5(10)-triène-3,17béta-diol,
- 4-chloro-11β-[4-[2-(diéthylamino)éthoxy]phényl]-19-nor-17alpha-pregna-1,3,5(10)-trièn-20-yne-3,17béta-diol,
- 4-chloro-11β-[4-[3-(1-pipéridinyl)propoxy]phényl]-estra-1,3,5(10)-triène-3,17béta-diol,
- 4-chloro-11β-[4-[4-(1-pipéridinyl)butoxy]phenyl]-estra-1,3,5(10)-triène-3,17béta-diol,
- 4-chloro-11β-[4-[5-(1-pipéridinyl)pentyloxy]phényl]-estra-1,3,5(10)-triène-3,17béta-diol,
- gamma lactone de l'acide (17alpha)-4-chloro-3,17béta-dihydroxy-11β-[4-[2-(diéthylamino)éthoxy]phényl]-19-nor-pregna-1,3,5(10)-triène-21-carboxylique,
- gamma lactone de l'acide (17alpha)-4-chloro-3,17béta-dihydroxy-11β-[4-[2-(1-pyrrolidinyl)éthoxy]phényl]-19-nor-pregna-1,3,5(10)-triène-21-carboxylique,
- gamma lactone de l'acide (17alpha)-4-chloro-3,17béta-dihydroxy-11β-[4-[2-(1-pipéridinyl)éthoxy]phényl]-19-nor-pregna-1,3,5(10)triène-21-carboxylique,
- gamma lactone de l'acide (17alpha)-4-chloro-3,17béta-dihydroxy-11β-[4-[3-(1-pipéridinyl)propoxy]phényl]-19-nor-pregna-1,3,5(10)-triène-21-carboxylique,
- gamma lactone de l'acide (17alpha)-4-chloro-3,17béta-dihydroxy-11β-[4-[4-(1-pipéridinyl)butoxy]phényl]-19-nor-pregna-1,3,5(10)-triène-21-carboxylique,
- gamma lactone de l'acide (17alpha)-4-chloro-3,17béta-dihydroxy-11β-[4-[5-(1-pipéridinyl)pentyloxy]phényl]-19-nor-pregna-1,3,5(10)-triène-21-carboxylique,
- (17béta)-4-chloro-11β-[4-[2-(diéthylamino)éthoxy]phényl]-spiro[estra-1,3,5(10)-triène-17,2'(5'H)furan]-3-ol,
- (17béta)-4-chloro-11β-[4-[2-(1-pipéridinyl)éthoxy]phényl]-spiro[estra-1,3,5(10)-triène-17,2'(5'H)furan]-3-ol,
- (17béta)-4-chloro-11β-[4-[2-(1-pyrrolidinyl)éthoxy]phényl]-spiro[estra-1,3,5(10)-triène-17,2'(5'H)furan]-3-ol,
- (17béta)-4-chloro-4',5'-dihydro-11β-[4-[2-(1-pipéridinyl) éthoxy]phényl]-spiro[estra-1,3,5(10)-triène-17,2'(3'H)furan]-3-ol,
- (17béta)-4-chloro-11β-[4-[3-(1-pipéridinyl)propoxy]phényl]-spiro[estra-1,3,5(10)-triène-17,2'(5'H)furan]-3-ol,
- (17béta)-4-chloro-4',5'-dihydro-11β-[4-[3-(1-pipéridinyl) propoxy]phényl]-spiro[estra-1,3,5(10)-triène-17,2'

(3'H) furan]-3-ol,

- (17béta)-4-chloro-11β-[4-[4-(1-pipéridinyl)butoxy]phényl]-spiro[estra-1,3,5(10)-triène-17,2'(5'H)furan]-3-ol,
- (17béta)-4-chloro-4',5'-dihydro-11β-[4-[4-(1-pipéridinyl)-butoxy]phényl]-spiro[estra-1,3,5(10)-triène-17,2'(3'H)furan]-3-ol,
- 4-chloro-11β-[4-[2-(diéthylamino)éthoxy]phényl]-17alpha-méthyl-estra-1,3,5(10)-triène-3,17béta-diol
- 4-chloro-17alpha-méthyl-11β-[4-[2-(1-pipéridinyl) éthoxy] phényl]-estra-1,3,5(10)-triène-3,17béta-diol,
- 4-chloro-17alpha-méthyl-11β-[4-[2-(1-pyrrolidinyl)éthoxy] phényl]-estra-1,3,5(10)-triène-3,17béta-diol,
- 11β-[4-[2-[2-aza-bicyclo(2.2.1)hept-2-yl]éthoxy] phényl]-4-chloro-17alpha-méthyl-estra-1,3,5(10)-triène-3,17béta-diol,
- 11β-[4-[2-[2-aza-bicyclo(2.2.1)hept-2-yl]éthoxy] phényl]-4-chloro-estra-1,3,5(10)-triène-3,17béta-diol,
- 4-chloro-11β-[4-[2-[méthyl(1-méthyléthyl)amino]éthoxy] phényl]-estra-1,3,5(10)-triène-3,17béta-diol,
- 4-chloro-11β-[4-[2-(tétrahydro-(1H)-1,4-thiazin-4-yl) éthoxy]phényl]-estra-1,3,5(10)-triène-3,17béta-diol,
- 4-chloro-11β-[4-[2-[éthyl(propyl)amino]éthoxy]phényl]-estra-1,3,5(10)-triène-3,17béta-diol,
- 4-chloro-11β-[4-[2-[éthyl(méthyl)amino]éthoxy]phényl]-estra-1,3,5(10)-triène-3,17béta-diol,
- 4-chloro-11β-[4-[2-[(1,1-diméthyl-2-propynyl)amino]éthoxy] phényl]-estra-1,3,5(10)-triène-3,17béta-diol,
- 4-chloro-11β-[4-[2-(hexahydro-1H-azépin-1-yl)éthoxy] phényl]-estra-1,3,5(10)-triène-3,17béta-diol,
- 4-chloro-11β-[4-[2-[cyclohexyl(méthyl)amino]éthoxy]phényl]-estra-1,3,5(10)-triène-3,17béta-diol,
- 4-chloro-11β-[4-[2-[butyl(éthyl)amino]éthoxy]phényl]-estra-1,3,5(10)-triène-3,17béta-diol,
- 4-chloro-11β-[4-[2-(1-azétidinyl)éthoxy]phényl]-estra-1,3,5(10)-triène-3,17béta-diol,
- 4-chloro-11β-[4-[2-[éthyl(2-propényl)amino]éthoxy]phényl]-estra-1,3,5(10)-triène-3,17béta-diol,
- 4-chloro-11β-[4-[2-[cyclopentyl(méthyl)amino]éthoxy] phényl]-estra-1,3,5(10)-triène-3,17béta-diol,
- 4-chloro-11β-[4-[2-(dipropylamino)éthoxy]phényl]-estra-1,3,5(10)-triène-3,17béta-diol,
- 4-chloro-11β-[4-[2-(3-thiazolidinyl)éthoxy]phényl]-estra-1,3,5(10)-triène-3,17béta-diol,
- 4-chloro-11β-[4-[2-[éthyl(1-méthyléthyl)amino]éthoxy] phényl]-estra-1,3,5(10)-triène-3,17béta-diol,
- 4-chloro-11β-[4-[2-[méthyl(propyl)amino]éthoxy]phényl]-estra-1,3,5(10)-triène-3,17béta-diol,
- 4-chloro-11β-[4-[2-[butyl(méthyl)amino]éthoxy]phényl]-estra-1,3,5(10)-triène-3,17béta-diol,
- 4-chloro-11β-[4-[2-[méthyl(2-propynyl)amino]éthoxy]phényl]-estra-1,3,5(10)-triène-3,17béta-diol,
- 4-chloro-11β-[4-[2-(4-méthyl-1-pipéridinyl)éthoxy]phényl]-estra-1,3,5(10)-triène-3,17béta-diol,
- 4-chloro-11β-[4-[2-(1-pipéridinyl)éthylthio]phényl]-estra-1,3,5(10)-triène-3,17béta-diol,
- 4-chloro-11β-[4-[2-(1-pipéridinyl)éthylsulfinyl]phényl]-estra-1,3,5(10)-triène-3,17béta-diol,
- acide 4-[4-chloro-11béta-[4-[2-(diéthylamino)éthoxy]phényl] 17béta-hydroxy-estra-1,3,5(10)-trièn-3-yl-oxy]-butanoïque,
- oxime de 4-chloro-3-hydroxy-llbéta-[4-[2-(1-pipéridinyl) éthoxy]phényl]-estra-1,3,5(10)-trièn-17-one,
- acide 4-[[2-[4-(4-chloro-3,17béta-dihydroxy-estra-1,3,5(10)-trièn-11béta-yl)phénoxy]éthyl]éthylamino]-butanoïque.

7. Composé de formule (I) ou (I') telle que définie à l'une quelconque des revendications 1 à 5, dont le nom suit :

- 4-chloro-11β-[4-[2-(diéthylamino)éthoxy]phényl]-estra-1,3,5(10)-triène-3,17béta-diol, ainsi que ses sels d'addition avec les acides.

8. Procédé de préparation des composés de formule générale (I) telle que définie à la revendication 1 ou 2, **caractérisé en ce que** l'on soumet un composé de formule générale (II) :

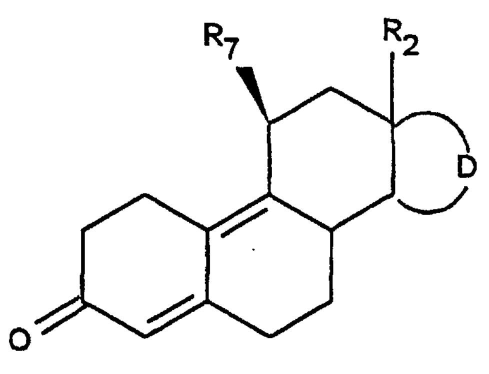

(II)

dans laquelle D et $R_2$ sont tels que définis à la revendication 1, $R_7$ représente un des groupements suivants :

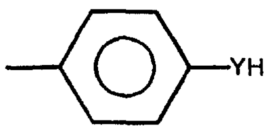

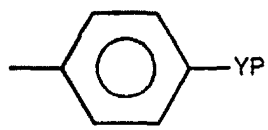

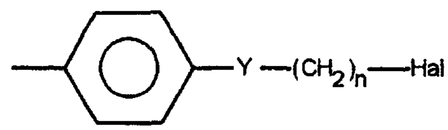

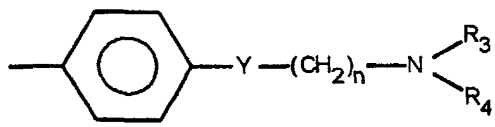

dans lesquels n, Y, $R_3$ et $R_4$ sont tels que définis à la revendication 1, P est un groupement protecteur, Hal représente un halogène,
à l'action d'un réactif d'halogénation afin d'obtenir le composé de formule (III) :

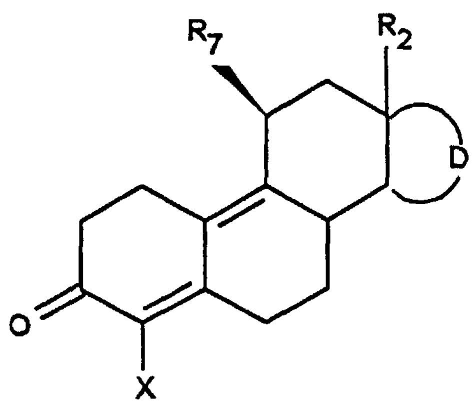

(III)

que l'on soumet à l'action d'un réactif d'aromatisation du cycle A, puis à l'action d'une base afin d'obtenir le composé de formule (IV) correspondant à certains composés de formule générale (I) :

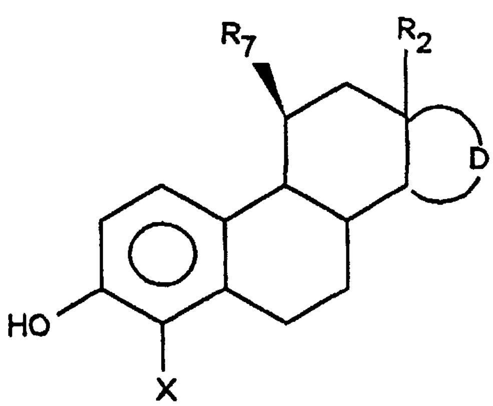

(IV)

composés de formule (II), (III) ou (IV)
que l'on soumet, si désiré et si nécessaire, dans un ordre approprié, à l'une ou plusieurs des réactions suivantes :

- protection des composés dans lesquels $R_7$ est un groupement -Ph-YH,
- déprotection des composés dans lesquels $R_7$ est un groupement Ph-YP,
- action d'un composé de formule $Hal_1-(CH_2)_n-Hal_2$ sur les composés dans lesquels $R_7$ est un groupement -Ph-YH, $Hal_1$ ou $Hal_2$ identiques ou différents représentant un halogène afin d'obtenir des composés dans lesquels $R_7$ est un groupement $-Ph-Y-(CH_2)_n-Hal_2$,
- action d'un composé de formule $R_3-NH-R_4$ sur les composés dans lesquels $R_7$ est un groupement $Ph-Y-(CH_2)_n-Hal_2$, afin d'obtenir des composés dans lesquels $R_7$ est un groupement $Ph-Y-(CH_2)_n-NR_3R_4$,
- action d'un sel d'halogénure ($M-Hal_3$) sur les composés dans lesquels $R_7$ est un groupement $Ph-Y-(CH_2)_n-Hal_2$ afin d'obtenir des composés dans lesquels $R_7$ est un groupement $-Ph-Y-(CH_2)_n-Hal3$,
- protection du groupement OH en position 3 ou 17,
- déprotection du groupement OH en position 3 ou 17,
- alkylation du groupement OH en position 3 ou 17,
- acylation du groupement OH en position 3 ou 17,
- action d'un agent de réduction lorsque D représente le reste d'un cycle pentagonal tel que défini à la revendication 2 et $R_5$ et $R_6$ forment ensemble un groupement oxo,
- action d'un organométallique sur les composés de formule (IV) avec D représentant le reste d'un cycle pentagonal tel que défini à la revendication 2 et $R_5$ et $R_6$ forment ensemble un groupement oxo,
- action d'un agent de lactonisation sur les composés de formule (IV) avec D représentant le reste d'un cycle pentagonal tel que défini à la revendication 2 et $R_5$ et $R_6$ formant ensemble un groupement oxo,
- action d'un agent de réduction de la double liaison, lorsque D représente le reste d'un cycle pentagonal tel que défini à la revendication 2 et $R_5$ et $R_6$ forment ensemble avec le carbone qui les porte, un groupement $O-(CH_2)_n'-CH=CH-$,
- action d'un agent de réduction de la double liaison, lorsque D représente le reste d'un cycle pentagonal tel que défini à la revendication 2, et $R_6$ est un radical alkényle ou alkynyle renfermant de 2 à 6 atomes de carbone,
- halogénation en position 4, puis aromatisation du cycle A, du composé de formule générale (II),
- aromatisation du cycle A du composé de formule (III),
- salification.

**9.** Procédé de préparation selon la revendication 8, des composés de formule générale (I') telle que définie à la revendication 3, **caractérisé en ce qu'**on soumet un composé de formule générale (II') :

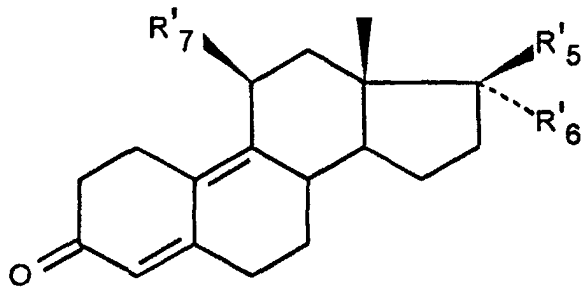

(II')

dans laquelle $R'_5$ et $R'_6$ sont tels que définis à la revendication 3, $R'_7$ représente :

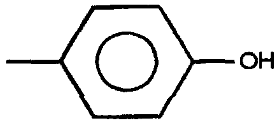

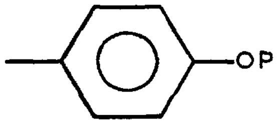

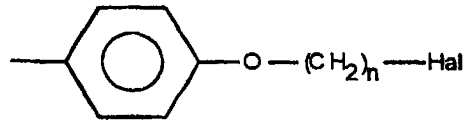

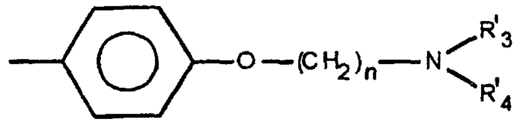

à l'action d'un réactif d'halogénation afin d'obtenir le composé de formule (III') :

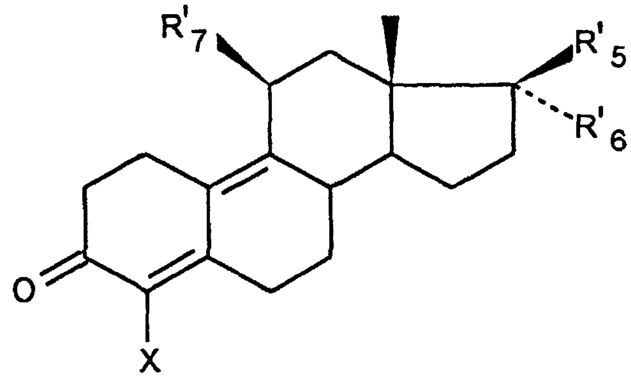

(III')

que l'on soumet à l'action d'un réactif d'aromatisation du cycle A, puis à l'action d'une base afin d'obtenir le composé de formule (IV') correspondant à certains composés de formule générale (I') :

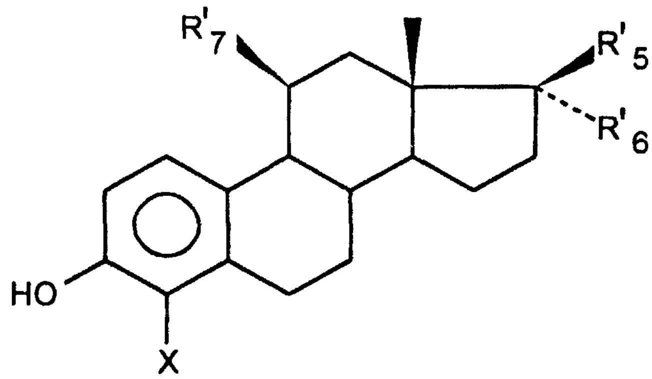

(IV')

composés de formule (II'), (III') ou (IV'), que l'on soumet, si désiré et si nécessaire, dans un ordre approprié, à l'une ou plusieurs des réactions suivantes :

- protection des composés dans lesquels $R'_7$ est un groupement -Ph-OH,
- déprotection des composés dans lesquels $R'_7$ est un groupement Ph-OP,
- action d'un composé de formule $Hal_1$-$(CH_2)_n$-$Hal_2$ sur les composés dans lesquels $R'_7$ est un groupement -Ph-OH, $Hal_1$ ou $Hal_2$ identiques ou différents représentant un halogène afin d'obtenir des composés dans lesquels $R_7$ est un groupement -Ph-O-$(CH_2)_n$-$Hal_2$,
- action d'un composé de formule $R'_3$-NH-$R'_4$ sur les composés dans lesquels $R'_7$ est un groupement Ph-O-$(CH_2)_n$-$Hal_2$, afin d'obtenir des composés dans lesquels $R'_7$ est un groupement Ph-O-$(CH_2)_n$-$NR'_3R'_4$,
- action d'un sel d'halogénure (M-$Hal_3$) sur les composés dans lesquels $R'_7$ est un groupement Ph-O-$(CH_2)_n$-$Hal_2$ afin d'obtenir des composés dans lesquels $R_7$ est un groupement -Ph-O-$(CH_2)_n$-$Hal_3$,
- protection du groupement OH en position 3 ou 17,
- déprotection du groupement OH en position 3 ou 17,
- alkylation du groupement OH en position 17,
- acylation du groupement OH en position 17,
- action d'un agent de réduction lorsque $R'_5$ et $R'_6$ forment ensemble un groupement oxo,
- action d'un organométallique sur les composés de formule (IV') avec $R'_5$ et $R'_6$ formant ensemble un groupement oxo,
- action d'un agent de lactonisation sur les composés de formule (IV') avec $R'_5$ et $R'_6$ formant $R'_5$ et $R'_6$ forment ensemble un groupement oxo,
- action d'un agent de réduction de la double liaison, lorsque $R'_5$ et $R'_6$ forment ensemble avec le carbone qui les porte, un groupement O-$(CH_2)_n$'-CH=CH-,
- action d'un agent de réduction de la double liaison, lorsque $R'_6$ est un radical alkényle ou alkynyle renfermant

de 2 à 6 atomes de carbone,

- halogénation en position 4 puis aromatisation du cycle A du composé de formule (II'),
- aromatisation du composé de formule (III'),
- salification.

**10.** A titre de médicaments les composés de formule (I) telle que définie à la revendication 1, ainsi que leurs sels d'addition avec les acides pharmaceutiquement acceptables.

**11.** A titre de médicaments les composés de formule (I) ou (I') telle que définie à l'une quelconque des revendications 2 à 5, ainsi que leurs sels d'addition avec les acides pharmaceutiquement acceptables.

**12.** A titre de médicaments les composés tels que définis à la revendication 6 ou 7, ainsi que leurs sels d'addition avec les acides pharmaceutiquement acceptables.

**13.** Compositions pharmaceutiques renfermant un ou plusieurs des médicaments tels que définis à l'une quelconque des revendications 10, 11 ou 12.

**14.** A titre de produits intermédiaires nouveaux, les composés de formule générale (III), (III'), (IV) ou (IV') telle que définie à la revendication 8 ou 9.

**15.** Médicament tel que défini aux revendications 10 à 12 pour la prévention ou le traitement de l'ostéoporose.

**16.** Médicament tel que défini aux revendications 10 à 12 pour la protection cardiovasculaire.

## Patentansprüche

**1.** Verbindungen der allgemeinen Formel (I):

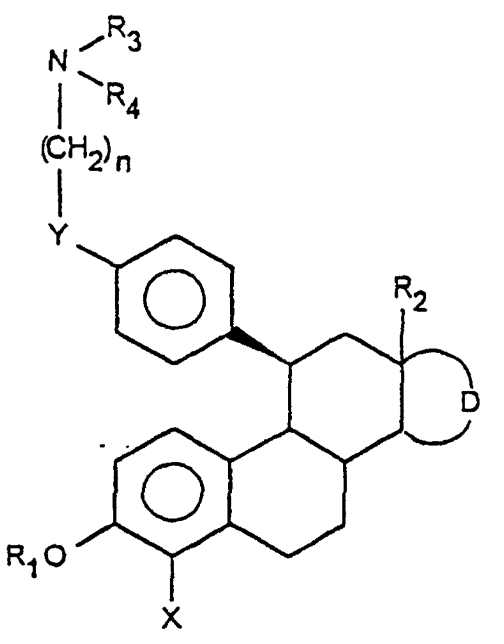

(I)

worin:

$R_1$ ein Wasserstoffatom, einen Rest $(CH_2)_m$-Ar, (CO)-Ar, $(CH_2)_m$-Alk oder (CO)-Alk darstellt,
$R_2$ einen Rest darstellt, der abgeleitet ist von einem linearen oder verzweigten, gesättigten oder ungesättigten Kohlenwasserstoff mit 1 bis 6 Kohlenstoffatomen,
D den Rest eines Fünf- oder Sechsrings darstellt, der gegebenenfalls substituiert ist und gegebenenfalls eine ungesättigte Bindung trägt,

X ein Halogenatom darstellt,
Y ausgewählt ist unter O, S, SO, $SO_2$ und NH,
n eine ganze Zahl von 2 bis 8 ist,

entweder $R_3$ und $R_4$, die gleich oder verschieden sind, ein Wasserstoffatom, eine Gruppe $(CH_2)_m$-Ar, $(CH_2)_m$-Het oder $(CH_2)_m$Alk darstellen,
oder $R_3$ und $R_4$ zusammen mit dem Stickstoffatom, an das sie gebunden sind, einen mono- oder polycyclischen Heterocyclus bilden, der gesättigt oder ungesättigt ist, aromatisch oder nicht aromatisch ist, mit 3 bis 15 Ringgliedern, der gegebenenfalls 1 bis 3 zusätzliche Heteroatome enthält, die ausgewählt sind unter Sauerstoff, Schwefel und Stickstoff, der nicht substituiert oder substituiert ist,
wobei Ar eine carbocyclische Arylgruppe mit 6 bis 18 Kohlenstoffatomen darstellt, Het einen Rest darstellt, der abgeleitet ist von einem aromatischen oder nicht aromatischen, gesättigten oder ungesättigten Heterocyclus mit 1 bis 9 Kohlenstoffatomen und 1 bis 5 Heteroatomen, die ausgewählt sind unter den Sauerstoff-, Stickstoff- oder Schwefelatomen, Alk einen Rest darstellt, der abgeleitet ist von einem Kohlenwasserstoff, der nicht aromatisch, linear, verzweigt oder cyclisch, gesättigt oder ungesättigt ist und 1 bis 12 Kohlenstoffatome umfasst, wobei die Reste Ar, Het oder Alk substituiert oder nicht substituiert sein können, m 0, 1, 2 oder 3 darstellt, sowie ihre Additionssalze mit den Basen oder den Säuren.

**2.** Verbindungen der allgemeinen Formel (I), wie in Anspruch 1 definiert, worin D darstellt den Rest eines Fünfrings der Formel:

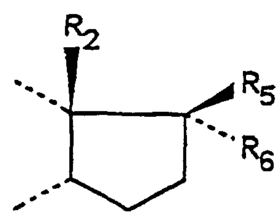

worin $R_2$ die gleiche Bedeutung wie in Anspruch 1 behält,
entweder $R_5$ darstellt einen Rest OH, O-$(CH_2)_m$-Alk, O-(CO)-Alk, O-$(CH_2)_m$-Ar, O-(CO)-Ar, O-$(CH_2)_m$-Het, O-(CO)-Het und $R_6$ darstellt ein Wasserstoffatom, einen Alkyl-, Alkenyl- oder Alkinylrest mit 1 bis 6 Kohlenstoffatomen, der substituiert oder nicht substituiert ist, wobei m, Alk, Ar und Het so wie in Anspruch 1 definiert sind,
oder $R_5$ und $R_6$ zusammen mit dem Kohlenstoffatom, das sie trägt, einen der folgenden Ringe bilden:

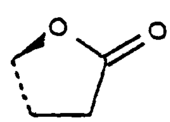 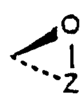 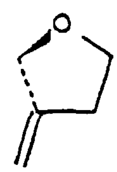

worin Z eine Gruppe $-(CH_2)_l-$ oder $-CH=CH-(CH_2)_{l'}$ darstellt, wobei l eine ganze Zahl zwischen 1 und 4 ist und l' eine ganze Zahl gleich 1 oder 2 ist,
oder $R_5$ und $R_6$ zusammen eine Oxogruppe oder =N-OH-Gruppe bilden, sowie ihre Additionssalze mit den Säuren oder den Basen.

**3.** Verbindungen der allgemeinen Formel (I), wie in Anspruch 1 definiert, die der allgemeinen Formel (I') entsprechen:

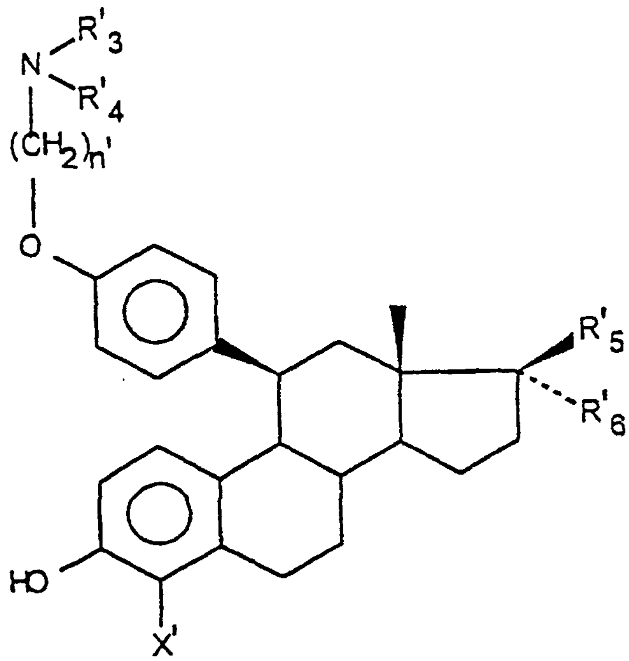

(I')

worin:

X' ein Chlor- oder Bromatom darstellt,
n' zwischen 2 und 5 liegt,

entweder $R'_3$ und $R'_4$, die gleich oder verschieden sind, einen Alkylrest mit 1 bis 6 Kohlenstoffatomen darstellen, oder $R'_3$ und $R'_4$ zusammen mit dem Stickstoffatom, an das sie gebunden sind, einen mono- oder polycyclischen gesättigten Rest mit 3 bis 15 Ringgliedern bilden, der gegebenenfalls ein zusätzliches Heteroatom enthält, das ausgewählt ist unter Sauerstoff, Schwefel und Stickstoff,
$R'_5$ und $R'_6$ die gleiche Bedeutung wie $R_5$ und $R_6$ gemäß Anspruch 2 haben, sowie ihre Additionssalze mit den Säuren und den Basen.

**4.** Verbindungen der allgemeinen Formel (I'), wie in Anspruch 3 definiert, worin:
entweder $R'_5$ einen OH-Rest darstellt und $R'_6$ ein Wasserstoffatom, einen Alkyl-, Alkenyl- oder Alkinylrest mit 1 bis 6 Kohlenstoffatomen, der substituiert oder nicht substituiert ist, darstellt,
oder $R'_5$ und $R'_6$ zusammen mit dem Kohlenstoffatom, das sie trägt, einen der folgenden Ringe bilden:

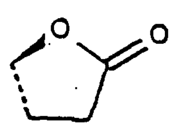 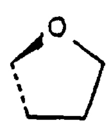 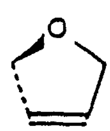 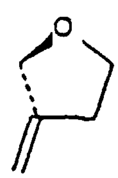

oder $R'_5$ und $R'_6$ zusammen eine Oxogruppe bilden,
sowie ihre Additionssalze mit den Säuren oder den Basen.

**5.** Verbindungen der allgemeinen Formel (I'), wie in Anspruch 3 oder 4 definiert, worin:

X' ein Chloratom darstellt,
n' gleich 2 ist,

entweder $R'_3$ und $R'_4$, die gleich oder verschieden sind, einen Alkylrest mit 1 bis 6 Kohlenstoffatomen darstellen, oder $R'_3$ und $R'_4$ zusammen mit dem Stickstoffatom die folgenden gesättigten Heterocyclen bilden:

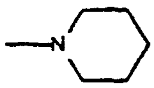 , 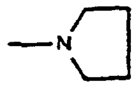 , 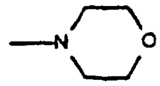 , 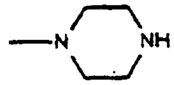 und 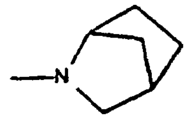

und entweder R'$_5$ einen OH-Rest darstellt und R'$_6$ ein Wasserstoffatom, einen Alkyl-, Alkenyl- oder Alkinylrest mit 1 bis 6 Kohlenstoffatomen, der substituiert oder nicht substituiert ist, darstellt,
oder R'$_5$ und R'$_6$ zusammen mit dem Kohlenstoffatom, das sie trägt, einen der folgenden Ringe bilden:

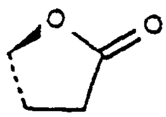 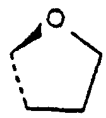 . 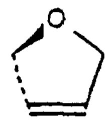 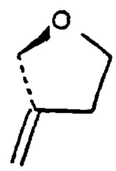

oder R'$_5$ und R'$_6$ zusammen eine Oxogruppe bilden,
sowie ihre Additionssalze mit den Säuren oder den Basen.

**6.** Verbindungen der Formel (I) oder (I'), wie in einem der Ansprüche 1 bis 5 definiert, deren Namen folgen:

- 4-Chlor-3-hydroxy-11β-[4-[2-(diethylamino)ethoxy]phenyl]-östra-1,3,5(10)-trien-17-on,
- 4-Chlor-3-hydroxy-11β-[4-[2-(dimethylamino)ethoxy]phenyl]-östra-1,3,5(10)-trien-17-on,
- 4-Chlor-3-hydroxy-11β-[4-[2-(1-piperidinyl)ethoxy]phenyl]-östra-1,3,5(10)-trien-17-on,
- 4-Chlor-3-hydroxy-11β-[4-[2-(1-pyrrolidinyl)ethoxy]phenyl]-östra-1,3,5(10)-trien-17-on,
- 4-Brom-3-hydroxy-11β-[4-[2-(1-piperidinyl)ethoxy]phenyl]-östra-1,3,5(10)-trien-17-on,
- 4-Chlor-11β-[4-[2-(dimethylamino)ethoxy]phenyl]-östra-1,3,5(10)-trien-3,17betadiol,
- 4-Chlor-11β-[4-[2-(diethylamino)ethoxy]phenyl]-östra-1,3,5(10)-trien-3,17beta-diol,
- 4-Chlor-11β-[4-[2-(1-piperidinyl)ethoxy]phenyl]-östra-1,3,5(10)-trien-3,17beta-diol,
- 4-Brom-11β-[4-[2-(1-piperidinyl)ethoxy]phenyl]-östra-1,3,5(10)-trien-3,17beta-diol,
- 4-Chlor-11β-[4-[2-(1 -pyrrolidinyl)ethoxy]phenyl]-östra-1,3,5(10)-trien-3,17beta-diol,
- 4-Chlor-11β-[4-[2-(diethylamino)ethoxy]phenyl]-19-nor-17alpha-pregna-1,3,5(10)-trien-20-in-3,17beta-diol,
- 4-Chlor-11β-[4-[3-(1-piperidinyl)propoxy]phenyl]-östra-1,3,5(10)-trien-3,17betadiol,
- 4-Chlor-11β-[4-[4-(1-piperidinyl)butoxy]phenyl]-östra-1,3,5(10)-trien-3,17beta-diol,
- 4-Chlor-11β-[4-[5-(1-piperidinyl)pentyloxy]phenyl]-östra-1,3,5(10)-trien-3,17betadiol,
- gamma-Lacton der (17alpha)-4-Chlor-3,17beta-dihydroxy-11β-[4-[2-(diethylamino)ethoxy]phenyl]-19-nor-pregna-1,3,5(10)-trien-21-carbonsäure,
- gamma-Lacton der (17alpha)-4-Chlor-3,17beta-dihydroxy-11β-[4-[2-(1-pyrrolidinyl)ethoxy]phenyl]-19-nor-pregna-1,3,5(10)-trien-21-carbonsäure,
- gamma-Lacton der (17alpha)-4-Chlor-3,17beta-dihydroxy-11β-[4-[2-(1-piperidinyl)ethoxy]phenyl]-19-nor-pregna-1,3,5(10)-trien-21-carbonsäure,
- gamma-Lacton der (17alpha)-4-Chlor-3,17beta-dihydroxy-11β-[4-[3-(1-piperidinyl)propoxy]phenyl]-19-nor-pregna-1,3,5(10)-trien-21-carbonsäure,
- gamma-Lacton der (17alpha)-4-Chlor-3,17beta-dihydroxy-11β-[4-[4-(1-piperidinyl)butoxy]phenyl]-19-nor-pregna-1,3,5(10)-trien-21-carbonsäure,
- gamma-Lacton der (17alpha)-4-Chlor-3,17beta-dihydroxy-11β-[4-[5-(1-piperidinyl)pentyloxy]phenyl]-19-nor-pregna-1,3,5(10)-trien-21-carbonsäure,
- (17beta)-4-Chlor-11β-[4-[2-(diethylamino)ethoxy]phenyl]-spiro[östra-1,3,5(10)-trien-17,2'(5'H)furan]-3-ol,
- (17beta)-4-Chlor-11β-[4-[2-(1-piperidinyl)ethoxy]phenyl]-spiro[östra-1,3,5(10)-trien-17,2'(5'H)furan]-3-ol,
- (17beta)-4-Chlor-11β-[4-[2-(1 -pyrrolidinyl)ethoxy]phenyl]-spiro[östra-1,3,5(10)-trien-17,2'(5'H)furan]-3-ol,
- (17beta)-4-Chlor-4',5'-dihydro-11β-[4-[2-(1-piperidinyl)ethoxy]phenyl]-spiro[östra-1,3,5(10)-trien-17,2'(3'H)furan]-3-ol,
- (17beta)-4-Chlor-11β-[4-[3-(1-piperidinyl)propoxy]phenyl]-spiro[östra-1,3,5(10)-trien-17,2'(5'H)furan]-3-ol,
- (17beta)-4-Chlor-4',5'-dihydro-11β-[4-[3-(1-piperidinyl)propoxy]phenyl]-spiro[östra-1,3,5(10)-trien-17,2'(3'H)furan]-3-ol,
- (17beta)-4-Chlor-11β-[4-[4-(1-piperidinyl)butoxy]phenyl]-spiro[östra-1,3,5(10)-trien-17,2'(5'H)furan]-3-ol,
- (17beta)-4-Chlor-4',5'-dihydro-11β-[4-[4-(1-piperidinyl)butoxy]phenyl]-spiro[östra-1,3,5(10)-trien-17,2'(3'H)furan]-3-ol,

- 4-Chlor-11β-[4-[2-(diethylamino)ethoxy]phenyl]-17alpha-methyl-östra-1,3,5(10)-trien-3,17beta-diol,
- 4-Chlor-17alpha-methyl-11β-[4-[2-(1-piperidinyl)ethoxy]phenyl]-östra-1,3,5(10)-trien-3,17beta-diol,
- 4-Chlor-17alpha-methyl-11β-[4-[2-(1-pyrrolidinyl)ethoxy]phenyl]-östra-1,3,5(10)-trien-3,17beta-diol,
- 11β-[4-[2-[2-Aza-bicyclo[2.2.1]hept-2-yl]ethoxy]phenyl]-4-chlor-17alpha-methyl-östra-1,3,5(10)-trien-3,17beta-diol,
- 11 β-[4-[2-[2-Aza-bicyclo[2.2.1]hept-2-yl]ethoxy]phenyl]-4-chlor-östra-1,3,5(10)-trien-3,17beta-diol,
- 4-Chlor-11β-[4-[2-[methyl(1 -methylethyl)amino]ethoxy]phenyl]-östra-1,3,5(10)-trien-3,17beta-diol,
- 4-Chlor-11β-[4-[2-(tetrahydro-(1H)-1,4-thiazin-4-yl)ethoxy]phenyl]-östra-1,3,5(10)-trien-3,17beta-diol,
- 4-Chlor-11β-[4-[2-[ethyl(propyl)amino]ethoxy]phenyl]-östra-1,3,5(10)-trien-3,17beta-diol,
- 4-Chlor-11β-[4-[2-[ethyl(methyl)amino]ethoxy]phenyl]-östra-1,3,5(10)-trien-3,17beta-diol,
- 4-Chlor-11β-[4-[2-[(1,1-dimethyl-2-propinyl)amino]ethoxy]phenyl]-östra-1,3,5(10)-trien-3,17beta-diol,
- 4-Chlor-11β-[4-[2-(hexahydro-1H-azepin-1-yl)ethoxy]phenyl]-östra-1,3,5(10)-trien-3,17beta-diol,
- 4-Chlor-11β-[4-[2-[cyclohexyl(methyl)amino]ethoxy]phenyl]-östra-1,3,5(10)-trien-3,17beta-diol,
- 4-Chlor-11β-[4-[2-[butyl(ethyl)amino]ethoxy]phenyl]-östra-1,3,5(10)-trien-3,17betadiol,
- 4-Chlor-11β-[4-[2-(1-azetidinyl)ethoxy]phenyl]-östra-1,3,5(10)-trien-3,17beta-diol,
- 4-Chlor-11β-[4-[2-[ethyl(2-propenyl)amino]ethoxy]phenyl]-östra-1,3,5(10)-trien-3,17beta-diol,
- 4-Chlor-11β-[4-[2-[cyclopentyl(methyl)amino]ethoxy]phenyl]-östra-1,3,5(10)-trien-3,17beta-diol,
- 4-Chlor-11β-[4-[2-(dipropylamino)ethoxy]phenyl]-östra-1,3,5(10)-trien-3,17betadiol,
- 4-Chlor-11β-[4-[2-(3-thiazolidinyl)ethoxy]phenyl]-östra-1,3,5(10)-trien-3,17betadiol,
- 4-Chlor-11β-[4-[2-[ethyl(1-methylethyl)amino]ethoxy]phenyl]-östra-1,3,5(10)-trien-3,17beta-diol,
- 4-Chlor-11β-[4-[2-[methyl(propyl)amino]ethoxy]phenyl]-östra-1,3,5(10)-trien-3,17beta-diol,
- 4-Chlor-11β-[4-[2-[butyl(methyl)amino]ethoxy]phenyl]-östra-1,3,5(10)-trien-3,17beta-diol,
- 4-Chlor-11β-[4-[2-[methy(2-propinyl)amino]ethoxy]phenyl]-östra-1,3,5(10)-trien-3,17beta-diol,
- 4-Chlor-11β-[4-[2-(4-methyl-1-piperidinyl)ethoxy]phenyl]-östra-1,3,5(10)-trien-3,17beta-diol,
- 4-Chlor-11β-[4-[2-(1-piperidinyl)ethylthio]phenyl]-östra-1,3,5(10)-trien-3,17betadiol,
- 4-Chlor-11β-[4-[2-(1 -piperidinyl)ethylsulfinyl]phenyl]-östra-1,3,5(10)-trien-3,17betadiol,
- 4-[4-Chlor-11beta-[4-[2-(diethylamino)ethoxy]phenyl]-17beta-hydroxy-östra-1,3,5(10)-trien-3-yl-oxy]butansäure,
- Oxim von 4-Chlor-3-hydroxy-11beta-[4-[2-(1-piperidinyl)ethoxy]phenyl]-östra-1,3,5(10)-trien-17-on,
- 4-[[2-[4-(4-Chlor-3,17beta-dihydroxy-östra-1,3,5(10)trien-11beta-yl)phenoxy]ethyl]-ethylamino]-butansäure.

7. Verbindung der Formel (I) oder (I'), wie in einem der Ansprüche 1 bis 5 definiert, deren Namen folgt:

- 4-Chlor-11β-[4-[2-(diethylamino)ethoxy]phenyl]-östra-1,3,5(10)-trien-3,17beta-diol, sowie ihre Additionssalze mit den Säuren.

8. Verfahren zur Herstellung der Verbindungen der allgemeinen Formel (I), wie in Anspruch 1 oder 2 definiert, **dadurch gekennzeichnet, dass** man eine Verbindung der allgemeinen Formel (II):

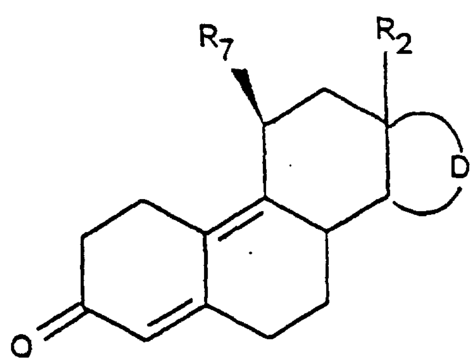

(II)

worin D und $R_2$ wie in Anspruch 1 definiert sind, $R_7$ eine der folgenden Gruppen darstellt:

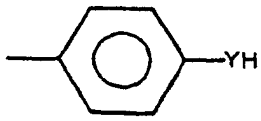
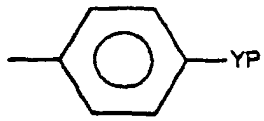
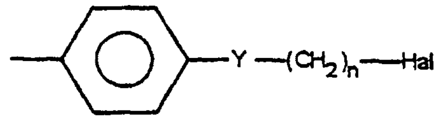

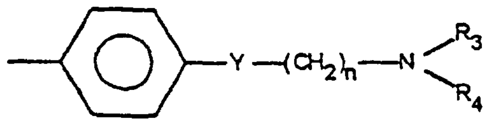

worin n, Y, $R_3$ und $R_4$ wie in Anspruch 1 definiert sind, P eine Schutzgruppe ist, Hal ein Halogen darstellt, der Einwirkung eines Halogenierungsreagenzes unterzieht, um die Verbindung der Formel (III) zu erhalten:

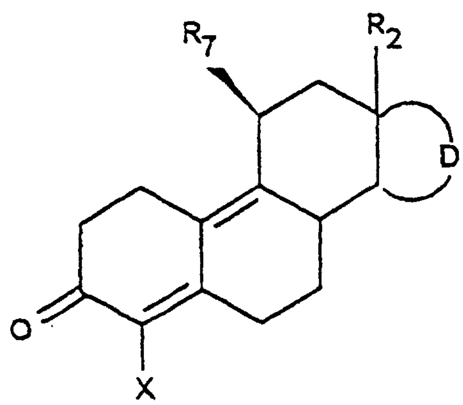

(III)

die man der Einwirkung eines Reagenzes zur Aromatisierung des Rings A, dann der Einwirkung einer Base unterzieht, um die Verbindung der Formel (IV) zu erhalten, die bestimmten Verbindungen der allgemeinen Formel (I) entspricht:

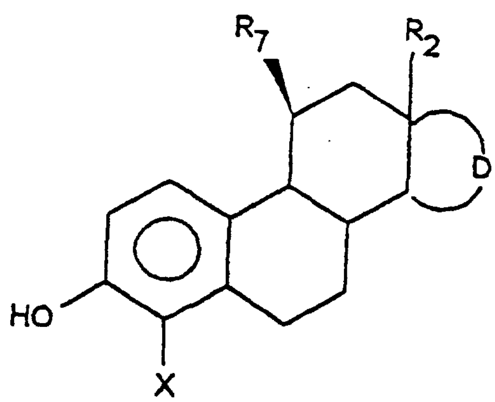

(IV)

Verbindungen der Formel (II), (III) oder (IV),
die man, wenn gewünscht und wenn notwendig, in einer geeigneten Reihenfolge einer oder mehreren der folgenden Reaktionen unterzieht:

- Schutz der Verbindungen, worin $R_7$ eine Gruppe -Ph-YH ist,

- Entfernung der Schutzgruppe der Verbindungen, worin $R_7$ eine Gruppe Ph-YP ist,
- Einwirkung einer Verbindung der Formel $Hal_1$-$(CH_2)_n$-$Hal_2$ auf die Verbindungen, worin $R_7$ eine Gruppe -Ph-YH ist, wobei $Hal_1$ oder $Hal_2$, die gleich oder verschieden sind, ein Halogen darstellen, um Verbindungen zu erhalten, worin $R_7$ eine Gruppe Ph-Y-$(CH_2)_n$-$Hal_2$ ist,
- Einwirkung einer Verbindung der Formel $R_3$-NH-$R_4$ auf die Verbindungen, worin $R_7$ eine Gruppe Ph-Y-$(CH_2)_n$-$Hal_2$ ist, um Verbindungen zu erhalten, worin $R_7$ eine Gruppe Ph-Y-$(CH_2)_n$-$NR_3R_4$ ist,
- Einwirkung eines Halogenidsalzes (M-$Hal_3$) auf die Verbindungen, worin $R_7$ eine Gruppe Ph-Y-$(CH_2)_n$-$Hal_2$ ist, um Verbindungen zu erhalten, worin $R_7$ eine Gruppe Ph-Y-$(CH_2)_n$-$Hal_3$ ist,
- Schutz der OH-Gruppe in Position 3 oder 17,
- Entfernung der Schutzgruppe der OH-Gruppe in Position 3 oder 17,
- Alkylierung der OH-Gruppe in Position 3 oder 17,
- Acylierung der OH-Gruppe in Position 3 oder 17,
- Einwirkung eines Reduktionsmittels, wenn D den Rest eines Fünfrings darstellt, wie in Anspruch 2 definiert, und $R_5$ und $R_6$ zusammen eine Oxogruppe bilden,
- Einwirkung einer metallorganischen Verbindung auf die Verbindungen der Formel (IV) mit D, das den Rest eines Fünfrings darstellt, wie in Anspruch 2 definiert, und $R_5$ und $R_6$, die zusammen eine Oxogruppe bilden,
- Einwirkung eines Mittels zur Lactonbildung auf die Verbindungen der Formel (IV) mit D, das den Rest eines Fünfrings darstellt, wie in Anspruch 2 definiert, und $R_5$ und $R_6$, die zusammen eine Oxogruppe bilden,
- Einwirkung eines Mittels zur Reduktion der Doppelbindung, wenn D den Rest eines Fünfrings darstellt, wie in Anspruch 2 definiert, und $R_5$ und $R_6$ zusammen mit dem Kohlenstoff, der sie trägt, eine Gruppe O-$(CH_2)_{n'}$-CH=CH- bilden,
- Einwirkung eines Mittels zur Reduktion der Doppelbindung, wenn D den Rest eines Fünfrings darstellt, wie in Anspruch 2 definiert, und $R_6$ ein Alkenyl- oder Alkinylrest mit 2 bis 6 Kohlenstoffatomen ist,
- Halogenierung in Position 4, dann Aromatisierung des Rings A der Verbindung der allgemeinen Formel (II),
- Aromatisierung des Rings A der Verbindung der Formel (III),
- Salzbildung.

**9.** Verfahren zur Herstellung gemäß Anspruch 8 der Verbindungen der allgemeinen Formel (I'), wie in Anspruch 3 definiert, **dadurch gekennzeichnet, dass** man eine Verbindung der allgemeinen Formel (II'):

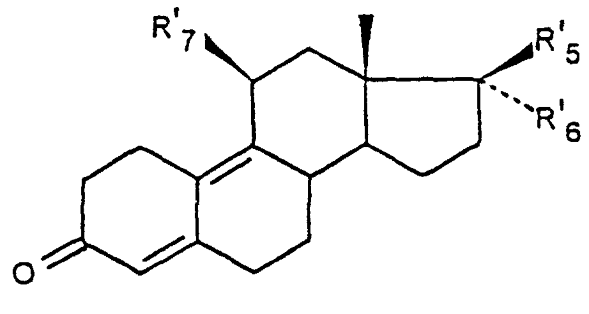

(II')

worin $R'_5$ und $R'_6$ wie in Anspruch 3 definiert sind, $R'_7$ darstellt:

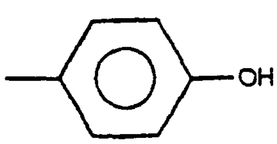

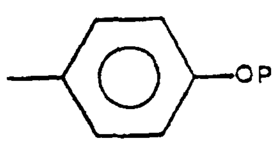

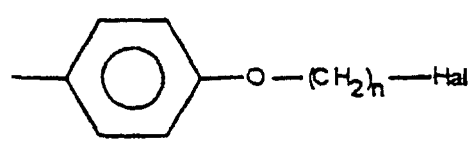

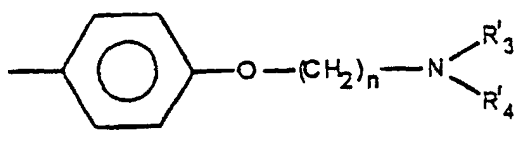

der Einwirkung eines Halogenierungsreagenzes unterzieht, um die Verbindung der Formel (III') zu erhalten:

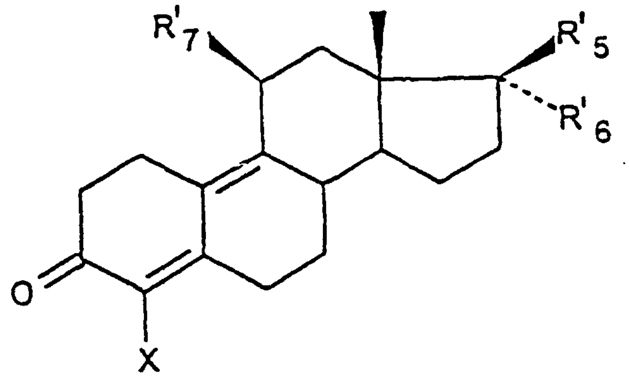

(III')

die man der Einwirkung eines Reagenzes zur Aromatisierung des Rings A, dann der Einwirkung einer Base unterzieht, um die Verbindung der Formel (IV') zu erhalten, die bestimmten Verbindungen der allgemeinen Formel (I') entspricht

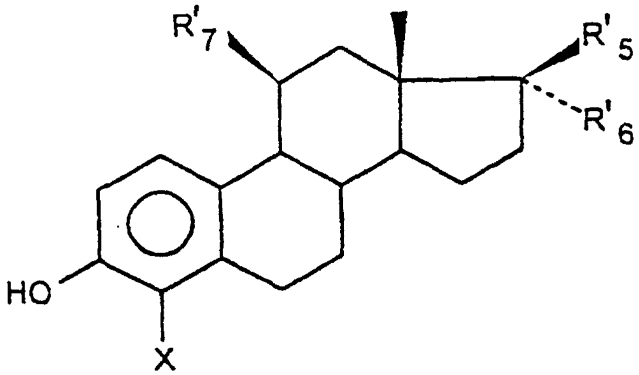

(IV')

Verbindungen der Formel (II'), (III') oder (IV), die man, wenn gewünscht und wenn notwendig, in einer geeigneten Reihenfolge einer oder mehreren der folgenden Reaktionen unterzieht:

- Schutz der Verbindungen, worin $R'_7$ eine Gruppe -Ph-OH ist,
- Entfernung der Schutzgruppe der Verbindungen, worin $R'_7$ eine Gruppe Ph-OP ist,
- Einwirkung einer Verbindung der Formel $Hal_1-(CH_2)_n-Hal_2$ auf die Verbindungen, worin $R'_7$ eine Gruppe -Ph-OH ist, wobei $Hal_1$ oder $Hal_2$, die gleich oder verschieden sind, ein Halogen darstellen, um Verbindungen zu erhalten, worin $R_7$ eine Gruppe Ph-O-$(CH_2)_n$-$Hal_2$ ist,
- Einwirkung einer Verbindung der Formel $R'_3$-NH-$R'_4$ auf die Verbindungen, worin $R'_7$ eine Gruppe Ph-O-$(CH_2)_n$-$Hal_2$ ist, um Verbindungen zu erhalten, worin $R'_7$ eine Gruppe Ph-O-$(CH_2)_n$-$NR'_3R'_4$ ist,
- Einwirkung eines Halogenidsalzes (M-$Hal_3$) auf die Verbindungen, worin $R'_7$ eine Gruppe Ph-O-$(CH_2)_n$-$Hal_2$ ist, um Verbindungen zu erhalten, worin $R_7$ eine Gruppe Ph-O-$(CH_2)_n$-$Hal_3$ ist,
- Schutz der OH-Gruppe in Position 3 oder 17,
- Entfernung der Schutzgruppe der OH-Gruppe in Position 3 oder 17,
- Alkylierung der OH-Gruppe in Position 17,
- Acylierung der OH-Gruppe in Position 17,
- Einwirkung eines Reduktionsmittels, wenn $R'_5$ und $R'_6$ zusammen eine Oxogruppe bilden,
- Einwirkung einer metallorganischen Verbindung auf die Verbindungen der Formel (IV') mit $R'_5$ und $R'_6$, die zusammen eine Oxogruppe bilden,
- Einwirkung eines Mittels zur Lactonbildung auf die Verbindungen der Formel (IV') mit $R'_5$ und $R'_6$, die zusammen eine Oxogruppe bilden,
- Einwirkung eines Mittels zur Reduktion der Doppelbindung, wenn $R'_5$ und $R'_6$ zusammen mit dem Kohlenstoff, der sie trägt, eine Gruppe O-$(CH_2)_{n'}$-CH=CH- bilden,

- Einwirkung eines Mittels zur Reduktion der Doppelbindung, wenn $R'_6$ ein Alkenyloder Alkinylrest mit 2 bis 6 Kohlenstoffatomen ist,
- Halogenierung in Position 4, dann Aromatisierung des Rings A der Verbindung der Formel (II'),
- Aromatisierung der Verbindung der Formel (III'),
- Salzbildung.

**10.** Als Medikamente die Verbindungen der Formel (I), wie in Anspruch 1 definiert, sowie ihre Additionssalze mit den pharmazeutisch annehmbaren Säuren.

**11.** Als Medikamente die Verbindungen der Formel (I) oder (I'), wie in einem der Ansprüche 2 bis 5 definiert, sowie ihre Additionssalze mit den pharmazeutisch annehmbaren Säuren.

**12.** Als Medikamente die Verbindungen, wie in Anspruch 6 oder 7 definiert, sowie ihre Additionssalze mit den pharmazeutisch annehmbaren Säuren.

**13.** Pharmazeutische Zusammensetzungen, die eins oder mehrere der Medikamente, wie in einem der Ansprüche 10, 11 oder 12 definiert, enthalten.

**14.** Als neue Zwischenprodukte die Verbindungen der allgemeinen Formel (III), (III'), (IV) oder (IV), wie in Anspruch 8 oder 9 definiert.

**15.** Medikament, wie in den Ansprüchen 10 bis 12 definiert, für die Vorbeugung oder die Behandlung der Osteoporose.

**16.** Medikament, wie in den Ansprüchen 10 bis 12 definiert, für den kardiovaskulären Schutz.

## Claims

**1.** Compounds of general formula (I):

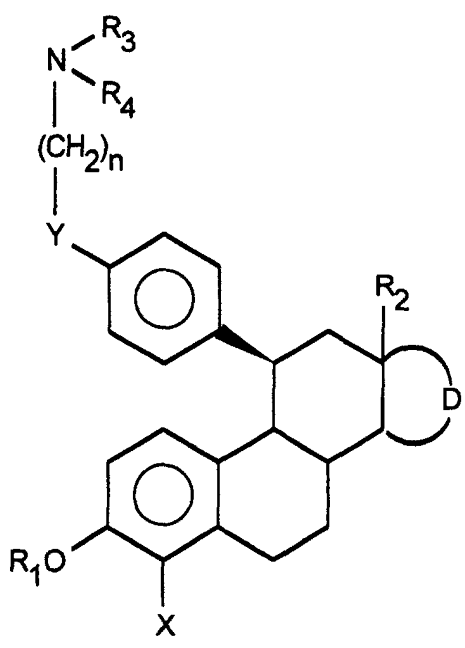

(I)

in which:

$R_1$ represents a hydrogen atom, a $(CH_2)_m$-Ar, (CO)-Ar, $(CH_2)_m$-Alk or (CO)-Alk radical,
$R_2$ represents a radical derived from a linear or branched, saturated or unsaturated hydrocarbon, containing from 1 to 6 carbon atoms

D represents the remainder of a pentagonal or hexagonal ring optionally substituted and optionally carrying an unsaturation,
X represents a halogen atom,
Y is chosen from O, S, SO, $SO_2$ and NH,
n is an integer ranging from 2 to 8,

either $R_3$ and $R_4$, identical or different, represent a hydrogen atom, a $(CH_2)_m$-Ar, $(CH_2)_m$-Het or $(CH_2)_m$Alk group, or $R_3$ and $R_4$ together with the nitrogen atom to which they are linked form a mono or polyclic, saturated or unsaturated, aromatic or non-aromatic heterocycle, with 3 to 15 members optionally containing from 1 to 3 additional heteroatoms chosen from oxygen, sulphur and nitrogen, non-substituted or substituted,
Ar representing a carbocyclic aryl group containing from 6 to 18 carbon atoms, Het representing a radical derived from an aromatic or non-aromatic, saturated or non-saturated heterocycle comprising 1 to 9 carbon atoms and 1 to 5 heteroatoms chosen from the oxygen, nitrogen or sulphur atoms, Alk representing a radical derived from a non-aromatic, linear, branched or cyclic, saturated or unsaturated hydrocarbon comprising 1 to 12 carbon atoms, the Ar, Het or Alk radicals being able to be substituted or non-substituted, m represents 0, 1, 2 or 3, as well as their addition salts with bases or acids.

**2.** Compounds of general formula (I) as defined in claim 1, in which D represents the remainder of a pentagonal ring of formula:

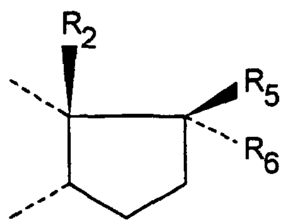

in which $R_2$ retains the same meaning as in claim 1,
either $R_5$ represents an OH, O-$(CH_2)_m$-Alk, O-(CO)-Alk, O-$(CH_2)_m$-Ar, O-(CO)-Ar, O-$(CH_2)_m$-Het, O-(CO)-Het radical and $R_6$ represents a hydrogen atom, an alkyl, alkenyl or alkynyl radical containing from 1 to 6 carbon atoms, substituted or non-substituted, m, Alk, Ar and Het being as defined in claim 1,
or $R_5$ and $R_6$ together with the carbon atom which carries them form one of the following rings:

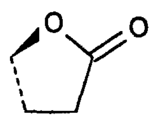

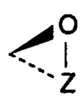

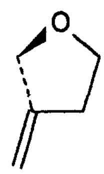

in which Z represents a $-(CH_2)_1-$ or $-CH=CH-(CH_2)_{1'}$ group, 1 being an integer comprised between 1 and 4 and 1' being an integer equal to 1 or 2,
or $R_5$ and $R_6$ together form an oxo or =N-OH group,
as well as their addition salts with acids or bases.

**3.** Compounds of general formula (I) as defined in claim 1, corresponding to general formula (I') :

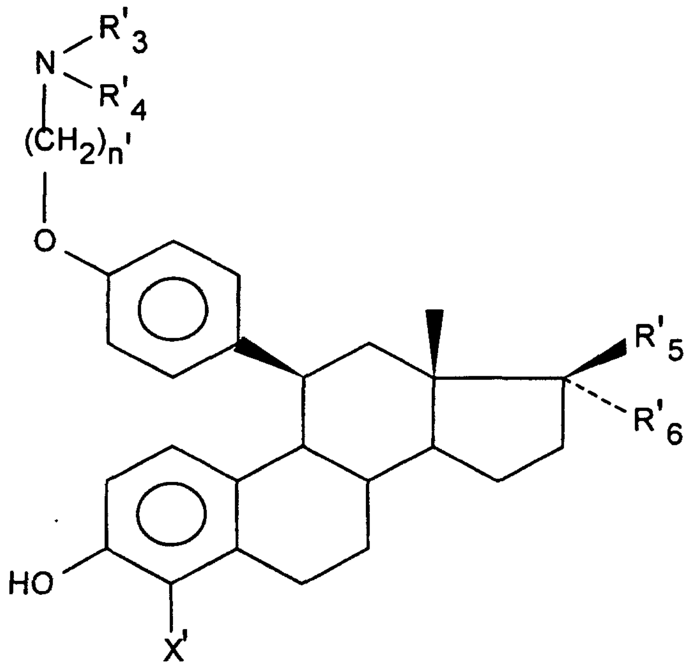

(I')

in which:

X' represents a chlorine or bromine atom
n' is comprised between 2 and 5,

<u>either</u> $R'_3$ and $R'_4$, identical or different, represent an alkyl radical containing from 1 to 6 carbon atoms <u>or</u> $R'_3$ and $R'_4$ together with the nitrogen atom to which they arelinked, form a mono or polyclic saturated remainder with 3 to 15 members optionally containing an additional heteroatom chosen from oxygen, sulphur and nitrogen, $R'_5$ and $R'_6$ have the same meaning as $R_5$ and $R_6$, according to claim 2 as well as their addition salts with acids and bases.

**4.** Compounds of general formula (I') as defined in claim 3, in which:
<u>either</u> $R'_5$ represents an OH radical and $R'_6$ represents a hydrogen atom, an alkyl, alkenyl or alkynyl radical containing from 1 to 6 carbon atoms, substituted or non-substituted,
<u>or</u> $R'_5$ and $R'_6$ together with the carbon atom which carries them form one of the following rings:

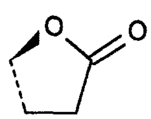 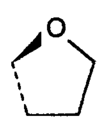 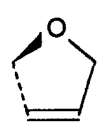 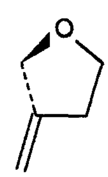

<u>or</u> $R'_5$ and $R'_6$ together form an oxo group,
as well as their addition salts with acids or bases.

**5.** Compounds of general formula (I') as defined in claim 3 or 4, in which:

X' represents a chlorine atom,
n' is equal to 2,

<u>either</u> $R'_3$ and $R'_4$, identical or different, represent an alkyl radical containing from 1 to 6 carbon atoms,
<u>or</u> $R'_3$ and $R'_4$ together with the nitrogen atom form the following saturated heterocycles:

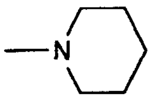 , 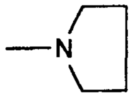 , 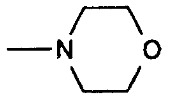 , 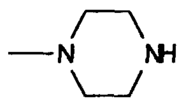 and 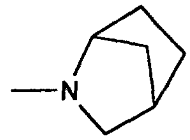

and <u>either</u> $R'_5$ represents an OH radical and $R'_6$ represents a hydrogen atom, an alkyl, alkenyl or alkynyl radical containing from 1 to 6 carbon atoms, substituted or non-substituted,
<u>or</u> $R'_5$ and $R'_6$ together with the carbon atom which carries them form one of the following rings:

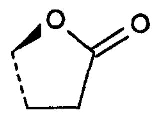 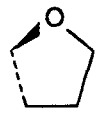 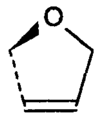 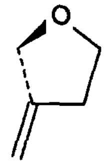

<u>or</u> $R'_5$ and $R'_6$ together form an oxo group,
as well as their addition salts with acids or bases.

**6.** Compounds of formula (I) or (I') as defined in any one of claims 1 to 5, the names of which follow:

- 4-chloro-3-hydroxy-11β-[4-[2-(diethylamino)ethoxy]phenyl]-estra-1,3,5(10)-trien-17-one,
- 4-chloro-3-hydroxy-11β-[4-[2-(dimethylamino)ethoxy]phenyl]-estra-1,3,5(10)-trien-17-one,
- 4-chloro-3-hydroxy-11β-[4-[2-(1-piperidinyl)ethoxy]phenyl]-estra-1,3,5(10)-trien-17-one,
- 4-chloro-3-hydroxy-11β-[4-[2-(1-pyrrolidinyl)ethoxy] phenyl]-estra-1,3,5(10)-trien-17-one,
- 4-bromo-3-hydroxy-11β-[4-[2-(1-piperidinyl)ethoxy]phenyl]-estra-1,3,5(10)-trien-17-one,
- 4-chloro-11β-[4-[2-(dimethylamino)ethoxy]phenyl]-estra-1,3,5(10)-triene-3,17beta-diol,
- 4-chloro-11β-[4-[2-(diethylamino)ethoxy]phenyl]-estra-1,3,5(10)-triene-3,17beta-diol,
- 4-chloro-11β-[4-[2-(1-piperidinyl)ethoxy]phenyl]-estra-1,3,5(10)-triene-3,17beta-diol,
- 4-bromo-11β-[4-[2-(1-piperidinyl)ethoxy]phenyl]-estra-1,3,5(10)-triene-3,17beta-diol,
- 4-chloro-11β-[4-[2-(1-pyrrolidinyl)ethoxy]phenyl]-estra-1,3,5(10)-triene-3,17beta-diol,
- 4-chloro-11β-[4-[2-(diethylamino)ethoxy]phenyl]-19-nor-17alpha-pregna-1,3,5(10)-trien-20-yne-3,17beta-diol,
- 4-chloro-11β-[4-[3-(1-piperidinyl)propoxy]phenyl]-estra-1,3,5(10)-triene-3,17beta-diol,
- 4-chloro-11β-[4-[4-(1-piperidinyl)butoxy]phenyl]-estra-1,3,5(10)-triene-3,17beta-diol,
- 4-chloro-11β-[4-[5-(1-piperidinyl)pentyloxy]phenyl]-estra-1,3,5(10)-triene-3,17beta-diol,
- gamma lactone of (17alpha)-4-chloro-3,17beta-dihydroxy-11β-[4-[2-(diethylamino)ethoxy]phenyl]-19-nor-pregna-1,3,5(10)-triene-21-carboxylic acid,
- gamma lactone of (17alpha)-4-chloro-3,17beta-dihydroxy-11β-[4-[2-(1-pyrrolidinyl)ethoxy]phenyl]-19-nor-pregna-1,3,5(10)-triene-21-carboxylic acid,
- gamma lactone of (17alpha)-4-chloro-3,17beta-dihydroxy-11β-[4-[2-(1-piperidinyl)ethoxy]phenyl]-19-norpregna-1,3,5(10)-triene-21-carboxylic acid,
- gamma lactone of (17alpha)-4-chloro-3,17beta-dihydroxy-11β-[4-[3-(1-piperidinyl)propoxy]phenyl]-19-nor-pregna-1,3,5(10)-triene-21-carboxylic acid,
- gamma lactone of (17alpha)-4-chloro-3,17beta-dihydroxy-11β-[4-[4-(1-piperidinyl)butoxy]phenyl]-19-norpregna-1,3,5(10)-triene-21-carboxylic acid,
- gamma lactone of (17alpha)-4-chloro-3,17beta-dihydroxy-11β-[4-[5-(1-piperidinyl)pentyloxy]phenyl]-19-nor-pregna-1,3,5(10)-triene-21-carboxylic acid,
- (17beta)-4-chloro-11β-[4-[2-(diethylamino)ethoxy]phenyl]-spiro[estra-1,3,5(10)-triene-17,2'(5'H)furan]-3-ol,
- (17beta)-4-chloro-11β-[4-[2-(1-piperidinyl)ethoxy]phenyl]-spiro[estra-1,3,5(10)-triene-17,2'(5'H)furan]-3-ol,
- (17beta)-4-chloro-11β-[4-[2-(1-pyrrolidinyl)ethoxy]phenyl]-spiro[estra-1,3,5(10)-triene-17,2'(5'H) furan]-3-ol,
- (17beta)-4-chloro-4',5'-dihydro-11β-[4-[2-(1-piperidinyl) ethoxy]phenyl]-spiro[estra-1,3,5(10)-triene-17,2'(3'H) furan]-3-ol,
- (17beta)-4-chloro-11β-[4-[3-(1-piperidinyl)propoxy]phenyl]-spiro[estra-1,3,5(10)-triene-17,2'(5'H)furan]-3-ol,
- (17beta)-4-chloro-4',5'-dihydro-11β-[4-(3-(1-piperidinyl) propoxy]phenyl]-spiro[estra-1,3,5(10)-triene-17,2'(3'H) furan]-3-ol,

- (17beta)-4-chloro-11β-[4-[4-(1-piperidinyl)butoxy]phenyl]-spiro[estra-1,3,5(10)-triene-17,2'(5'H)furan]-3-ol,
- (17beta)-4-chloro-4',5'-dihydro-11β-[4-[4-(1-piperidinyl)-butoxy]phenyl]-spiro[estra-1,3,5(10)-triene-17,2'(3'H)furan]-3-ol,
- 4-chloro-11β-[4-[2-(diethylamino)ethoxy]phenyl]-17alpha-methyl-estra-1,3,5(10)-triene-3,17beta-diol
- 4-chloro-17alpha-methyl-11β-[4-[2-(1-piperidinyl) ethoxy] phenyl]-estra-1,3,5(10)-triene-3,17beta-diol,
- 4-chloro-17alpha-methyl-11β-[4-[2-(1-pyrrolidinyl)ethoxy] phenyl]-estra-1,3,5(10)-triene-3,17beta-diol,
- 11β-[4-[2-[2-aza-bicyclo(2.2.1)hept-2-yl]ethoxy] phenyl]-4-chloro-17alpha-methyl-estra-1,3,5(10)-triene-3,17beta-diol,
- 11β-[4-[2-[2-aza-bicyclo(2.2.1)hept-2-yl]ethoxy] phenyl]-4-chloro-estra-1,3,5(10)-triene-3,17beta-diol,
- 4-chloro-11β-[4-[2-[methyl(1-methylethyl)amino]ethoxy] phenyl]-estra-1,3,5(10)-triene-3,17beta-diol,
- 4-chloro-11β-[4-[2-(tetrahydro-(1H)-1,4-thiazin-4-yl) ethoxy]phenyl]-estra-1,3,5(10)-triene-3,17beta-diol,
- 4-chloro-11β-[4-[2-[ethyl(propyl)amino]ethoxy]phenyl]-estra-1,3,5(10)-triene-3,17beta-diol,
- 4-chloro-11β-[4-[2-[ethyl(methyl)amino]ethoxy]phenyl]-estra-1,3,5(10)-triene-3,17beta-diol,
- 4-chloro-11β-[4-[2-[(1,1-dimethyl-2-propynyl)amino]ethoxy] phenyl]-estra-1,3,5(10)-triene-3,17beta-diol,
- 4-chloro-11β-[4-[2-[hexahydro-1H-azepin-1-yl)ethoxy] phenyl]-estra-1,3,5(10)-triene-3,17beta-diol,
- 4-chloro-11β-[4-[2-[cyclohexyl(methyl)amino]ethoxy]phenyl]-estra-1,3,5(10)-triene-3,17beta-diol,
- 4-chloro-11β-[4-[2-[butyl(ethyl)amino]ethoxy]phenyl]-estra-1,3,5(10)-triene-3,17beta-diol,
- 4-chloro-11β-[4-[2-(1-azetidinyl)ethoxy]phenyl]-estra-1,3,5(10)-triene-3,17beta-diol,
- 4-chloro-11β-[4-[2-[ethyl(2-propenyl)amino]ethoxy]phenyl]-estra-1,3,5(10)-triene-3,17beta-diol,
- 4-chloro-11β-[4-[2-[cyclopentyl(methyl)amino]ethoxy] phenyl]-estra-1,3,5(10)-triene-3,17beta-diol,
- 4-chloro-11β-[4-[2-(dipropylamino)ethoxy]phenyl]-estra-1,3,5(10)-triene-3,17beta-diol,
- 4-chloro-11β-[4-[2-(3-thiazolidinyl)ethoxy]phenyl]-estra-1,3,5(10)-triene-3,17beta-diol,
- 4-chloro-11β-[4-[2-[ethyl(1-methylethyl)amino]ethoxy] phenyl]-estra-1,3,5(10)-triene-3,17beta-diol,
- 4-chloro-11β-[4-[2-[methyl(propyl)amino]ethoxy]phenyl]-estra-1,3,5(10)-triene-3,17beta-diol,
- 4-chloro-11β-[4-[2-[butyl(methyl)amino]ethoxy]phenyl]-estra-1,3,5(10)-triene-3,17beta-diol,
- 4-chloro-11β-[4-[2-[methyl(2-propynyl)amino]ethoxy]phenyl]-estra-1,3,5(10)-triene-3,17beta-diol,
- 4-chloro-11β-[4-[2-(4-methyl-1-piperidinyl)ethoxy]phenyl]-estra-1,3,5(10)-triene-3,17beta-diol,
- 4-chloro-11β-[4-[2-(1-piperidinyl)ethylthio]phenyl]-estra-1,3,5(10)-triene-3,17beta-diol,
- 4-chloro-11β-[4-[2-(1-piperidinyl)ethylsulphinyl]phenyl]-estra-1,3,5(10)-triene-3,17beta-diol,
- 4-[4-chloro-llbeta-[4-[2-(diethylamino)ethoxy]phenyl] 17beta-hydroxy-estra-1,3,5(10)-trien-3-yl-oxy]-butanoic acid,
- 4-chloro-3-hydroxy-11beta-[4-[2-(1-piperidinyl) ethoxy]phenyll-estra-1,3,5(10)-trien-17-one oxime,
- 4-[[2-[4-(4-chloro-3,17beta-dihydroxy-estra-1,3,5(10)-trien-11beta-yl)phenoxy]ethyl]ethylamino]-butanoic acid.

**7.** Compound of formula (I) or (I') as defined in any one of claims 1 to 5, the names of which follow:

- 4-chloro-11β-[4-[2-(diethylamino)ethoxy]phenyl]-estra-1,3,5(10)-triene-3,17beta-diol, as well as its addition salts with acids.

**8.** Process for the preparation of the compounds of general formula (I) as defined in claim 1 or 2, **characterized in that** a compound of general formula (II):

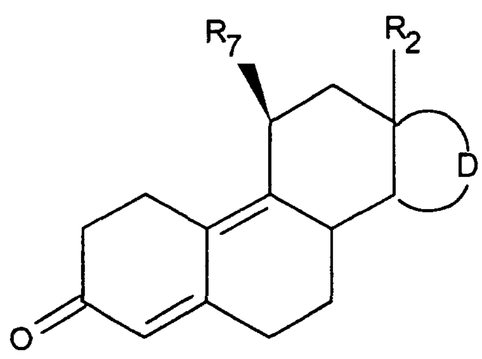

(II)

in which D and $R_2$ are as defined in claim 1, $R_7$ represents one of the following groups:

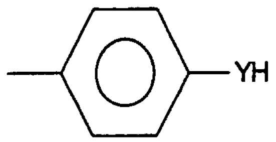

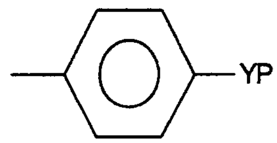

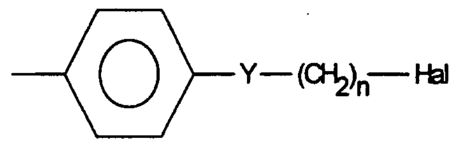

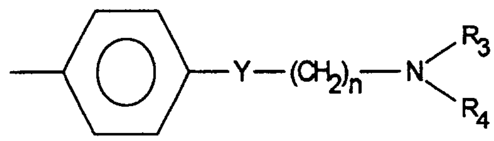

in which n, Y, $R_3$ and $R_4$ are as defined in claim 1, P is a protective group, Hal represents a halogen, is subjected to the action of a halogenation reagent in order to obtain the compound of formula (III):

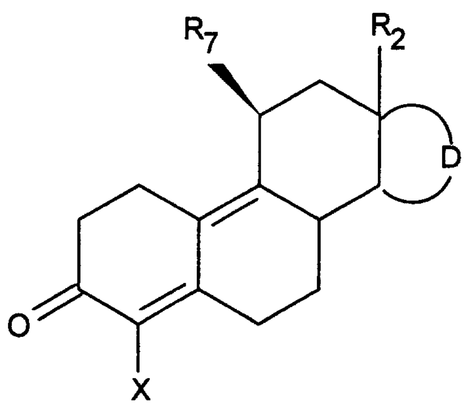

(III)

which is subjected to the action of an aromatization reagent of ring A, then to the action of a base in order to obtain the compound of formula (IV) corresponding to certain compounds of general formula (I):

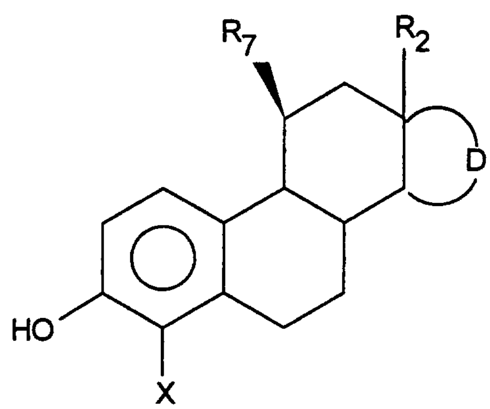

(IV)

which compounds of formula (II), (III) or (IV)
are subjected, if required and if necessary, in an appropriate order, to one or more of the following reactions:

- protection of the compounds in which $R_7$ is a -Ph-YH group,
- deprotection of the compounds in which $R_7$ is a Ph-YP group,
- action of a compound of formula $Hal_1$-$(CH_2)_n$-$Hal_2$ on the compounds in which $R_7$ is a -Ph-YH group, $Hal_1$ or $Hal_2$, identical or different, representing a halogen in order to obtain compounds in which $R_7$ is a -Ph-Y-$(CH_2)_n$-$Hal_2$ group,
- action of a compound of formula $R_3$-NH-$R_4$ on the compounds in which $R_7$ is a Ph-Y-$(CH_2)_n$-$Hal_2$ group, in order to obtain compounds in which $R_7$ is a Ph-Y-$(CH_2)_n$-$NR_3R_4$ group,
- action of a halide salt (M-$Hal_3$) on the compounds in which $R_7$ is a Ph-Y-$(CH_2)_n$-$Hal_2$ group in order to obtain compounds in which $R_7$ is a -Ph-Y-$(CH_2)_n$-$Hal_3$ group,
- protection of the OH group in position 3 or 17,
- deprotection of the OH group in position 3 or 17,
- alkylation of the OH group in position 3 or 17,
- acylation of the OH group in position 3 or 17,
- action of a reducing agent when D represents the remainder of a pentagonal ring As defined in claim 2 and $R_5$ and $R_6$ form together an oxo group,
- action of an organometallic on the compounds of formula (IV) with D representing the remainder of a pentagonal ring as defined in claim 2 and $R_5$ and $R_6$ together form an oxo group,
- action of a lactonization agent on the compounds of formula (IV) with D representing the remainder of a pentagonal ring as defined in claim 2 and $R_5$ and $R_6$ together forming an oxo group,
- action of a reducing agent of the double bond, when D represents the remainder of a pentagonal ring as defined in claim 2 and $R_5$ and $R_6$ together with the carbon which carries them, form an O-$(CH_2)_{n'}$-CH=CH- group,
- action of a reducing agent of the double bond, when D represents the remainder of a pentagonal ring as defined in claim 2, and $R_6$ is an alkenyl or alkynyl radical containing from 2 to 6 carbon atoms,
- halogenation in position 4, then aromatization of ring A, of the compound of general formula (II),
- aromatization of ring A of the compound of formula (III),
- salification.

**9.** Process according to claim 8, for the preparation of compounds of general formula (I') as defined in claim 3, **characterized in that** a compound of general formula (II'):

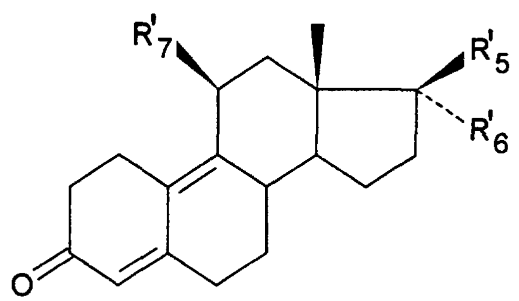

(II')

in which $R'_5$ and $R'_6$ are as defined in claim 3, $R'_7$ represents:

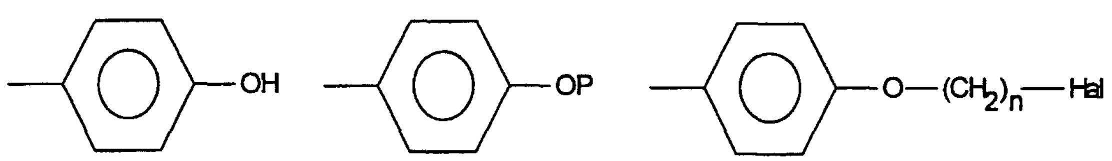

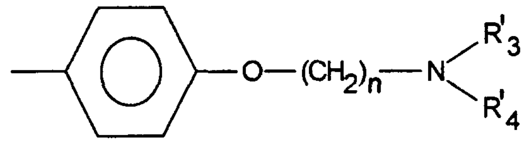

is subjected to the action of a halogenation reagent in order to obtain the compound of formula (III') :

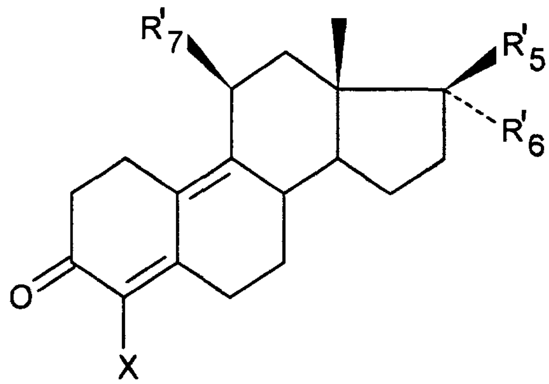

(III')

which is subjected to the action of an aromatization reagent of ring A, then to the action of a base in order to obtain the compound of formula (IV') corresponding to certain compounds of general formula (I'):

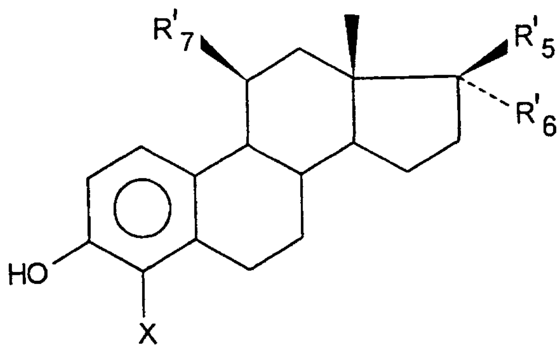

(IV')

which compounds of formula (II'), (III') or (IV'), are subjected, if required and if necessary, in an appropriate order, to one or more of the following reactions:

- protection of the compounds in which $R'_7$ is a -Ph-OH group,
- deprotection of the compounds in which $R'_7$ is a Ph-OP group,
- action of a compound of formula $Hal_1-(CH_2)_n-Hal_2$ on the compounds in which $R'_7$ is a -Ph-OH group, $Hal_1$ or $Hal_2$, identical or different, representing a halogen in order to obtain compounds in which $R_7$ is a $-Ph-O-(CH_2)_n-Hal_2$ group,
- action of a compound of formula $R'_3-NH-R'_4$ on the compounds in which $R'_7$ is a $Ph-O-(CH_2)_n-Hal_2$ group, in order to obtain compounds in which $R'_7$ is a $Ph-O-(CH_2)_n-NR'_3R'_4$ group,
- action of a halide salt ($M-Hal_3$) on the compounds in which $R'_7$ is a $Ph-O-(CH_2)_n-Hal_2$ group in order to obtain compounds in which $R_7$ is a $-Ph-O-(CH_2)_n-Hal_3$ group,
- protection of the OH group in position 3 or 17,
- deprotection of the OH group in position 3 or 17,
- alkylation of the OH group in position 17,
- acylation of the OH group in position 17,

- action of a reducing agent when $R'_5$ and $R'_6$ together form an oxo group,
- action of an organometallic on the compounds of formula (IV') with $R'_5$ and $R'_6$ together forming an oxo group,
- action of a lactonization agent on the compounds of formula (IV') with $R'_5$ and $R'_6$ forming $R'_5$ and $R'_6$ together forming an oxo group,
- action of a reducing agent of the double bond, when $R'_5$ and $R'_6$ together with the carbon which carries them, form an $O-(CH_2)_n'-CH=CH-$ group,
- action of a reducing agent of the double bond, when $R'_6$ is an alkenyl or alkynyl radical containing from 2 to 6 carbon atoms,
- halogenation in position 4 then aromatization of ring A of the compound of formula (II'),
- aromatization of the compound of formula (III'),
- salification.

**10.** As medicaments the compounds of formula (I) as defined in claim 1, as well as their addition salts with pharmaceutically acceptable acids.

**11.** As a medicaments the compounds of formula (I) or (I') as defined in any one of the claims 2 to 5, as well as their addition salts with pharmaceutically acceptable acids.

**12.** As medicaments the compounds as defined in claim 6 or 7, as well as their addition salts with pharmaceutically acceptable acids.

**13.** Pharmaceutical compositions containing one or more of the medicaments as defined in any one of claims 10, 11 or 12.

**14.** As new intermediate products, the compounds of general formula (III), (III'), (IV) or (IV') as defined in claim 8 or 9.

**15.** Medicament as defined in claims 10 to 12 for the prevention or the treatment of osteoporosis.

**16.** Medicament as defined in claims 10 to 12 for cardiovascular protection.